(19) Europäisches Patentamt
European Patent Office
Office européen des brevets

(11) **EP 4 324 476 A2**

(12) **EUROPEAN PATENT APPLICATION**

(43) Date of publication:
**21.02.2024 Bulletin 2024/08**

(21) Application number: **23201798.8**

(22) Date of filing: **09.09.2016**

(51) International Patent Classification (IPC):
***A61K 39/00*** *(2006.01)*

(52) Cooperative Patent Classification (CPC):
**C07K 16/22; A61P 25/00; A61P 25/28;**
A61K 2039/505; A61K 2039/545; C07K 2317/21;
C07K 2317/76; C07K 2317/94

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.09.2015 US 201562217672 P
01.06.2016 US 201662344024 P
15.07.2016 US 201662362931 P
30.08.2016 US 201662381322 P**

(62) Document number(s) of the earlier application(s) in
accordance with Art. 76 EPC:
**16770154.9 / 3 347 378**

(71) Applicants:
• **AbbVie Inc.**
**North Chicago, IL 60064 (US)**
• **AbbVie Deutschland GmbH & Co KG**
**65189 Wiesbaden (DE)**

(72) Inventors:
• **GREENBERG, Steven J.**
**North Chicago, 60064 (US)**
• **MUELLER, Bernhard K.**
**65189 Wiesbaden (DE)**
• **POPP, Andreas**
**65189 Wiesbaden (DE)**
• **ROSEBRAUGH, Matthew R.**
**North Chicago, 60064 (US)**
• **HAIG, George, M.**
**North Chicago, 60064 (US)**
• **LIN, Shao-Lee**
**North Chicago, 60064 (US)**

(74) Representative: **Plasseraud IP**
**66, rue de la Chaussée d'Antin**
**75440 Paris Cedex 09 (FR)**

Remarks:
•The complete document including Reference Table(s) and the Sequence Listing(s) can be downloaded from the EPO website
•This application was filed on 05.10.2023 as a divisional application to the application mentioned under INID code 62.
•Claims filed after the date of filing of the application (Rule 68(4) EPC).

(54) **METHODS FOR TREATING RELAPSING FORMS OF MULTIPLE SCLEROSIS**

(57) Disclosed herein are anti-RGMa antibodies and methods of using these antibodies to treat multiple sclerosis, including relapsing forms of multiple sclerosis such as relapsing-remitting multiple sclerosis or relapsing-secondary progressive multiple sclerosis.

EP 4 324 476 A2

## Description

### TECHNICAL FIELD

The present invention relates to anti-RGMa antibodies and methods of using these antibodies to treat multiple sclerosis, including relapsing forms of multiple sclerosis such as relapsing-remitting multiple sclerosis or relapsing-secondary progressive multiple sclerosis.

### BACKGROUND

Multiple Sclerosis (MS) is a chronic autoimmune and neurodegenerative disorder of the central nervous system (CNS) that is characterized by inflammation, demyelination, axonal transection, and neuronal loss. The disease affects approximately 2.5 million people worldwide and is the most common cause of neurologic disability among young adults. It is usually diagnosed between the ages of 20 to 40 years with twice as many women affected as men.

Approximately 85% of MS patients are diagnosed initially with relapsing-remitting MS (RRMS). Patients with RRMS experience discrete episodes of neurological dysfunction (referred to as relapses, exacerbations, or attacks), each lasting several days to several weeks, which occur intermittently over many years and are characterized by loss of neurological function separated by periods of relative stability. Neurological functional recovery after a relapse is variable, but recovery tends to be incomplete over time and an estimated 42% to 57% of relapses are associated with residual neurological deficits. Clinical symptoms are variable and involve motor, sensory, visual, bladder and bowel dysfunction and imbalance. Brain atrophy, emblematic of loss of axons and myelin and coincident with loss of cognitive function, occurs early and is progressive throughout the clinical course. A majority of patients with RRMS eventually develop secondary progressive MS (SPMS) in which disability progresses independent of clinically distinct relapses. Relapses may occur in patients with SPMS, especially during the transition from RRMS to SPMS and during the early course of SPMS (relapsing SPMS) and may be associated with acute inflammatory lesions detected by gadolinium enhancement on T1 weighted magnetic resonance imaging (MRI). Thus, the term relapsing forms of MS (RFMS) refers to patients that have RRMS or relapsing SPMS. However, over time, relapses become much less frequent and may cease to occur in parallel with a commensurate reduction in acute inflammatory lesions detected on MRI (non-relapsing SPMS).

The major cause of irreversible disability in patients with MS is due to the cumulative axon/neuronal and myelin/oligodendroglial damage over time. Axon damage, including transection of the axon, begins early in MS and correlates with inflammatory activity, but may occur in areas with little or no evidence of inflammation. Several mechanisms lead to axon loss, including inflammatory secretions, loss of oligodendroglial-derived support, disruption of axonal ion concentrations, energy failure, and calcium accumulation. Both the innate and the adaptive arms of the immune system are involved in the aberrant response to several antigens associated with the myelin sheath and oligodendrocytes after the activation of immune cells by self- or cross-reactive microbial pathogens. The cellular markers on T cells (CD4+ Th1 cell, in particular), have been implicated, but are facilitated by a variety of other cell types (CD8+ T cells, B cells, macrophages, and microglia) and soluble products (proteases, cytokines, and nitric oxide) that act both outside of and within the CNS. Axonal transection and axonal loss described in postmortem studies have been shown to be associated with factors inhibitory to remyelination and neuroregeneration. In addition, brain and spinal cord atrophy are hallmark features in MS patients and estimates of the total axon loss in spinal cord lesions at end stage disease approach 70%.

Thus, there is growing recognition that despite the major therapeutic advances over the last two decades in the development of more robust immune-modulatory, anti inflammatory drugs, these treatment modalities are only modestly effective in preventing and reversing the neurodegenerative components of axonopathy and oligodendroglial apoptosis, which represent the major causes of permanent neurological disability in MS patients. Therefore, there is a need in the art for new methods of treating MS patients that are effective in preventing, reversing and restoring the neurodegenerative components of axonopathy and oligodendroglial apoptosis.

### SUMMARY

In one aspect, the present disclosure provides a method of treating a relapsing form of multiple sclerosis in a subject in need thereof. The method comprises administering a therapeutically effective amount of an antibody or antigen-binding fragment thereof that specifically binds Repulsive Guidance Molecule A (RGMa), wherein the antibody or antigen binding fragment comprises:

(a) a variable heavy chain comprising a complementarity determining region (CDR)-1 comprising an amino acid sequence of SEQ ID NO:2, a CDR-2 comprising an amino acid sequence of SEQ ID NO:3, and a CDR-3 comprising an amino acid sequence of SEQ ID NO:4; and

(b) a variable light chain comprising a CDR-1 comprising an amino acid sequence of SEQ ID NO:6, a CDR-2 comprising an amino acid sequence of SEQ ID NO:7, and a CDR-3 comprising an amino acid sequence of SEQ ID NO:8.

The relapsing form of multiple sclerosis treated pursuant to the above method can be relapsing remitting multiple sclerosis (RRMS) or relapsing-secondary progressive multiple sclerosis (SPMS).

In the above method, the antibody or antigen-binding fragment thereof can be administered to a subject in an amount of from about 50 mg to about 4000 mg, or in an amount of from about 50 mg to about 2500 mg.

More specifically, the antibody or antigen-binding fragment thereof can be administered to a subject in an amount of about 50 mg, 75 mg, 100 mg, 120 mg, 125 mg, 150 mg, 175 mg, 200 mg, 250 mg, 300 mg, 325 mg, 350 mg, 375 mg, 400 mg, 425 mg, 450 mg, 475 mg, 500 mg, 600 mg, 1000 mg, 1200 mg, 1600 mg, 1800 mg, 2400 mg, or 3600 mg.

Such antibody or antigen-binding fragment can be administered to a subject intravenously. Alternatively, such antibody or antigen-binding fragment can be administered to a subject subcutaneously.

The antibody or antigen-binding fragment employed in the above method has a variable heavy chain comprising an amino acid sequence of SEQ ID NO: 13 and the variable light chain comprising an amino acid sequence of SEQ ID NO: 14. Additionally, the antibody may be selected from the group consisting of a human antibody, an immunoglobulin molecule, a disulfide linked Fv, a monoclonal antibody, an affinity matured antibody, a scFv, a chimeric antibody, a CDR-grafted antibody, a diabody, a humanized antibody, a multispecific antibody, a Fab, a dual specific antibody, a DVD, a Fab', a bispecific antibody, a F(ab')$_2$, and a Fv. In one aspect, the antibody can be a human antibody. In another aspect, the antibody can be a monoclonal antibody. In yet another aspect, the antibody can be an affinity matured antibody. In still yet another aspect, the antibody can be a chimeric antibody. Still in yet a further aspect, the antibody is a humanized antibody. In yet another aspect, the antibody is a Fab, a Fab', a F(ab')$_2$ or Fv. In still a further aspect, the antibody is a dual specific antibody, a DVD or a bispecific antibody.

Additionally, the antibody employed in the above method can further comprise a constant sequence of SEQ ID NO: 12.

In certain aspects, the method disclosed herein comprises a multiple variable dose regimen, wherein the regimen comprises at least two phases. For example, a multiple variable dose regimen may comprise a first phase including administration of at least one loading dose of the antibody or antigen-binding fragment thereof followed by a subsequent phase including administration of at least one treatment dose that is less than the loading dose. The treatment dose can be an amount that is an amount that is at least 10% less, at least 20% less, at least 30% less, at least 40% less, at least 50% less, at least 60% less, at least 70% less, at least 80% less, at least 90% less than the loading dose.

In another aspect, the method disclosed herein further comprises administering an additional therapeutic agent to a subject. The additional therapeutic agent can be an immunosuppressant or an agent that treats one or more symptoms associated with multiple sclerosis. For example, the additional therapeutic agent can comprise an alpha or beta interferon (e.g., Avonex or Betaseron), a systemic corticosteroid such as methylprednisolone (Solu-Medrol) or prednisone (Deltasone), glatiramer (Copaxone), fingolimod (Gilenya), natalizumab (Tysabri), mitoxantrone (Novantrone), teriflunimide (Aubagio), BG-12 (Tecfidera), alemtuzumab (Lemtrada), daclizumab (Zinbryta), ocrelizumab (Ocrevus), amantadine (Symmetrel), amitriptyline (Elavil), nortriptyline, modafinil (Provigil), dalfampridine (Ampyra), a cognitive enhancing drug, an immunomodulatory drug, or a neuroprotective drug. The cognitive enhancing drug can comprise an acetylcholine receptor agonist, an acetylcholinesterase inhibitor, a butyrylcholinesterase inhibitor, an N-methyl-D-aspartate (NMDA) receptor antagonist, an activity-dependent neuroprotective protein (ADNP) agonist, a serotonin 5-HT1A receptor agonist, a 5-HT4 receptor agonist, a 5-HT6 receptor antagonist, a serotonin 1A receptor antagonist, a histamine H3 receptor antagonist, a calpain inhibitor, a vascular endothelial growth factor (VEGF) protein or agonist, a trophic growth factor, an anti-apoptotic compound, an AMPA-type glutamate receptor activator, a L-type or N-type calcium channel blocker or modulator, a potassium channel blocker, a hypoxia inducible factor (HIF) activator, a HIF prolyl 4-hydroxylase inhibitor, an anti-inflammatory agent, an inhibitor of amyloid A$\beta$ peptide or amyloid plaque, an inhibitor of tau hyperphosphorylation, a phosphodiesterase 5 inhibitor, a phosphodiesterase 4 inhibitor, a monoamine oxidase inhibitor, pharmaceutically acceptable salts thereof, or a combination thereof. For example, the cognitive enhancing drug can comprise donepezil (Aricept®), rivastigmine (Exelon®), galanthamine (Reminyl®), memantine (Namenda®), or a combination thereof. AE-12-1-Y-QL, as well as other anti-RGMa antibodies disclosed herein, may be used in combination with additional therapeutic agent. The anti-RGMa antibodies disclosed herein, including a fully human monoclonal antibody such as AE-12-1-Y-QL, represent new molecular entities with a specific, distinct, and novel mechanism of action from the aforementioned additional therapeutic agents. Moreover, AE-12-1-Y-QL does not induce cytochrome enzyme activity and, therefore, the chances of drug-drug-interaction are unlikely.

**BRIEF DESCRIPTION OF THE DRAWINGS**

Fig. 1 shows mean (+SD) (standard deviation) concentration-time profile of AE-12-1-Y-QL following a single dose at the amount and by the route indicated. The left panel is a linear scale and the right panel is a log scale.

Fig. 2 shows mean (+SD) concentration-time profile of 150 mg AE-12-1-Y-QL following a single dose administered by either the intravenous (IV) or subcutaneous (SC) route. The left panel is a linear scale and the right panel is a log scale.

Fig. 3 shows mean ($\pm$SD) dose-normalized $C_{max}$ and $AUC_{\infty}$ (area under the curve from time 0 to infinite time) following a single dose of AE-12-1-Y-QL.

Fig. 4 shows AE-12-1-Y-QL concentration in serum (ug/mL) and cerbrospinal fluid (ng/mL) at day 7 following administration of a single dose of AE-12-1-Y-QL at the amount indicated.

Fig. 5 shows bound RGMa levels (ng/mL) at baseline and at day 7 following administration of a single dose of AE-12-1-Y-QL at the amount and by the route indicated.

Fig. 6 shows free RGMa levels (ng/mL) at baseline and at day 7 following administration of a single dose of AE-12-1-Y-QL at the amount and by the route indicated.

Fig. 7 shows total RGMa levels (ng/mL) at baseline and at day 7 following administration of a single dose of AE-12-1-Y-QL at the amount and by the route indicated.

## DETAILED DESCRIPTION

Provided herein are methods of treating multiple sclerosis, particularly relapsing forms of multiple sclerosis (such as relapsing-remitting multiple sclerosis and relapsing-secondary progressive multiple sclerosis), by administering to a patient in need thereof a therapeutically effective amount of one or more anti-RGMa antibodies.

## 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of' and "consisting essentially of," the embodiments or elements presented herein, whether explicitly set forth or not.

"About" as used herein may refer to approximately a +/- 10% variation from the stated value. It is to be understood that such a variation is always included in any given value provided herein, whether or not specific reference is made to it.

"Affinity Matured Antibody" is used herein to refer to an antibody with one or more alterations in one or more CDRs, which result in an improvement in the affinity (i.e. $K_D$, $k_d$ or $k_a$) of the antibody for a target antigen compared to a parent antibody, which does not possess the alteration(s). Exemplary affinity matured antibodies will have nanomolar or even picomolar affinities for the target antigen. A variety of procedures for producing affinity matured antibodies are known in the art, including the screening of a combinatory antibody library that has been prepared using bio-display. For example, Marks et al., BioTechnology, 10: 779-783 (1992) describes affinity maturation by VH and VL domain shuffling. Random mutagenesis of CDR and/or framework residues is described by Barbas et al., Proc. Nat. Acad. Sci. USA, 91: 3809-3813 (1994); Schier et al., Gene, 169: 147-155 (1995); Yelton et al., J. Immunol., 155: 1994-2004 (1995); Jackson et al., J. Immunol., 154(7): 3310-3319 (1995); and Hawkins et al, J. Mol. Biol., 226: 889-896 (1992). Selective mutation at selective mutagenesis positions and at contact or hypermutation positions with an activity-enhancing amino acid residue is described in U.S. Pat. No. 6,914,128 B1.

"Antibody" and "antibodies" as used herein refers to monoclonal antibodies, multispecific antibodies, human antibodies, humanized antibodies (fully or partially humanized), animal antibodies such as, but not limited to, a bird (for example, a duck or a goose), a shark, a whale, and a mammal, including a non-primate (for example, a cow, a pig, a camel, a llama, a horse, a goat, a rabbit, a sheep, a hamster, a guinea pig, a cat, a dog, a rat, a mouse, etc.) or a non-human primate (for example, a monkey, a chimpanzee, etc.), recombinant antibodies, chimeric antibodies, single-chain Fvs ("scFv"), single chain antibodies, single domain antibodies, Fab fragments, F(ab') fragments, F(ab')$_2$ fragments, disulfide-linked Fvs ("sdFv"), and anti-idiotypic ("anti-Id") antibodies, dual-domain antibodies, dual variable domain (DVD) or triple variable domain (TVD) antibodies (dual-variable domain immunoglobulins and methods for making them are described in Wu, C., et al., Nature Biotechnology, 25(11):1290-1297 (2007) and PCT International Application WO 2001/058956, the contents of each of which are herein incorporated by reference), and functionally active epitope-binding fragments of any of the above. In particular, antibodies include immunoglobulin molecules and immunologically active fragments of immunoglobulin molecules, namely, molecules that contain an analyte-binding site. Immunoglobulin molecules can be of any type (for example, IgG, IgE, IgM, IgD, IgA and IgY), class (for example, IgG1, IgG2, IgG3, IgG4, IgA1 and IgA2) or subclass. For simplicity sake, an antibody against an analyte is frequently referred to herein as being either an "anti-analyte antibody," or merely an "analyte antibody" (e.g., an anti-RGMa antibody or an RGMa antibody).

"Antibody fragment" as used herein refers to a portion of an intact antibody comprising the antigen-binding site or variable region. The portion does not include the constant heavy chain domains (i.e. CH2, CH3 or CH4, depending on the antibody isotype) of the Fc region of the intact antibody. Examples of antibody fragments include, but are not limited to, Fab fragments, Fab' fragments, Fab'-SH fragments, F(ab')$_2$ fragments, Fd fragments, Fv fragments, diabodies, single-chain Fv (scFv) molecules, single-chain polypeptides containing only one light chain variable domain, single-chain polypeptides containing the three CDRs of the light-chain variable domain, single-chain polypeptides containing only one heavy chain variable region, and single-chain polypeptides containing the three CDRs of the heavy chain variable region.

"Bispecific antibody" is used herein to refer to a full-length antibody that is generated by quadroma technology (see Milstein et al., Nature, 305(5934): 537-540 (1983)), by chemical conjugation of two different monoclonal antibodies (see, Staerz et al., Nature, 314(6012): 628-631 (1985)), or by knob-into-hole or similar approaches, which introduce mutations in the Fc region (see Holliger et al., Proc. Natl. Acad. Sci. USA, 90(14): 6444-6448 (1993)), resulting in multiple different immunoglobulin species of which only one is the functional bispecific antibody. A bispecific antibody binds one antigen (or epitope) on one of its two binding arms (one pair of HC/LC), and binds a different antigen (or epitope) on its second arm (a different pair of HC/LC). By this definition, a bispecific antibody has two distinct antigen-binding arms (in both specificity and CDR sequences), and is monovalent for each antigen to which it binds.

"CDR" is used herein to refer to the "complementarity determining region" within an antibody variable sequence. There are three CDRs in each of the variable regions of the heavy chain and the light chain, which are designated "CDR1", "CDR2", and "CDR3", for each of the variable regions. The term "CDR set" as used herein refers to a group of three CDRs that occur in a single variable region that binds the antigen. The exact boundaries of these CDRs have been defined differently according to different systems. The system described by Kabat (Kabat et al., Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as "Kabat CDRs". Chothia and coworkers (Chothia and Lesk, J. Mol. Biol., 196: 901-917 (1987); and Chothia et al., Nature, 342: 877-883 (1989)) found that certain sub-portions within Kabat CDRs adopt nearly identical peptide backbone conformations, despite having great diversity at the level of amino acid sequence. These sub-portions were designated as "L1", "L2", and "L3", or "H1", "H2", and "H3", where the "L" and the "H" designate the light chain and the heavy chain regions, respectively. These regions may be referred to as "Chothia CDRs", which have boundaries that overlap with Kabat CDRs. Other boundaries defining CDRs overlapping with the Kabat CDRs have been described by Padlan, FASEB J., 9: 133-139 (1995), and MacCallum, J. Mol. Biol., 262(5): 732-745 (1996). Still other CDR boundary definitions may not strictly follow one of the herein systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although certain embodiments use Kabat- or Chothia-defined CDRs.

"Derivative" of an antibody as used herein may refer to an antibody having one or more modifications to its amino acid sequence when compared to a genuine or parent antibody and exhibit a modified domain structure. The derivative may still be able to adopt the typical domain configuration found in native antibodies, as well as an amino acid sequence, which is able to bind to targets (antigens) with specificity. Typical examples of antibody derivatives are antibodies coupled to other polypeptides, rearranged antibody domains or fragments of antibodies. The derivative may also comprise at least one further compound, e.g. a protein domain, said protein domain being linked by covalent or non-covalent bonds. The linkage can be based on genetic fusion according to the methods known in the art. The additional domain present in the fusion protein comprising the antibody employed in accordance with the invention may preferably be linked by a flexible linker, advantageously a peptide linker, wherein said peptide linker comprises plural, hydrophilic, peptide-bonded amino acids of a length sufficient to span the distance between the C-terminal end of the further protein domain and the N-terminal end of the antibody or vice versa. The antibody may be linked to an effector molecule having a conformation suitable for biological activity or selective binding to a solid support, a biologically active substance (e.g. a cytokine or growth hormone), a chemical agent, a peptide, a protein or a drug, for example.

"Dual-specific antibody" is used herein to refer to a full-length antibody that can bind two different antigens (or epitopes) in each of its two binding arms (a pair of HC/LC) (see PCT publication WO 02/02773). Accordingly a dual-specific binding protein has two identical antigen binding arms, with identical specificity and identical CDR sequences, and is bivalent for each antigen to which it binds.

"Dual variable domain" is used herein to refer to two or more antigen binding sites on a binding protein, which may be divalent (two antigen binding sites), tetravalent (four antigen binding sites), or multivalent binding proteins. DVDs may be monospecific, i.e., capable of binding one antigen (or one specific epitope), or multispecific, i.e., capable of binding two or more antigens (i.e., two or more epitopes of the same target antigen molecule or two or more epitopes of different target antigens). A preferred DVD binding protein comprises two heavy chain DVD polypeptides and two light chain DVD polypeptides and is referred to as a "DVD immunoglobulin" or "DVD-Ig". Such a DVD-Ig binding protein is thus tetrameric and reminiscent of an IgG molecule, but provides more antigen binding sites than an IgG molecule. Thus, each half of a tetrameric DVD-Ig molecule is reminiscent of one half of an IgG molecule and comprises a heavy chain DVD polypeptide and a light chain DVD polypeptide, but unlike a pair of heavy and light chains of an IgG molecule that provides a single antigen binding domain, a pair of heavy and light chains of a DVD-Ig provide two or more antigen binding sites.

Each antigen binding site of a DVD-Ig binding protein may be derived from a donor ("parental") monoclonal antibody and thus comprises a heavy chain variable domain (VH) and a light chain variable domain (VL) with a total of six CDRs involved in antigen binding per antigen binding site. Accordingly, a DVD-Ig binding protein that binds two different epitopes (i.e., two different epitopes of two different antigen molecules or two different epitopes of the same antigen molecule) comprises an antigen binding site derived from a first parental monoclonal antibody and an antigen binding site of a second parental monoclonal antibody.

A description of the design, expression, and characterization of DVD-Ig binding molecules is provided in PCT Publication No. WO 2007/024715, U.S. Pat. No. 7,612,181, and Wu et al., Nature Biotech., 25: 1290-1297 (2007). A preferred example of such DVD-Ig molecules comprises a heavy chain that comprises the structural formula VD1-(X1)n-VD2-C-(X2)n, wherein VD1 is a first heavy chain variable domain, VD2 is a second heavy chain variable domain, C is a heavy chain constant domain, X1 is a linker with the proviso that it is not CH1, X2 is an Fc region, and n is 0 or 1, but preferably 1; and a light chain that comprises the structural formula VD1-(X1)n-VD2-C-(X2)n, wherein VD1 is a first light chain variable domain, VD2 is a second light chain variable domain, C is a light chain constant domain, X1 is a linker with the proviso that it is not CH1, and X2 does not comprise an Fc region; and n is 0 or 1, but preferably 1. Such a DVD-Ig may comprise two such heavy chains and two such light chains, wherein each chain comprises variable domains linked in tandem without an intervening constant region between variable regions, wherein a heavy chain and a light chain associate to form tandem functional antigen binding sites, and a pair of heavy and light chains may associate with another pair of heavy and light chains to form a tetrameric binding protein with four functional antigen binding sites. In another example, a DVD-Ig molecule may comprise heavy and light chains that each comprise three variable domains (VD 1, VD2, VD3) linked in tandem without an intervening constant region between variable domains, wherein a pair of heavy and light chains may associate to form three antigen binding sites, and wherein a pair of heavy and light chains may associate with another pair of heavy and light chains to form a tetrameric binding protein with six antigen binding sites.

In an embodiment, a DVD-Ig binding protein according to the invention not only binds the same target molecules bound by its parental monoclonal antibodies, but also possesses one or more desirable properties of one or more of its parental monoclonal antibodies. For example, such an additional property is an antibody parameter of one or more of the parental monoclonal antibodies. Antibody parameters that may be contributed to a DVD-Ig binding protein from one or more of its parental monoclonal antibodies include, but are not limited to, antigen specificity, antigen affinity, potency, biological function, epitope recognition, protein stability, protein solubility, production efficiency, immunogenicity, pharmacokinetics, bioavailability, tissue cross reactivity, and orthologous antigen binding.

A DVD-Ig binding protein binds at least one epitope of RGMa. Non-limiting examples of a DVD-Ig binding protein include a DVD-Ig binding protein that binds one or more epitopes of RGMa, a DVD-Ig binding protein that binds an epitope of a human RGMa and an epitope of a RGMa of another species (for example, mouse), and a DVD-Ig binding protein that binds an epitope of a human RGMa and an epitope of another target molecule (for example, VEGFR2 or VEGFR1).

"Epitope," or "epitopes," or "epitopes of interest" refer to a site(s) on any molecule that is recognized and can bind to a complementary site(s) on its specific binding partner. The molecule and specific binding partner are part of a specific binding pair. For example, an epitope can be on a polypeptide, a protein, a hapten, a carbohydrate antigen (such as, but not limited to, glycolipids, glycoproteins or lipopolysaccharides), or a polysaccharide. Its specific binding partner can be, but is not limited to, an antibody.

"Framework" (FR) or "Framework sequence" as used herein may mean the remaining sequences of a variable region minus the CDRs. Because the exact definition of a CDR sequence can be determined by different systems (for example, see above), the meaning of a framework sequence is subject to correspondingly different interpretations. The six CDRs (CDR-L1, -L2, and -L3 of light chain and CDR-H1, -H2, and -H3 of heavy chain) also divide the framework regions on the light chain and the heavy chain into four sub-regions (FR1, FR2, FR3, and FR4) on each chain, in which CDR1 is positioned between FR1 and FR2, CDR2 between FR2 and FR3, and CDR3 between FR3 and FR4. Without specifying the particular sub-regions as FR1, FR2, FR3, or FR4, a framework region, as referred by others, represents the combined FRs within the variable region of a single, naturally occurring immunoglobulin chain. As used herein, a FR represents one of the four sub-regions, and FRs represents two or more of the four sub-regions constituting a framework region.

Human heavy chain and light chain FR sequences are known in the art that can be used as heavy chain and light chain "acceptor" framework sequences (or simply, "acceptor" sequences) to humanize a non-human antibody using techniques known in the art. In one embodiment, human heavy chain and light chain acceptor sequences are selected from the framework sequences listed in publicly available databases such as V-base or in the international ImMunoGeneTics® (IMGT®) information system.

"Functional antigen binding site" as used herein may mean a site on a binding protein (e.g. an antibody) that is capable of binding a target antigen. The antigen binding affinity of the antigen binding site may not be as strong as the parent binding protein, e.g., parent antibody, from which the antigen binding site is derived, but the ability to bind antigen must be measurable using any one of a variety of methods known for evaluating protein, e.g., antibody, binding to an antigen. Moreover, the antigen binding affinity of each of the antigen binding sites of a multivalent protein, e.g., multivalent antibody, herein need not be quantitatively the same.

"Human antibody" as used herein may include antibodies having variable and constant regions derived from human germline immunoglobulin sequences. The human antibodies described herein may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

"Humanized antibody" is used herein to describe an antibody that comprises heavy and light chain variable region sequences from a non-human species (e.g. a mouse) but in which at least a portion of the VH and/or VL sequence has been altered to be more "human-like," i.e., more similar to human germline variable sequences. A "humanized antibody" is an antibody or a variant, derivative, analog, or fragment thereof, which immunospecifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 80%, at least 85%, at least 90%, at least 95%, at least 98% or at least 99% identical to the amino acid sequence of a non-human antibody CDR. A humanized antibody comprises substantially all of at least one, and typically two, variable domains (Fab, Fab', F(ab')$_2$, FabC, Fv) in which all or substantially all of the CDR regions correspond to those of a non-human immunoglobulin (i.e., donor antibody) and all or substantially all of the framework regions are those of a human immunoglobulin consensus sequence. In an embodiment, a humanized antibody also comprises at least a portion of an immunoglobulin constant region (Fc), typically that of a human immunoglobulin. In some embodiments, a humanized antibody contains the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

A humanized antibody can be selected from any class of immunoglobulins, including IgM, IgG, IgD, IgA, and IgE, and any isotype, including without limitation IgG1, IgG2, IgG3, and IgG4. A humanized antibody may comprise sequences from more than one class or isotype, and particular constant domains may be selected to optimize desired effector functions using techniques well-known in the art.

The framework regions and CDRs of a humanized antibody need not correspond precisely to the parental sequences, e.g., the donor antibody CDR or the consensus framework may be mutagenized by substitution, insertion, and/or deletion of at least one amino acid residue so that the CDR or framework residue at that site does not correspond to either the donor antibody or the consensus framework. In a preferred embodiment, such mutations, however, will not be extensive. Usually, at least 80%, preferably at least 85%, more preferably at least 90%, and most preferably at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (see, e.g., Winnaker, From Genes to Clones (Verlagsgesellschaft, Weinheim, 1987)). A "consensus immunoglobulin sequence" may thus comprise a "consensus framework region(s)" and/or a "consensus CDR(s)". In a family of immunoglobulins, each position in the consensus sequence is occupied by the amino acid occurring most frequently at that position in the family. If two amino acids occur equally frequently, either can be included in the consensus sequence.

"Linking sequence" or "linking peptide sequence" refers to a natural or artificial polypeptide sequence that is connected to one or more polypeptide sequences of interest (e.g., full-length, fragments, etc.). The term "connected" refers to the joining of the linking sequence to the polypeptide sequence of interest. Such polypeptide sequences are preferably joined by one or more peptide bonds. Linking sequences can have a length of from about 4 to about 50 amino acids. Preferably, the length of the linking sequence is from about 6 to about 30 amino acids. Natural linking sequences can be modified by amino acid substitutions, additions, or deletions to create artificial linking sequences. Exemplary linking sequences include, but are not limited to: (i) Histidine (His) tags, such as a 6X His tag (SEQ ID NO: 15), which has an amino acid sequence of HHHHHH (SEQ ID NO: 15), are useful as linking sequences to facilitate the isolation and purification of polypeptides and antibodies of interest; (ii) Enterokinase cleavage sites, like His tags, are used in the isolation and purification of proteins and antibodies of interest. Often, enterokinase cleavage sites are used together with His tags in the isolation and purification of proteins and antibodies of interest. Various enterokinase cleavage sites are known in the art. Examples of enterokinase cleavage sites include, but are not limited to, the amino acid sequence of DDDDK (SEQ ID NO: 16) and derivatives thereof (e.g., ADDDDK (SEQ ID NO: 17), etc.); (iii) Miscellaneous sequences can be used to link or connect the light and/or heavy chain variable regions of single chain variable region fragments. Examples of other linking sequences can be found in Bird et al., Science 242: 423-426 (1988); Huston et al., PNAS USA 85: 5879-5883 (1988); and McCafferty et al., Nature 348: 552-554 (1990). Linking sequences also can be modified for additional functions, such as attachment of drugs or attachment to solid supports. In the context of the present disclosure, the monoclonal antibody, for example, can contain a linking sequence, such as a His tag, an enterokinase cleavage site, or both.

"Multiple variable dose regimen" refers to a treatment schedule or regimen that includes administration of different doses of an anti-RGMa antibody or antigen-binding fragment thereof at various time points throughout the course of treatment. For example, a multiple variable dose regimen may comprise a loading dose administered at a first time and one or more treatment doses administered thereafter. In one embodiment, the loading dose is a higher dose than the subsequent treatment dose(s).

"Loading dose" as used herein refers to the first dose(s) of an anti-RGMa antibody or antigen-binding fragment thereof that is initially used to treat a relapsing form of multiple sclerosis in a subject. The loading dose may be larger in comparison to the subsequent treatment dose. The loading dose can be a single dose or, alternatively, a set of doses. For example, a 3600 mg dose may be administered as a single 3600 mg dose, as two doses of 1800 mg each, or four doses of 900 mg each. In one embodiment, a loading dose is subsequently followed by administration of smaller doses, e.g., the treatment dose(s).

"Treatment dose" as used herein refers to subsequent dose(s) of an anti-RGMa antibody or antigen-binding fragment thereof that is administered to a subject after a loading dose. A treatment dose is administered to a subject to maintain or continue a desired therapeutic effect. A treatment dose can be a single dose or, alternatively, a set of doses. In one embodiment, a treatment dose(s) is smaller than the loading dose(s) and each treatment dose may be equal to each other when administered in succession.

The term "Columbia-Suicide Severity Rating Scale" or "C-SSRS" as used interchangeably herein refers to a systematically administered instrument developed to track suicidal adverse events across a treatment study. The instrument is designed to assess suicidal behavior and ideation, track and assess all suicidal events, as well as the lethality of attempts. Additional features assessed include frequency, duration, controllability, reason for ideation, and deterrents. The C-SSRS is considered a low-burden instrument as it takes less than 5 minutes to administer.

The term "Expanded Disability Status Scale (EDSS)" or "EDSS" as used interchangeably herein refers to a standardized rating scale using an ordered (ordinal) rating scale requiring human assessment. The EDSS quantifies disability in eight Functional Systems (FS): pyramidal, cerebellar, brainstem, sensory, bowel and bladder, visual, cerebral and other. The EDSS allows neurologists to assign a Functional System Score (FSS) in each of these.

The term "Multiple Sclerosis Functional Composite" or "MFSC" as used interchangeably herein refers to a performance measure that uses standardized procedures for testing human function, and consists of the Timed 25 Foot Walk (T25FW); the 9 Hole Peg Test (9HPT); and the Paced Auditory Serial Arithmetic Test (PASAT). Due to certain MSFC limitations, i.e., its abstract and dimensionless nature of the summary score, the fact that many clinicians are unfamiliar with z scores, and because the reference population affects the absolute values for the components and their weighting, the MSFC scores are not easily interpreted clinically or compared across studies. An alternative analytical approach will be used to define worsening as an increase in score by a pre-specified amount in any of the component tests and can be determined reliably and has clinical relevance (e.g., 20%), and can demonstrate worsening in the same component at two sequential time points

The term "Multiple Sclerosis Impact Scale, Physical", "MSIS-29 PHYS" or "MSIS-29"as used interchangeably herein refers to a disease-specific, patient-reported outcome measure used to evaluate the physical and psychological impact of MS. The MSIS-29 includes the 20-item physical impact subscale (MSIS-29 PHYS) and the 9-item psychological impact subscale (MSIS-29 PSYCH). A $\geq$7.50-point worsening from baseline in the MSIS-29 PHYS has been shown to be clinically meaningful in a clinical study population. The proportion of patients with a clinically meaningful worsening in the patient-reported physical impact of MS ($\geq$ 7.5-point worsening on MSIS-29 PHYS) with be assessed at serial time points over the course of the study.

The term "Multiple Sclerosis Quality of Life-54" or "MSQOL-54" as used interchangeably herein refers to a multidimensional health-related quality of life measure that combines both generic and MS-specific items into a single instrument. This 54-item instrument generates 12 subscales along with two summary scores, and two additional single-item measures. The subscales are: physical function, role limitations-physical, role limitations-emotional, pain, emotional well-being, energy, health perceptions, social function, cognitive function, health distress, overall quality of life, and sexual function. The summary scores are the physical health composite summary and the mental health composite summary. The single item measures are satisfaction with sexual function and change in health.

"Multiple Sclerosis" (MS) refers to the chronic and often disabling disease of the central nervous system characterized by the progressive destruction of the myelin. There are four internationally recognized forms of MS, namely, primary progressive multiple sclerosis (PPMS), relapsing-remitting multiple sclerosis (RRMS), secondary progressive multiple sclerosis (SPMS), and relapsing-secondary progressive multiple sclerosis (RSPMS). Relapsing forms of MS include relapsing-remitting multiple sclerosis and relapsing-secondary progressive multiple sclerosis.

"Relapsing-remitting multiple sclerosis" or "RRMS" is a relapsing form of multiple sclerosis characterized by clearly defined disease relapses (also known as exacerbations) with full recovery or with sequelae and residual deficit upon recovery periods between disease relapses characterized by a lack of disease progression. The defining elements of RRMS are episodes of acute worsening of neurologic function followed by a variable degree of recovery, with a stable course between attacks (Lublin, F. D. & Reingold, S. O, Neurology (46) 907-911 (1996)). Relapses can last for days, weeks or months and recovery can be slow and gradual or almost instantaneous. The vast majority of subjects presenting with MS are first diagnosed with RRMS. This is typically when they are in their twenties or thirties, though diagnoses occurring much earlier or later are known. Twice as many women as men present with this sub-type of MS. During relapses, myelin, a protective insulating sheath around the nerve fibers (neurons) in the white matter regions of the central nervous system (CNS), may be damaged in an inflammatory response by the body's own immune system. This causes a wide variety of neurological symptoms that vary considerably depending on which areas of the CNS are damaged. Immediately after a relapse, the inflammatory response dies down and a special type of glial cell in the CNS (called an oligodendrocyte) sponsors remyelination--a process whereby the myelin sheath around the axon may be repaired. It is this remyelination that may be responsible for the remission.

"Primary progressive multiple sclerosis" or "PPMS" occurs after the relapsing-remitting disease course (RRMS). Of the 85 percent of people who are initially diagnosed with RRMS, most will eventually transition to SPMS, which means that after a period of time in which they experience relapses and remissions, the disease will begin to progress more steadily (although not necessarily more quickly), with or without any relapses (also called attacks or exacerbations). At any one time, SPMS accounts for approximately 30% of all subjects with multiple sclerosis. The natural history of MS indicates that 50 percent of those diagnosed with RRMS would transition to secondary-progressive MS (SPMS) within 10 years, and 90 percent would transition within 25 years.

The distinguishing transition from RRMS to SPMS occurs when, independent of relapses, there is progressive worsening of neurological function. In SPMS, people may or may not continue to experience relapses caused by inflammation; the disease gradually changes from the inflammatory process seen in RRMS to a more steadily progressive phase characterized by nerve damage or loss. "Relapsing-secondary progressive multiple sclerosis", or "relapsing-SPMS", comprises those subjects during the early stages after transitioning to SPMS that still exhibit features of relapse activity and inflammation, as documented on neuroimaging studies as new T1 gadolinium enhancing lesions or new or newly enlarging T2 lesions on brain or spinal cord MRI.

"Multivalent binding protein" is used herein to refer to a binding protein comprising two or more antigen binding sites (also referred to herein as "antigen binding domains"). A multivalent binding protein is preferably engineered to have three or more antigen binding sites, and is generally not a naturally occurring antibody. The term "multispecific binding protein" refers to a binding protein that can bind two or more related or unrelated targets, including a binding protein capable of binding two or more different epitopes of the same target molecule.

"Recombinant antibody" and "recombinant antibodies" refer to antibodies prepared by one or more steps, including cloning nucleic acid sequences encoding all or a part of one or more monoclonal antibodies into an appropriate expression vector by recombinant techniques and subsequently expressing the antibody in an appropriate host cell. The terms include, but are not limited to, recombinantly produced monoclonal antibodies, chimeric antibodies, humanized antibodies (fully or partially humanized), multi-specific or multi-valent structures formed from antibody fragments, bifunctional antibodies, heteroconjugate Abs, DVD-Ig's, and other antibodies as described in (i) herein. (Dual-variable domain immunoglobulins and methods for making them are described in Wu, C., et al., Nature Biotechnology, 25:1290-1297 (2007)). The term "bifunctional antibody," as used herein, refers to an antibody that comprises a first arm having a specificity for one antigenic site and a second arm having a specificity for a different antigenic site, i.e., the bifunctional antibodies have a dual specificity.

"Specific binding" or "specifically binding" as used herein may refer to the interaction of an antibody, a protein, or a peptide with a second chemical species, wherein the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

"Treat", "treating" or "treatment" are each used interchangeably herein to describe reversing, alleviating, or inhibiting the progress of a disease, or one or more symptoms of such disease, to which such term applies. A treatment may be either performed in an acute or chronic way. The term also refers to reducing the severity of a disease or symptoms associated with such disease prior to affliction with the disease. Such reduction of the severity of a disease prior to affliction refers to administration of an antibody or pharmaceutical composition described herein to a subject that is not at the time of administration afflicted with the disease. "Treatment" and "therapeutically," refer to the act of treating, as "treating" is defined above.

"Variant" is used herein to describe a peptide or polypeptide that differs in amino acid sequence by the insertion, deletion, or conservative substitution of amino acids, but retain at least one biological activity. Representative examples of "biological activity" include the ability to be bound by a specific antibody or to promote an immune response. Variant is also used herein to describe a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art. Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of $\pm 2$ are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. U.S. Patent No. 4,554,101, incorporated herein by reference. Substitution of amino acids having similar hydrophilicity values can result in peptides retaining biological activity, for example immunogenicity, as is understood in the art. Substitutions may be performed with amino acids having hydrophilicity values within $\pm 2$ of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties. "Variant" also can be used to refer to an antigenically reactive fragment of an anti-RGMa antibody that differs from the corresponding fragment of anti-RGMa antibody in amino acid sequence but is still antigenically reactive and can compete with the corresponding fragment of anti-RGMa antibody for binding with RGMa. "Variant" also can be used to describe a polypeptide or a fragment thereof that has been differentially processed, such as by proteolysis, phosphorylation, or other post-translational modification, yet retains its antigen reactivity.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

**2. Anti-RGMa antibodies**

Provided herein are methods of treating multiple sclerosis, particularly relapsing forms of multiple sclerosis (such as relapsing-remitting multiple sclerosis and relapsing-secondary progressive multiple sclerosis) by administering to a patient in need thereof one or more anti-RGMa antibodies. The anti-RGMa antibodies for use in the methods described herein bind to RGMa, while minimizing or eliminating reactivity with Repulsive Guidance Molecule c ("RGMc"). Because antibodies raised against RGMa can often cross-react with RGMc and, at high intravenous doses may result in iron accumulation in hepatocytes, the specific binding of the herein described antibodies for RGMa is of therapeutic benefit. Further, the high selectivity of these antibodies offers large therapeutic dose windows or ranges for treatment.

RGMa exists in membrane-bound and soluble forms. RGMa plays a role in neural tube development and, in addition, has a role in cell adhesion, cell migration, cell polarity, and cell differentiation, which together influence early morphogenetic events. RGMa also inhibits neuroregeneration. The effects of RGMa (both membrane-bound and soluble forms) may be mediated by neogenin and/or bone morphogenetic protein (BMP) receptors. For example, the RGMa-BMP interaction may potentiate neurite growth inhibition (e.g., through neuronal BMP receptors) and may inhibit remyelination (e.g., through glial BMP receptors). As another example, the RGMa-neogenin interaction may inhibit axonal growth.

In certain embodiments, the anti-RGMa antibodies provided herein are capable of binding to and neutralizing human RGMa. In certain embodiments, the anti-RGMa antibodies provided herein possess a unique combination of neuroregenerative and neurorestorative properties and demonstrate axon regeneration, neuroprotection, and remyelination in animal models. In view of the pleiotropic role of RGMa, it is desirable to establish a margin of safety with anti-RGMa antibodies in mammals, and, in particular, humans.

**a. RGMa-Recognizing Antibody**

An antibody that can be used to treat patients with multiple sclerosis, particularly relapsing forms of multiple sclerosis (such as relapsing-remitting multiple sclerosis and relapsing-secondary progressive multiple sclerosis), is an antibody that binds to RGMa, a fragment or variant thereof. The antibody may be a fragment of the anti-RGMa antibody or a variant or a derivative thereof. The antibody may be a polyclonal or monoclonal antibody. The antibody may be a chimeric antibody, a single chain antibody, an affinity matured antibody, a human antibody, a humanized antibody, a fully human antibody or an antibody fragment, such as a Fab fragment, or a mixture thereof. Antibody fragments or derivatives may comprise F(ab')$_2$, Fv or scFv fragments. The antibody derivatives can be produced by peptidomimetics. Further, techniques described for the production of single chain antibodies can be adapted to produce single chain antibodies.

Human antibodies may be derived from phage-display technology or from transgenic mice that express human immunoglobulin genes. The human antibody may be generated as a result of a human in vivo immune response and isolated. See, for example, Funaro et al., BMC Biotechnology, 2008(8):85. Therefore, the antibody may be a product of the human and not animal repertoire. Because it is of human origin, the risks of reactivity against self-antigens may be minimized. Alternatively, standard yeast display libraries and display technologies may be used to select and isolate human anti-RGMa antibodies. For example, libraries of naive human single chain variable fragments (scFv) may be used to select human anti-RGMa antibodies. Transgenic animals may be used to express human antibodies.

Humanized antibodies may be antibody molecules from non-human species antibody that binds the desired antigen having one or more complementarity determining regions (CDRs) from the non-human species and framework regions from a human immunoglobulin molecule.

The antibody may specifically bind to RGMa. The RGMa-specific RGMa antibody may comprise SEQ ID NOs: 2-4 and 6-8, SEQ ID NOs: 13-14 or SEQ ID Nos: 2-4, 6-8 and 13-14. The antibody may bind to SEQ ID NO: 18, SEQ ID NO: 19, SEQ ID NO: 20, or a fragment or variant thereof. The antibody may recognize and specifically bind an epitope present on a RGMa polypeptide or a variant as described above. The epitope may be SEQ ID NO: 18 (full-length human RGMa), SEQ ID NO: 19 (a human RGMa fragment which corresponds to amino acids 47-168 of SEQ ID NO: 18), SEQ ID NO:20 (a human RGMa fragment), or a variant thereof, the sequences of which are provided below:

MQPPRERLVVTGRAGWmgmgrgagrsalgfwptlafllcsfpaatspckilkcnsefwsatsgshapasdd tpefcaalrsyalctrrtartcrgdlayhsavhgiedlmsqhncskdgptsqprlrtlppagdsqersdspeichyeksfhkhsatpnythc glfgdphlrtftdrfqtckvqgawplidnnylnvqvtntpvlpgsaatatskltiifknfqecvdqkvyqaemdelpaafvdgsknggdk hganslkitekvsgqhveiqakyigttivvrqvgryltfavrmpeevvnavedwdsqglylclrgcplnqqidfqafhtnaegtgarrla aaspaptapetfpyetavakckeklpvedlyyqacvfdllttgdvnftlaayyaledvkmlhsnkdklhlyertrdlpgraaaglplaprp llgalvpllallpvfc (SEQ ID NO: 18)

pckilkcnsefwsatsgshapasddtpefcaalrsyalctrrtartcrgdlayhsavhgiedlmsqhncskdgptsqprlrtl ppagdsqersdspeichyeksfhkhsatpnythcglfgd (SEQ ID NO: 19)

PCKII,KCNSEFWSATSGSHAPAS (SEQ ID NO: 20).

**(1) Antibody Structure**

**(a) Heavy Chain and Light Chain CDRs**

The antibody may immunospecifically bind to RGMa (SEQ ID NO: 18), SEQ ID NO: 19, SEQ ID NO: 20, a fragment thereof, or a variant thereof and comprise a variable heavy chain and/or variable light chain shown in Table 1. The antibody may immunospecifically bind to RGMa, a fragment, derivative, or a variant thereof and comprise one or more of the heavy chain or light chain CDR sequences also shown in Table 1. The light chain of the antibody may be a kappa chain or a lambda chain. For example, see Table 1. Methods for making the antibodies shown in Table 1 are described in WO 2013/112922, the contents of which are herein incorporated by reference.

**Table 1 List of Amino Acid Sequences of VH and VL Regions of Fully Human Anti-RGMa Monoclonal Antibody -AE12-1-Y-QL**

| PROTEIN REGION | SEQ ID NO. | SEQUENCE |
|---|---|---|
| AE12-1-Y-QL (VH) | 1 | EVQLVQSGAEVKKPGASVKVSCKAS GYTFTSHGISWVRQAPGQGLDWMG WISPYSGNTNYAQKLQGRVTMTTD TSTSTAYMELSSLRSEDTAVYYCAR VGSGPYYYMDVWGQGTLVTVSS |
| AE12-1-Y-QL (VH) CDR-H1 | 2 | SHGIS |
| AE12-1-Y-QL (VH) CDR-H2 | 3 | WISPYSGNTNYAQKLQG |
| AE12-1-Y-QL (VH) CDR-H3 | 4 | VGSGPYYYMDV |
| AE12-1-Y-QL (VL) (Lambda chain) | 5 | QSALTQPRSVSGSPGQSVTISCTG TSSSVGDSIYVSWYQQHPGKAPK LMLYDVTKRPSGVPDRFSGSKSG NTASLTISGLQAEDEADYYCYSY AGTDTLFGGGTKVTVL |
| AE12-1-Y-QL (VH) CDR-L1 | 6 | TGTSSSVGDSIYVS |
| AE12-1-Y-QL (VH) CDR-L2 | 7 | DVTKRPS |
| AE12-1-Y-QL (VH) CDR-L3 | 8 | YSYAGTDTL |

The antibody or variant or derivative thereof may contain one or more amino acid sequences that are greater than 95%, 90%, 85%, 80%, 75%, 70%, 65%, 60%, 55%, or 50% identical to one or more of SEQ ID NOs: 1-8 or 13-14. The antibody or variant or derivative thereof may be encoded by one or more nucleic acid sequences that are greater than 95%, 90%, 85%, 80%, 75%, 70%, 65%, 60%, 55%, or 50% identical to one or more of SEQ ID NOs: 1-8 or 13-14. Polypeptide identity and homology can be determined, for example, by the algorithm described in the report: Wilbur, W. J. and Lipman, D. J. Proc. Natl. Acad. Sci. USA 80, 726-730 (1983).

The antibody may be an IgG, IgE, IgM, IgD, IgA and IgY molecule class (for example, IgG1, IgG2, IgG3, IgG4, IgA1 and IgA2) or subclass. For example, the antibody may be an IgG1 molecule having the following constant region sequence:

ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPE**AA**GG PSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSR

EEMTKNQVSLTCLVKGFYPSDIAVEWESNGQPENNYKTTPPVLDSDGSFFLYSKLTVDK

SRWQQGNVFSCSVMHEALHNHYTQKSLSLSPGK (SEQ ID NO:9).

The above constant region in SEQ ID NO: 9 contains two (2) mutations of the wildtype constant region sequence at positions 234 and 235. Specifically, these mutations are leucine to alanine changes at each of positions 234 and 235 (which are referred to as the "LLAA" mutations). These mutations are shown above in bold and underlining. The purpose of these mutations is to eliminate the effector function.

Alternatively, an IgG1 molecule can have the above constant region sequence (SEQ ID NO: 9) containing one or more mutations. For example, the constant region sequence of SEQ ID NO: 9 may containing a mutation at amino acid 250 where threonine is replaced with glutamine (SEQ ID NO: 10), a mutation at amino acid 428 where methionine is replaced with leucine (SEQ ID NO: 11) or mutations at amino acid 250 where threonine is replaced with glutamine and a mutation at amino acid 428 where methionine is replaced with leucine (SEQ ID NO: 12) as shown below in Table 2.

**Table 2**

| Amino acid Mutation | SEQ ID NO: | SEQUENCE |
|---|---|---|
| None | 9 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKD**T**LMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSV**M**HEALHNHYTQKSLSLSPGK |
| T250Q | 10 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKD**Q**LMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSV**M**HEALHNHYTQKSLSLSPGK |
| M428L | 11 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKD**T**LMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSV**L**HEALHNHYTQKSLSLSPGK |

(continued)

| Amino acid Mutation | SEQ ID NO: | SEQUENCE |
|---|---|---|
| T250Q and M428L | 12 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPEAAGGPSVFLFP PKPKD**Q**LMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSV**L**HEALHNHYTQKSLSLSPGK |

Alternatively, an IgG1 molecule can contain a heavy chain comprising: AE12-1 (VH) CDR-H1 (SEQ ID NO: 2), AE12-1 (VH) CDR-H2 (SEQ ID NO: 3), AE12-1 (VH) CDR-H3 (SEQ ID NO: 4) and a light chain comprising: AE12-1 (VL) CDR-L1 (SEQ ID NO: 6), AE12-1 (VL) CDR-L2 (SEQ ID NO: 7) and AE12-1-Y (VL) CDR-L3 (SEQ ID NO: 8) and a constant sequence of SEQ ID NO: 12 as shown below in Table 3 (this antibody is referred to as AE12-1-Y-QL and has a light chain sequence of SEQ ID NO: 13 and a heavy chain sequence of SEQ ID NO: 14).

**Table 3**

| PROTEIN REGION | SEQ ID NO: | SEQUENCE |
|---|---|---|
| AE12-1-Y-QL Light chain (CDRs underlined and mutations bolded) | 13 | QSALTQPRSVSGSPGQSVTISCTGTSSSVGDSIYVSWYQQHPGKA PKLMLYDVTKRPSGVPDRFSGSKSGNTASLTISGLQAEDEADYYC **Y**SYAGTDTLFGGGTKVTVLGQPKAAPSVTLFPPSSEELQANKATL VCLISDFYPGAVTVAWKADSSPVKAGVETTTPSKQSNNKYAASSY LSLTPEQWKSHRSYSCQVTHEGSTVEKTVAPTECS* |
| AE12-1-Y-QL Heavy chain (CDRs underlined and mutations bolded) | 14 | EVQLVQSGAEVKKPGASVKVSCKASGYTFTSHGISWVRQAPGQGL DWMGWISPYSGNTNYAQKLQGRVTMTTDTSTSTAYMELSSLRSED TAVYYCARVGSGPYYYMDVWGQGTLVTVSSASTKGPSVFPLAPSS KSTSGGTAALGCLVKDYFPEPVTVSWNSGALTSGVHTFPAVLQSS GLYSLSSVVTVPSSSLGTQTYICNVNHKPSNTKVDKKVEPKSCDK THTCPPCPAPEAAGGPSVFLFPPKPKD**Q**LMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPSREE MTKNQVSLTCLVKGFYPSDIAVEWESNGQPENNYKTTPPVLDSDG SFFLYSKLTVDKSRWQQGNVFSCSV**L**HEALHNHYTQKSLSLSPGK * |

## 3. Pharmaceutical Compositions

The antibody may be a component in a pharmaceutical composition. The pharmaceutical composition may also contain a pharmaceutically acceptable carrier. The pharmaceutical compositions comprising antibodies described herein are for use in treating multiple sclerosis, particularly relapsing forms of multiple sclerosis (such as relapsing-remitting multiple sclerosis and relapsing-secondary progressive multiple sclerosis). In a specific embodiment, a composition comprises one or more antibodies described herein. In another embodiment, the pharmaceutical composition comprises one or more antibodies described herein and one or more prophylactic or therapeutic agents other than antibodies described herein for treating multiple sclerosis, particularly, relapsing forms of multiple sclerosis (such as relapsing-remitting multiple sclerosis and relapsing-secondary progressive multiple sclerosis). In a further embodiment, the prophylactic or therapeutic agents are known to be useful for, or have been, or are currently being used in the prevention, treatment, management, or amelioration of multiple sclerosis, or one or more symptoms thereof. In accordance with these embodiments, the composition may further comprise of a carrier, diluent or excipient.

The antibodies described herein can be incorporated into pharmaceutical compositions suitable for administration to a subject. Typically, the pharmaceutical composition comprises an antibody described herein (such as, for example, AE-12-1-Y-QL) and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody.

In a further embodiment, the pharmaceutical composition comprises at least one additional therapeutic agent for treating multiple sclerosis, particularly relapsing forms of multiple sclerosis (such as relapsing-remitting multiple sclerosis and relapsing-secondary progressive multiple sclerosis) as described herein.

Various delivery systems are known and can be used to administer one or more antibodies described herein or the combination of one or more antibodies described herein and a prophylactic agent or therapeutic agent useful for preventing, managing, treating, or ameliorating multiple sclerosis, such as relapsing forms of multiple sclerosis (such as relapsing-remitting multiple sclerosis and relapsing-secondary progressive multiple sclerosis), or one or more symptoms thereof, e.g., encapsulation in liposomes, microparticles, microcapsules, recombinant cells capable of expressing the antibody or antibody fragment, receptor-mediated endocytosis (see, e.g., Wu and Wu, J. Biol. Chem. 262:4429-4432 (1987)), construction of a nucleic acid as part of a retroviral or other vector, etc. Methods of administering a prophylactic or therapeutic agent include, but are not limited to, parenteral administration (e.g., intradermal, intramuscular, intraperitoneal, intravenous, intrathecal and subcutaneous), epidural administration, intratumoral administration, and mucosal administration (e.g., intranasal and oral routes). In addition, pulmonary administration can be employed, e.g., by use of an inhaler or nebulizer, and formulation with an aerosolizing agent. See, e.g., U.S. Pat. Nos. 6,019,968; 5,985,320; 5,985,309; 5,934,272; 5,874,064; 5,855,913; 5,290,540; and 4,880,078; and PCT Publication Nos. WO 92/19244; WO97/32572; WO97/44013; WO98/31346; and WO99/66903, each of which is incorporated herein by reference in their entireties. In one embodiment, an antibody described herein, combination therapy, or a composition described herein is administered using Alkermes AIR® pulmonary drug delivery technology (Alkermes, Inc., Cambridge, Mass.). In a specific embodiment, prophylactic or therapeutic agents of the antibodies described herein are administered intramuscularly, intravenously, intratumorally, orally, intranasally, pulmonary, or subcutaneously. The prophylactic or therapeutic agents may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

In a specific embodiment, it may be desirable to administer the antibodies described herein locally to the area in need of treatment; this may be achieved by, for example, and not by way of limitation, local infusion, by injection, or by means of an implant, said implant being of a porous or non-porous material, including membranes and matrices, such as sialastic membranes, polymers, fibrous matrices (e.g., Tissuel®), or collagen matrices. In one embodiment, an effective amount of one or more antibodies described herein is administered locally to the affected area to a subject to prevent, treat, manage, and/or ameliorate a disorder or a symptom thereof. In another embodiment, an effective amount of one or more antibodies described herein is administered locally to the affected area in combination with an effective amount of one or more therapies (e.g., one or more prophylactic or therapeutic agents) other than an antibody described herein to a subject to prevent, treat, manage, and/or ameliorate a disorder or one or more symptoms thereof.

In another embodiment, the antibody can be delivered in a controlled release or sustained release system. In one embodiment, a pump may be used to achieve controlled or sustained release (see Langer, supra; Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the therapies described herein (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Pat. No. 5,679,377; U.S. Pat. No. 5,916,597; U. S. Pat. No. 5,912,015; U.S. Pat. No. 5,989,463; U.S. Pat. No. 5,128,326; PCT Publication No. WO99/15154; and PCT Publication No. WO99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N- vinyl pyrrolidone), poly(vinyl alcohol), polyacrylamide, polyethylene glycol), polylactides (PLA), poly(lactide-co-glycolides) (PLGA), and polyorthoesters. In a particular embodiment, the polymer used in a sustained release formulation is inert, free of leachable impurities, stable on storage, sterile, and biodegradable. In yet another embodiment, a controlled or sustained release system can be placed in proximity of the prophylactic or therapeutic target, thus requiring only a fraction of the systemic dose (see, e.g., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more antibodies described herein. See, e.g., U. S. Pat. No. 4,526, 938, PCT publication WO91/05548, PCT publication WO96/20698, Ning et al., 1996, "Intratumoral Radioimmunotheraphy of a Human Colon Cancer Xenograft Using a Sustained-Release Gel," Radiotherapy &Oncology 39:179-189; Song et al., 1995, "Antibody Mediated Lung Targeting of Long- Circulating Emulsions," PDA Journal of Pharmaceutical Science & Technology 50:372-397; Cleek et al., 1997, "Biodegradable Polymeric Carriers for a bFGF Antibody for Cardiovascular Application," Pro. Int'l. Symp. Control. Rel. Bioact. Mater. 24:853-854; and Lam et al., 1997, "Microencapsulation of Recombinant Humanized Monoclonal Antibody for Local Delivery," Proc. Int'l. Symp. Control Rel. Bioact. Mater. 24:759- 760, each of which is incorporated herein by reference in their entireties.

A pharmaceutical composition is formulated to be compatible with its intended route of administration. Examples of routes of administration include, but are not limited to, parenteral, e.g., intravenous, intrathecal, intradermal, subcutaneous, oral, intranasal (e.g., inhalation), transdermal (e.g., topical), transmucosal, and rectal administration. In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

The method described herein may comprise administration of a composition formulated for parenteral administration by injection (e.g., by bolus injection or continuous infusion). Formulations for injection may be presented in unit dosage form (e.g., in ampoules or in multidose containers) with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle (e.g., sterile pyrogen-free water) before use. The methods described herein may additionally comprise of administration of compositions formulated as depot preparations. Such long acting formulations may be administered by implantation (e.g., subcutaneously, intrathecally or intramuscularly) or by intramuscular injection. Thus, for example, the compositions may be formulated with suitable polymeric or hydrophobic materials (e.g., as an emulsion in an acceptable oil) or ion exchange resins, or as sparingly soluble derivatives (e.g., as a sparingly soluble salt).

The methods described herein encompass administration of compositions formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Generally, the ingredients of compositions are supplied either separately or mixed together in unit dosage form, for example, as a dry lyophilized powder or water free concentrate in a hermetically sealed container such as an ampoule or sachette indicating the quantity of active agent. Where the mode of administration is infusion, composition can be dispensed with an infusion bottle containing sterile pharmaceutical grade water or saline. Where the mode of administration is by injection, an ampoule of sterile water for injection or saline can be provided so that the ingredients may be mixed prior to administration.

In particular, the methods described herein also contemplate that one or more of the antibodies or pharmaceutical compositions described herein are packaged in a hermetically sealed container such as an ampoule or sachette indicating the quantity of the antibody. In one embodiment, one or more of the antibodies, or pharmaceutical compositions described herein are supplied as a dry sterilized lyophilized powder or water free concentrate in a hermetically sealed container and can be reconstituted (e.g., with water or saline) to the appropriate concentration for administration to a subject. In one embodiment, one or more of the antibodies or pharmaceutical compositions described herein are supplied as a dry sterile lyophilized powder in a hermetically sealed container at a unit dosage of at least 5 mg, for example at least 10 mg, at least 15 mg, at least 25 mg, at least 35 mg, at least 45 mg, at least 50 mg, at least 75 mg, or at least 100 mg. The lyophilized antibodies or pharmaceutical compositions described herein should be stored at between 2°C and 8°C. in its original container and the antibodies, or pharmaceutical compositions described herein should be administered within 1 week, for example within 5 days, within 72 hours, within 48 hours, within 24 hours, within 12 hours, within 6 hours, within 5 hours, within 3 hours, or within 1 hour after being reconstituted. In an alternative embodiment, one or more of the antibodies or pharmaceutical compositions described herein is supplied in liquid form in a hermetically sealed container indicating the quantity and concentration of the antibody. In a further embodiment, the liquid form of the administered composition is supplied in a hermetically sealed container at least 0.25 mg/ml, for example at least 0.5 mg/ml, at least 1 mg/ml, at least 2.5 mg/ml, at least 5 mg/ml, at least 8 mg/ml, at least 10 mg/ml, at least 15 mg/ml, at least 25 mg/ml, at least 50 mg/ml, at least 75 mg/ml or at least 100 mg/ml. The liquid form should be stored at between 2°C and 8°C in its original container.

The antibodies described herein can be incorporated into a pharmaceutical composition suitable for parenteral administration. In one aspect, antibodies will be prepared as an injectable solution containing 0.1-500 mg/ml antibody. The injectable solution can be composed of either a liquid or lyophilized dosage form in a flint or amber vial, ampule or pre-filled syringe. The buffer can be L-histidine (1-50 mM), optimally 5-10 mM, at pH 5.0 to 7.0 (optimally pH 6.0). Other suitable buffers include but are not limited to, sodium succinate, sodium citrate, sodium phosphate or potassium phosphate. Sodium chloride can be used to modify the tonicity of the solution at a concentration of 0-300 mM (optimally 150 mM for a liquid dosage form). Cryoprotectants can be included for a lyophilized dosage form, principally 0-10% sucrose (optimally 0.5-1.0%). Other suitable cryoprotectants include trehalose and lactose. Bulking agents can be included for a lyophilized dosage form, principally 1-10% mannitol (optimally 2-4%). Stabilizers can be used in both liquid and lyophilized dosage forms, principally 1-50 mM L-Methionine (optimally 5-10 mM). Other suitable bulking agents include glycine, arginine, can be included as 0-0.05% polysorbate-80 (optimally 0.005-0.01%). Additional surfactants include but are not limited to polysorbate 20 and BRIJ surfactants. The pharmaceutical composition comprising the antibodies described herein prepared as an injectable solution for parenteral administration, can further comprise an agent useful as an adjuvant, such as those used to increase the absorption, or dispersion of the antibody. A particularly useful adjuvant is hyaluronidase, such as Hylenex® (recombinant human hyaluronidase). Addition of hyaluronidase in the injectable solution improves human bioavailability following parenteral administration, particularly subcutaneous administration. It also allows for greater injection site volumes (i.e. greater than 1 ml) with less pain and discomfort, and minimum incidence of injection site reactions. (See International Appln. Publication No. WO 04/078140 and U.S. Patent Appln. Publication No. US2006104968, incorporated herein by reference.)

The compositions described herein may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Compositions can be in the form of injectable or infusible solutions, such as compositions similar to those used for passive immunization of humans with other antibodies. In one embodiment, the antibody is administered by intravenous infusion or injection. In another embodiment, the antibody is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (i.e., a binding protein, e.g. an antibody described herein) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile, lyophilized powders for the preparation of sterile injectable solutions, methods of preparation comprise vacuum drying and spray-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including, in the composition, an agent that delays absorption, for example, monostearate salts and gelatin.

The antibodies described herein can be administered by a variety of methods known in the art. For example, the route/mode of administration may be subcutaneous injection, intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J.R. Robinson, ed., Marcel Dekker, Inc., New York, 1978.

In certain embodiments, an antibody described herein may be orally administered, for example, with an inert diluent or an assimilable edible carrier. The antibody (and other ingredients, if desired) may also be enclosed in a hard or soft shell gelatin capsule, compressed into tablets, or incorporated directly into the subject's diet. For oral therapeutic administration, the antibody may be incorporated with excipients and used in the form of ingestible tablets, buccal tablets, troches, capsules, elixirs, suspensions, syrups, wafers, and the like. To administer an antibody described herein by other than parenteral administration, it may be necessary to coat the antibody with, or co-administer the antibody with, a material to prevent its inactivation.

Supplementary active compounds can also be incorporated into the compositions. In certain embodiments, an antibody described herein is co-formulated with and/or co-administered with one or more additional therapeutic agents that are useful for treating disorders or diseases described herein. For example, an anti-RGMa antibody described herein may be co-formulated and/or co-administered with one or more additional antibodies that bind other targets (e.g., antibodies that bind other soluble antigens or that bind cell surface molecules). Furthermore, one or more antibodies described herein may be used in combination with two or more of the foregoing therapeutic agents. Such combination therapies may advantageously utilize lower dosages of the administered therapeutic agents, thus avoiding possible toxicities or complications associated with the various monotherapies.

In certain embodiments, an antibody described herein is linked to a half-life extending vehicle known in the art. Such vehicles include, but are not limited to, the Fc domain, polyethylene glycol, and dextran. Such vehicles are described, e.g., in U.S. Application Serial No. 09/428,082 and published PCT Application No. WO 99/25044, which are hereby incorporated by reference for any purpose.

It should be understood that the antibodies described herein can be used alone or in combination with one or more additional agents, e.g., a therapeutic agent (for example, a small molecule or biologic), said additional agent being selected by the skilled artisan for its intended purpose. For example, the additional therapeutic agent may be an immunosuppressant or an agent that treats one or more symptoms associated with multiple sclerosis. The additional agent may be an alpha or beta interferon. Alpha or beta interferons, such as Avonex, Betaseron, Extavia and Rebif, may slow the rate at which multiple sclerosis symptoms worsen over time. The additional agent may be a corticosteroid, such as methylprednisolone (Solu-Medrol) or prednisone (Deltasone). The additional agent may be glatiramer (Copaxone), which may block the immune system's attack on myelin. The additional agent may be fingolimod (Gilenya), which may trap immune cells in lymph nodes. The additional agent may be natalizumab (Tysabri), which may interfere with the movement of potentially damaging immune cells from the bloodstream to the brain and spinal cord. The additional agent may be mitoxantrone (Novantrone), which is an immunosuppressant drug. The additional agent may be teriflunimide (Aubagio). The additional agent may be BG-12 (Tecfidera). The additional agent may be alemtuzumab (Lemtrada). The additional agent may be daclizumab (Zinbryta), which is an interleukin-2 receptor blocking antibody. The additional agent may be ocrelizumab (Ocrevus), which is an anti-CD20 antibody. The additional agent may be amantadine (Symmetrel). The additional agent may be amitriptyline (Elavil). The additional agent may be nortriptyline, modafinil (Provigil). The additional agent may be dalfampridine (Ampyra),

The additional therapeutic agent can be a "cognitive enhancing drug," which is a drug that improves impaired human cognitive abilities of the brain (namely, thinking, learning, and memory). Cognitive enhancing drugs work by altering the availability of neurochemicals (e.g., neurotransmitters, enzymes, and hormones), by improving oxygen supply, by stimulating nerve growth, or by inhibiting nerve damage. Examples of cognitive enhancing drugs include a compound that increases the activity of acetylcholine such as, but not limited to, an acetylcholine receptor agonist (e.g., a nicotinic $\alpha$-7 receptor agonist or allosteric modulator, an $\alpha4\beta2$ nicotinic receptor agonist or allosteric modulators), an acetylcholinesterase inhibitor (e.g., donepezil, rivastigmine, and galantamine), a butyrylcholinesterase inhibitor, an N-methyl-D-aspartate (NMDA) receptor antagonist (e.g., memantine), an activity-dependent neuroprotective protein (ADNP) agonist, a serotonin 5-HT1A receptor agonist (e.g., xaliproden), a 5-HT4 receptor agonist, a 5-HT6 receptor antagonist, a serotonin 1A receptor antagonist, a histamine H3 receptor antagonist, a calpain inhibitor, a vascular endothelial growth factor (VEGF) protein or agonist, a trophic growth factor, an anti-apoptotic compound, an AMPA-type glutamate receptor activator, a L-type or N-type calcium channel blocker or modulator, a potassium channel blocker, a hypoxia inducible factor (HIF) activator, a HIF prolyl 4-hydroxylase inhibitor, an anti-inflammatory agent, an inhibitor of amyloid A$\beta$ peptide or amyloid plaque, an inhibitor of tau hyperphosphorylation, a phosphodiesterase 5 inhibitor (e.g., tadalafil, sildenafil), a phosphodiesterase 4 inhibitor, a monoamine oxidase inhibitor, or pharmaceutically acceptable salt thereof. Specific examples of such cognitive enhancing drugs include, but are not limited to, cholinesterase inhibitors such as donepezil (Aricept®), rivastigmine (Exelon®), galanthamine (Reminyl®), N-methyl-D-aspartate antagonists such as memantine (Namenda®). At least one cognitive enhancing drug can be administered simultaneously with the antibodies described herein or sequentially with the antibodies described herein (and in any order) including those agents currently recognized, or in the future being recognized, as useful to treat the disease or condition being treated by an antibody described herein). Additionally, it is believed that the combinations described herein may have additive or synergistic effects when used in the above described treatment. The additional agent also can be an agent that imparts a beneficial attribute to the therapeutic composition, e.g., an agent that affects the viscosity of the composition.

It should further be understood that the combinations are those combinations useful for their intended purpose. The agents set forth above are for illustrative purposes and not intended to be limiting. The combinations can comprise an antibody and at least one additional agent selected from the lists below. The combination can also include more than one additional agent, e.g., two or three additional agents if the combination is such that the formed composition can perform its intended function.

The pharmaceutical compositions may include a "therapeutically effective amount" or a "prophylactically effective amount" of an antibody. A "therapeutically effective amount" refers to an amount effective, at dosages and for periods of time necessary, to achieve the desired therapeutic result. A therapeutically effective amount of the antibody may be determined by a person skilled in the art and may vary according to factors such as the disease state, age, sex, and weight of the individual, and the ability of the antibody to elicit a desired response in the individual. A therapeutically effective amount is also one in which toxic or detrimental effects, if any, of the antibody are outweighed by the therapeutically beneficial effects. In certain embodiments, a therapeutically effective amount is an amount that neutralizes RGMa. In certain embodiments, a therapeutically effective amount is an amount that reduces the inhibitory effect of RGMa on neuroregeneration. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

For purposes of therapy, antibodies are administered to a patient in a therapeutically effective amount in a pharmaceutically acceptable carrier. In certain embodiments, a "therapeutically effective amount" is one that is physiologically significant. The antibody is physiologically significant if its presence results in a detectable change in the physiology of a recipient patient. In the present context, the antibody may be physiologically significant if its presence results in, for example, decreased interferon-$\gamma$ (INF-$\gamma$), interleukin-2 (IL-2), IL-4 and/or IL-17 secretion from CD4+ T cells. An agent is physiologically significant if its presence results in, for example, reduced proliferative responses and/or pro-inflammatory cytokine expression in peripheral blood mononuclear cells (PBMCs). In certain embodiments, an amount of an anti-RGMa antibody is physiologically significant if it results in reduced levels of free RGMa, such as reduced levels of free RGMa in CSF.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

### 4. Method of Treating, Preventing, Modulating or Attenuating Relapsing Forms of Multiple Sclerosis

### a. Relapsing Remitting Multiple Sclerosis (RRMS)

In a patient diagnosed with multiple sclerosis, an assessment may be made as to whether the subject has relapsing-remitting multiple sclerosis. The assessment may indicate an appropriate course of therapy, such as preventative therapy, maintenance therapy, or modulative therapy. Accordingly, provided herein is a method of treating, preventing, modulating, or attenuating relapsing-remitting form of multiple sclerosis by administering a therapeutically effective amount of one or more of the antibodies described herein (such as, for example, antibody AE12-1-Y-QL) to a patient in need thereof.

In one embodiment, a fixed dosage of one or more antibodies described herein may be administered to a subject. An exemplary, non-limited range for a therapeutically or prophylactically effective amount of an antibody or antibody portion described herein is from about 50 mg to about 4000 mg, about 50 mg to about 3900 mg, about 50 mg to about 3800 mg, about 50 mg to about 3700 mg, about 50 mg to about 3600 mg, about 50 mg to about 3500 mg, about 50 mg to about 3400 mg, about 50 mg to about 3300 mg, about 50 mg to about 3200 mg, about 50 mg to about 3100 mg, about 50 mg to about 3000 mg, about 50 mg to about 2900 mg, about 50 mg to about 2800 mg, about 50 mg to about 2700 mg, about 50 mg to about 2600 mg, about 50 mg to about 2500 mg, about 50 mg to about 2400 mg, about 50 mg to about 2300 mg, about 50 mg to about 2200 mg, about 50 mg to about 2100 mg, about 50 mg to about 2000 mg, about 50 mg to about 1900 mg, about 50 mg to about 1800 mg, about 50 mg to about 1700 mg, about 50 mg to about 1600 mg, about 50 mg to about 1500 mg, about 50 mg to about 1400 mg, about 50 mg to about 1300 mg, about 50 mg to about 1200 mg, about 50 mg to about 1100 mg, about 50 mg to about 1000 mg, about 50 mg to about 900 mg, about 50 mg to about 800 mg, about 50 mg to about 700 mg, about 50 mg to about 600 mg, about 50 mg to about 500 mg, about 50 mg to about 400 mg, about 50 mg to about 300 mg, about 100 mg to about 4000 mg, about 100 mg to about 3900 mg, about 100 mg to about 3800 mg, about 100 mg to about 3700 mg, about 100 mg to about 3600 mg, about 100 mg to about 3500 mg, about 100 mg to about 3400 mg, about 100 mg to about 3300 mg, about 100 mg to about 3200 mg, about 100 mg to about 3100 mg, about 100 mg to about 3000 mg, about 100 mg to about 2900 mg, about 100 mg to about 2800 mg, about 100 mg to about 2700 mg, about 100 mg to about 2600 mg, about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 4000 mg, about 150 mg to about 3900 mg, about 150 mg to about 3800 mg, about 150 mg to about 3700 mg, about 150 mg to about 3600 mg, about 150 mg to about 3500 mg, about 150 mg to about 3400 mg, about 150 mg to about 3300 mg, about 150 mg to about 3200 mg, about 150 mg to about 3100 mg, about 150 mg to about 3000 mg, about 150 mg to about 2900 mg, about 150 mg to about 2800 mg, about 150 mg to about 2700 mg, about 150 mg to about 2600 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 4000 mg, about 200 mg to about 3900 mg, about 200 mg to about 3800 mg, about 200 mg to about 3700 mg, about 200 mg to about 3600 mg, about 200 mg to about 3500 mg, about 200 mg to about 3400 mg, about 200 mg to about 3300 mg, about 200 mg to about 3200 mg, about 200 mg to about 3100 mg, about 200 mg to about 3000 mg, about 200 mg to about 2900 mg, about 200 mg to about 2800 mg, about 200 mg to about 2700 mg, about 200 mg to about 2600 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 4000 mg, about 250 mg to about 3900 mg, about 250 mg to about 3800 mg, about 250 mg to about 3700 mg, about 250 mg to about 3600 mg, about 250 mg to about 3500 mg, about 250 mg to about 3400 mg, about 250 mg to about 3300 mg, about 250 mg to about 3200 mg, about 250 mg to about 3100 mg, about 250 mg to about 3000 mg, about 250 mg to about 2900 mg, about 250 mg to about 2800 mg, about 250 mg to about 2700 mg, about 250 mg to about 2600 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 4000 mg, about 300 mg to about 3900 mg, about 300 mg to about 3800 mg, about 300 mg to about 3700 mg, about 300 mg to about 3600 mg, about 300 mg to about 3500 mg, about 300 mg to about 3400 mg, about 300 mg to about 3300 mg, about 300 mg to about 3200 mg, about 300 mg to about 3100 mg, about 300 mg to about 3000 mg, about 300 mg to about 2900 mg, about 300 mg to about 2800 mg, about 300 mg to about 2700 mg, about 300 mg to about 2600 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a subject may be administered 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, 2500 mg, 2525 mg, 2550 mg, 2575 mg, 2600 mg, 2625 mg, 2650 mg, 2675 mg, 2700 mg, 2725 mg, 2750 mg, 2775 mg, 2800 mg, 2825 mg, 2850 mg, 2875 mg, 2900 mg, 2925 mg, 2950 mg, 2975 mg, 3000 mg, 3025 mg, 3050 mg, 3075 mg, 3100 mg, 3125 mg, 3150 mg, 3175 mg, 3200 mg, 3225 mg, 3250 mg, 3275 mg, 3300 mg, 3325 mg, 3350 mg, 3375 mg, 3400 mg, 3425 mg, 3450 mg, 3475 mg, 3500 mg, 3525 mg, 3550 mg, 3575 mg, 3600 mg, 3625 mg, 3650 mg, 3675 mg, 3700 mg, 3725 mg, 3750 mg, 3775 mg, 3800 mg, 3825 mg, 3850 mg, 3875 mg, 3900 mg, 3925 mg, 3950 mg, 3975 mg, or 4000 mg.

The dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In one embodiment, a fixed dosage of one or more antibodies described herein may be administered to a subject. An exemplary, non-limited amount for a therapeutically or prophylactically effective amount of an antibody or antibody portion described herein includes up to about 200 mg/kg, up to about 190 mg/kg, up to about 180 mg/kg, up to about 170 mg/kg, up to about 160 mg/kg, up to about 150 mg/kg, up to about 140 mg/kg, up to about 130 mg/kg, up to about 120 mg/kg, up to about 110 mg/kg, up to about 100 mg/kg, up to about 90 mg/kg, up to about 80 mg/kg, up to about 70 mg/kg, up to about 60 mg/kg, up to about 50 mg/kg, up to about 40 mg/kg, up to about 30 mg/kg, up to about 20 mg/kg, up to about 10 mg/kg, up to about 5 mg/kg, or up to about 2.5 mg/kg.

Administration of antibodies to a patient can be intravenous, intraarterial, intraperitoneal, intramuscular, subcutaneous, intrapleural, intrathecal, intraocular, intravitreal, by perfusion through a regional catheter, or by direct intralesional injection. When administering therapeutic proteins by injection, the administration may be by continuous infusion or by single or multiple boluses. Intravenous injection provides a useful mode of administration due to the thoroughness of the circulation in rapidly distributing antibodies. The antibody may be administered orally, for example, with an inert diluent or an assimilable edible carrier. The antibody and other ingredients, if desired, may be enclosed in a hard or soft shell gelatin capsule, compressed into tablets, buccal tablets, troches, capsules, elixirs, suspensions, syrups, wafers, and the like.

In one embodiment, when one or more of the antibodies described herein is administered intravenously to a patient as fixed dose, a therapeutically or prophylactically effective amount of an antibody or antibody portion that can be administered is from about 50 mg to about 4000 mg, about 50 mg to about 3900 mg, about 50 mg to about 3800 mg, about 50 mg to about 3700 mg, about 50 mg to about 3600 mg, about 50 mg to about 3500 mg, about 50 mg to about 3400 mg, about 50 mg to about 3300 mg, about 50 mg to about 3200 mg, about 50 mg to about 3100 mg, about 50 mg to about 3000 mg, about 50 mg to about 2900 mg, about 50 mg to about 2800 mg, about 50 mg to about 2700 mg, about 50 mg to about 2600 mg, about 50 mg to about 2500 mg, about 50 mg to about 2400 mg, about 50 mg to about 2300 mg, about 50 mg to about 2200 mg, about 50 mg to about 2100 mg, about 50 mg to about 2000 mg, about 50 mg to about 1900 mg, about 50 mg to about 1800 mg, about 50 mg to about 1700 mg, about 50 mg to about 1600 mg, about 50 mg to about 1500 mg, about 50 mg to about 1400 mg, about 50 mg to about 1300 mg, about 50 mg to about 1200 mg, about 50 mg to about 1100 mg, about 50 mg to about 1000 mg, about 50 mg to about 900 mg, about 50 mg to about 800 mg, about 50 mg to about 700 mg, about 50 mg to about 600 mg, about 50 mg to about 500 mg, about 50 mg to about 400 mg, about 50 mg to about 300 mg, about 100 mg to about 4000 mg, about 100 mg to about 3900 mg, about 100 mg to about 3800 mg, about 100 mg to about 3700 mg, about 100 mg to about 3600 mg, about 100 mg to about 3500 mg, about 100 mg to about 3400 mg, about 100 mg to about 3300 mg, about 100 mg to about 3200 mg, about 100 mg to about 3100 mg, about 100 mg to about 3000 mg, about 100 mg to about 2900 mg, about 100 mg to about 2800 mg, about 100 mg to about 2700 mg, about 100 mg to about 2600 mg, about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 4000 mg, about 150 mg to about 3900 mg, about 150 mg to about 3800 mg, about 150 mg to about 3700 mg, about 150 mg to about 3600 mg, about 150 mg to about 3500 mg, about 150 mg to about 3400 mg, about 150 mg to about 3300 mg, about 150 mg to about 3200 mg, about 150 mg to about 3100 mg, about 150 mg to about 3000 mg, about 150 mg to about 2900 mg, about 150 mg to about 2800 mg, about 150 mg to about 2700 mg, about 150 mg to about 2600 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 4000 mg, about 200 mg to about 3900 mg, about 200 mg to about 3800 mg, about 200 mg to about 3700 mg, about 200 mg to about 3600 mg, about 200 mg to about 3500 mg, about 200 mg to about 3400 mg, about 200 mg to about 3300 mg, about 200 mg to about 3200 mg, about 200 mg to about 3100 mg, about 200 mg to about 3000 mg, about 200 mg to about 2900 mg, about 200 mg to about 2800 mg, about 200 mg to about 2700 mg, about 200 mg to about 2600 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 4000 mg, about 250 mg to about 3900 mg, about 250 mg to about 3800 mg, about 250 mg to about 3700 mg, about 250 mg to about 3600 mg, about 250 mg to about 3500 mg, about 250 mg to about 3400 mg, about 250 mg to about 3300 mg, about 250 mg to about 3200 mg, about 250 mg to about 3100 mg, about 250 mg to about 3000 mg, about 250 mg to about 2900 mg, about 250 mg to about 2800 mg, about 250 mg to about 2700 mg, about 250 mg to about 2600 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 4000 mg, about 300 mg to about 3900 mg, about 300 mg to about 3800 mg, about 300 mg to about 3700 mg, about 300 mg to about 3600 mg, about 300 mg to about 3500 mg, about 300 mg to about 3400 mg, about 300 mg to about 3300 mg, about 300 mg to about 3200 mg, about 300 mg to about 3100 mg, about 300 mg to about 3000 mg, about 300 mg to about 2900 mg, about 300 mg to about 2800 mg, about 300 mg to about 2700 mg, about 300 mg to about 2600 mg, about 300 mg to about 2500 mg, about 300 mg to about 2400 mg, about 300 mg to about 2300 mg, about 300 mg to about 2200 mg, about 300 mg to about 2100 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a patient may be administered 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, 2500 mg, 2525 mg, 2550 mg, 2575 mg, 2600 mg, 2625 mg, 2650 mg, 2675 mg, 2700 mg, 2725 mg, 2750 mg, 2775 mg, 2800 mg, 2825 mg, 2850 mg, 2875 mg, 2900 mg, 2925 mg, 2950 mg, 2975 mg, 3000 mg, 3025 mg, 3050 mg, 3075 mg, 3100 mg, 3125 mg, 3150 mg, 3175 mg, 3200 mg, 3225 mg, 3250 mg, 3275 mg, 3300 mg, 3325 mg, 3350 mg, 3375 mg, 3400 mg, 3425 mg, 3450 mg, 3475 mg, 3500 mg, 3525 mg, 3550 mg, 3575 mg, 3600 mg, 3625 mg, 3650 mg, 3675 mg, 3700 mg, 3725 mg, 3750 mg, 3775 mg, 3800 mg, 3825 mg, 3850 mg, 3875 mg, 3900 mg, 3925 mg, 3950 mg, 3975 mg, or 4000 mg.

The dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In another embodiment, when one or more of the antibodies described herein is administered subcutaneously to a patient as fixed dose, a therapeutically or prophylactically effective amount of an antibody or antibody portion that can be administered is from about 50 mg to about 500 mg, 50 mg to about 400 mg, about 50 mg to about 300 mg, about 50 mg to about 200 mg, about 50 mg to about 100 mg, about 75 mg to about 500 mg, 75 mg to about 400 mg, about 75 mg to about 300 mg, about 75 mg to about 200 mg, about 75 mg to about 100 mg, about 100 mg to about 500 mg, about 100 mg to about 400 mg, about 100 mg to about 300 mg, about 100 mg to about 200 mg, about 125 mg to about 500 mg, 125 mg to about 400 mg, about 125 mg to about 300 mg, about 125 mg to about 200 mg, about 150 mg to about 500 mg, 150 mg to about 400 mg, about 150 mg to about 300 mg, about 150 mg to about 200 mg, about 175 mg to about 500 mg, 175 mg to about 400 mg, about 175 mg to about 300 mg, about 175 mg to about 200 mg, about 200 mg to about 500 mg, 200 mg to about 400 mg or about 200 mg to about 300 mg. Specifically, a subject may be administered 5 mg, 10 mg, 15 mg, 20 mg, 25 mg, 30 mg, 40 mg, 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg or 500 mg.

The dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In one embodiment, a treatment regimen of one or more antibodies described herein may be administered to a subject. An exemplary, non-limiting regimen is a multiple variable dose regimen. For example, a multiple variable dose regimen may comprise a loading dose followed by at least one treatment dose that is less than the loading dose.

Anti-RGMa antibodies may be subject to elimination via target mediated disposition. In certain embodiments, the loading dose is effective to overcome target mediated disposition.

In certain embodiments, the methods comprise early attainment steady state levels of the antibody by providing a loading dose of an anti-RGMa antibody followed by subsequent doses of smaller amounts of the antibody.

In certain embodiments, the methods comprise early attainment of an efficacious target trough serum concentration by providing a loading dose of an anti-RGMa antibody followed by subsequent doses of smaller amounts of the antibody.

In certain embodiments, the methods comprise early attainment of clinical efficacy by providing a loading dose of an anti-RGMa antibody followed by subsequent doses of smaller amounts of the antibody.

For example, the steady state levels, efficacious target trough serum concentration, and/or clinical efficacy is reached in 4 weeks or less, preferably 3 weeks or less, more preferably 2 weeks or less, and most preferably 1 week or less, including 6 days or less, 5 days or less, 4 days or less, 3 days or less, 2 days or less, and 1 day or less. The steady state level is thereafter maintained by the administration of maintenance doses of smaller amounts for the remainder of the treatment regimen or until suppression of disease symptoms is achieved.

In one embodiment, the treatment dose is an amount that is at least 10% less, at least 20% less, at least 30% less, at least 40% less, at least 50% less, at least 60% less, at least 70% less, at least 80% less, at least 90% less than the loading dose. Alternatively, the treatment dose can be about 10%, about 20%, about 30%, about 40%, about 50%, about 60%, about 70%, about 80%, or about 90% of the loading dose.

In one embodiment, a loading dose is from about 100 mg to about 4000 mg, about 100 mg to about 3900 mg, about 100 mg to about 3800 mg, about 100 mg to about 3700 mg, about 100 mg to about 3600 mg, about 100 mg to about 3500 mg, about 100 mg to about 3400 mg, about 100 mg to about 3300 mg, about 100 mg to about 3200 mg, about 100 mg to about 3100 mg, about 100 mg to about 3000 mg, about 100 mg to about 2900 mg, about 100 mg to about 2800 mg, about 100 mg to about 2700 mg, about 100 mg to about 2600 mg, about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 4000 mg, about 150 mg to about 3900 mg, about 150 mg to about 3800 mg, about 150 mg to about 3700 mg, about 150 mg to about 3600 mg, about 150 mg to about 3500 mg, about 150 mg to about 3400 mg, about 150 mg to about 3300 mg, about 150 mg to about 3200 mg, about 150 mg to about 3100 mg, about 150 mg to about 3000 mg, about 150 mg to about 2900 mg, about 150 mg to about 2800 mg, about 150 mg to about 2700 mg, about 150 mg to about 2600 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 4000 mg, about 200 mg to about 3900 mg, about 200 mg to about 3800 mg, about 200 mg to about 3700 mg, about 200 mg to about 3600 mg, about 200 mg to about 3500 mg, about 200 mg to about 3400 mg, about 200 mg to about 3300 mg, about 200 mg to about 3200 mg, about 200 mg to about 3100 mg, about 200 mg to about 3000 mg, about 200 mg to about 2900 mg, about 200 mg to about 2800 mg, about 200 mg to about 2700 mg, about 200 mg to about 2600 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 4000 mg, about 250 mg to about 3900 mg, about 250 mg to about 3800 mg, about 250 mg to about 3700 mg, about 250 mg to about 3600 mg, about 250 mg to about 3500 mg, about 250 mg to about 3400 mg, about 250 mg to about 3300 mg, about 250 mg to about 3200 mg, about 250 mg to about 3100 mg, about 250 mg to about 3000 mg, about 250 mg to about 2900 mg, about 250 mg to about 2800 mg, about 250 mg to about 2700 mg, about 250 mg to about 2600 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a subject may be administered a loading dose of 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, 2500 mg, 2525 mg, 2550 mg, 2575 mg, 2600 mg, 2625 mg, 2650 mg, 2675 mg, 2700 mg, 2725 mg, 2750 mg, 2775 mg, 2800 mg, 2825 mg, 2850 mg, 2875 mg, 2900 mg, 2925 mg, 2950 mg, 2975 mg, 3000 mg, 3025 mg, 3050 mg, 3075 mg, 3300 mg, 3325 mg, 3350 mg, 3375 mg, 3200 mg, 3225 mg, 3250 mg, 3275 mg, 3300 mg, 3325 mg, 3350 mg, 3375 mg, 3400 mg, 3425 mg, 3450 mg, 3475 mg, 3500 mg, 3525 mg, 3550 mg, 3575 mg, 3600 mg, 3625 mg, 3650 mg, 3675 mg, 3700 mg, 3725 mg, 3750 mg, 3775 mg, 3800 mg, 3825 mg, 3850 mg, 3875 mg, 3900 mg, 3925 mg, 3950 mg, 3975 mg, or 4000 mg.

In certain embodiments, the loading dose comprises one or more doses, which may be administered over one or more days. In certain embodiments, the loading dose comprises two or more doses, each dose being administered on a separate day. Thus, a loading dose may be administered as one, two, or more doses, each dose being administered during a loading dose phase. The loading dose phase may comprise about 14, about 13, about 12, about 11, about 10, about 9, about 8, about 7, about 6, about 5, about 4, about 3, about 2, or about 1 day(s). For example, a loading dose may be administered as two doses on two consecutive days. In certain embodiments, the loading dose comprises a first dose of about 1800 mg and a second dose of about 1800 mg, optionally administered one or more days apart during the loading dose phase. In certain other embodiments, the loading dose comprises a single dose, such as a single dose of about 100 mg, about 300 mg, or about 1200 mg.

In certain embodiments, more than one loading dose is administered. For example, a first loading dose may be administered (over one or more days) at a first time and a subsequent loading dose may be administered (over one or more days) at a subsequent time. In certain embodiments, the interval between the first time and the subsequent time is at least one week, at least two weeks, at least three weeks, at least four weeks, at least one month, at least five weeks, at least six weeks, at least seven weeks, at least eight weeks, at least two months, at least nine weeks, at least ten weeks, at least eleven weeks, or at least twelve weeks. In particular embodiments, the interval between the first time and the subsequent time is about one week, about two weeks, about three weeks, about four weeks, about five weeks, about six weeks, about seven weeks, about eight weeks, about nine weeks, about ten weeks, about eleven weeks, or about twelve weeks.

In one embodiment, a treatment dose is from about 50 mg to about 2500 mg, about 50 mg to about 2400 mg, about 50 mg to about 2300 mg, about 50 mg to about 2200 mg, about 50 mg to about 2100 mg, about 50 mg to about 2000 mg, about 50 mg to about 1900 mg, about 50 mg to about 1800 mg, about 50 mg to about 1700 mg, about 50 mg to about 1600 mg, about 50 mg to about 1500 mg, about 50 mg to about 1400 mg, about 50 mg to about 1300 mg, about 50 mg to about 1200 mg, about 50 mg to about 1100 mg, about 50 mg to about 1000 mg, about 50 mg to about 900 mg, about 50 mg to about 800 mg, about 50 mg to about 700 mg, about 50 mg to about 600 mg, about 50 mg to about 500 mg, about 50 mg to about 400 mg, about 50 mg to about 300 mg, 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a subject may be administered a treatment dose of 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, or 2500 mg.

In one embodiment, the loading dose is two times the treatment dose. For example, the loading dose can be 100 mg of the antibody or antigen-binding fragment thereof and subsequent treatment dose(s) can be 50 mg. As another example, the loading dose can be 300 mg of the antibody or antigen-binding fragment thereof and subsequent treatment dose(s) can be 150 mg. As yet another example, the loading dose can be 1200 mg of the antibody or antigen-binding fragment thereof and subsequent treatment dose(s) can be 600 mg. As still another example, the loading dose can be 3600 mg of the antibody or antigen-binding fragment thereof and subsequent treatment dose(s) can be 1800 mg.

The treatment dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In certain exemplary embodiments, the method comprises administering from about 150 mg to about 1000 mg of an antibody once every 28 days.

In certain exemplary embodiments, the method comprises administering from about 300 mg to about 1200 mg of an antibody every month. For example, the method may comprise administering about 450 mg of an antibody every month.

In certain exemplary embodiments, the method comprises administering a loading dose of the antibody or antigen-binding fragment thereof and subsequently administering at least one treatment dose of the antibody or antigen-binding fragment thereof.

In some such embodiments, the loading dose is given in its entirety on one day. In other such embodiments, the loading dose is divided over multiple days (e.g., divided over two days). For example, the loading dose may be administered as two or more doses over two or more days.

In some such embodiments, the treatment dose is administered at least one week following administration of the loading dose. For example, a treatment dose may be administered one week following administration of the loading dose, two weeks following administration of the loading dose, three weeks following administration of the loading dose, four weeks following administration of the loading dose, five weeks following administration of the loading dose, six weeks following administration of the loading dose, seven weeks following administration of the loading dose, eight weeks following administration of the loading dose, nine weeks following administration of the loading dose, ten weeks following administration of the loading dose, eleven weeks following administration of the loading dose, or twelve weeks following administration of the loading dose.

In some such embodiments, the method comprises administering one or more treatment doses. For example, the method may comprise administering a loading dose, followed by one, two, three, four, five, six, seven, eight, nine, ten, eleven, twelve, thirteen, fourteen, fifteen, sixteen, seventeen, eighteen, nineteen, or twenty subsequent treatment doses. In a particular embodiment, the method may comprise administering a loading dose, followed by three treatment doses for a total of four doses. The interval between the loading dose and a first treatment dose may be at least one week, at least two weeks, at least three weeks, at least four weeks, at least one month, at least five weeks, at least six weeks, at least seven weeks, at least eight weeks, at least two months, at least nine weeks, at least ten weeks, at least eleven weeks, or at least twelve weeks. In particular, the interval between the loading dose and a first treatment dose may be about one week, about two weeks, about three weeks, about four weeks, about five weeks, about six weeks, about seven weeks, about eight weeks, about nine weeks, about ten weeks, about eleven weeks, or about twelve weeks. The one or more treatment doses may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

The antibodies may be administered alone or they may be conjugated to liposomes, and can be formulated according to known methods to prepare pharmaceutically useful compositions, whereby the antibodies are combined in a mixture with a pharmaceutically acceptable carrier. A "pharmaceutically acceptable carrier" may be tolerated by a recipient patient. Sterile phosphatebuffered saline is one example of a pharmaceutically acceptable carrier. Other suitable carriers are well known to those in the art. See, for example, REMINGTON'S PHARMACEUTICAL SCIENCES, 19th Ed. (1995).

Additional treatment methods may be employed to control the duration of action of an antibody in a therapeutic application. Control release preparations can be prepared through the use of polymers to complex or adsorb the antibody.

For example, biocompatible polymers include matrices of poly(ethylene-co-vinyl acetate) and matrices of a polyanhydride copolymer of a stearic acid dimer and sebacic acid. Sherwood et al., Bio/Technology 10:1446 (1992). The rate of release of an antibody from such a matrix depends upon the molecular weight of the protein, the amount of antibody within the matrix, and the size of dispersed particles. Saltzman et al., Biophys. J. 55:163 (1989); Sherwood et al., supra. Other solid dosage forms are described in REMINGTON'S PHARMACEUTICAL SCIENCES, 19th ed. (1995).

**b. Secondary Progressive Multiple Sclerosis (SPMS)**

In a patient diagnosed with multiple sclerosis, an assessment may be made as to whether the subject has secondary progressive multiple sclerosis. The assessment may indicate an appropriate course of therapy, such as preventative therapy, maintenance therapy, or modulative therapy. Accordingly, provided herein is a method of treating, preventing, modulating, or attenuating secondary progressive multiple sclerosis by administering a therapeutically effective amount of one or more of the antibodies described herein (such as, for example, antibody AE12-1-Y-QL) to a patient in need thereof.

In one embodiment, a fixed dosage of one or more antibodies described herein may be administered to a subject. An exemplary, non-limited range for a therapeutically or prophylactically effective amount of an antibody or antibody portion described herein is from about 50 mg to about 4000 mg, about 50 mg to about 3900 mg, about 50 mg to about 3800 mg, about 50 mg to about 3700 mg, about 50 mg to about 3600 mg, about 50 mg to about 3500 mg, about 50 mg to about 3400 mg, about 50 mg to about 3300 mg, about 50 mg to about 3200 mg, about 50 mg to about 3100 mg, about 50 mg to about 3000 mg, about 50 mg to about 2900 mg, about 50 mg to about 2800 mg, about 50 mg to about 2700 mg, about 50 mg to about 2600 mg, about 50 mg to about 2500 mg, about 50 mg to about 2400 mg, about 50 mg to about 2300 mg, about 50 mg to about 2200 mg, about 50 mg to about 2100 mg, about 50 mg to about 2000 mg, about 50 mg to about 1900 mg, about 50 mg to about 1800 mg, about 50 mg to about 1700 mg, about 50 mg to about 1600 mg, about 50 mg to about 1500 mg, about 50 mg to about 1400 mg, about 50 mg to about 1300 mg, about 50 mg to about 1200 mg, about 50 mg to about 1100 mg, about 50 mg to about 1000 mg, about 50 mg to about 900 mg, about 50 mg to about 800 mg, about 50 mg to about 700 mg, about 50 mg to about 600 mg, about 50 mg to about 500 mg, about 50 mg to about 400 mg, about 50 mg to about 300 mg, about 100 mg to about 4000 mg, about 100 mg to about 3900 mg, about 100 mg to about 3800 mg, about 100 mg to about 3700 mg, about 100 mg to about 3600 mg, about 100 mg to about 3500 mg, about 100 mg to about 3400 mg, about 100 mg to about 3300 mg, about 100 mg to about 3200 mg, about 100 mg to about 3100 mg, about 100 mg to about 3000 mg, about 100 mg to about 2900 mg, about 100 mg to about 2800 mg, about 100 mg to about 2700 mg, about 100 mg to about 2600 mg, about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 4000 mg, about 150 mg to about 3900 mg, about 150 mg to about 3800 mg, about 150 mg to about 3700 mg, about 150 mg to about 3600 mg, about 150 mg to about 3500 mg, about 150 mg to about 3400 mg, about 150 mg to about 3300 mg, about 150 mg to about 3200 mg, about 150 mg to about 3100 mg, about 150 mg to about 3000 mg, about 150 mg to about 2900 mg, about 150 mg to about 2800 mg, about 150 mg to about 2700 mg, about 150 mg to about 2600 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 4000 mg, about 200 mg to about 3900 mg, about 200 mg to about 3800 mg, about 200 mg to about 3700 mg, about 200 mg to about 3600 mg, about 200 mg to about 3500 mg, about 200 mg to about 3400 mg, about 200 mg to about 3300 mg, about 200 mg to about 3200 mg, about 200 mg to about 3100 mg, about 200 mg to about 3000 mg, about 200 mg to about 2900 mg, about 200 mg to about 2800 mg, about 200 mg to about 2700 mg, about 200 mg to about 2600 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 4000 mg, about 250 mg to about 3900 mg, about 250 mg to about 3800 mg, about 250 mg to about 3700 mg, about 250 mg to about 3600 mg, about 250 mg to about 3500 mg, about 250 mg to about 3400 mg, about 250 mg to about 3300 mg, about 250 mg to about 3200 mg, about 250 mg to about 3100 mg, about 250 mg to about 3000 mg, about 250 mg to about 2900 mg, about 250 mg to about 2800 mg, about 250 mg to about 2700 mg, about 250 mg to about 2600 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 4000 mg, about 300 mg to about 3900 mg, about 300 mg to about 3800 mg, about 300 mg to about 3700 mg, about 300 mg to about 3600 mg, about 300 mg to about 3500 mg, about 300 mg to about 3400 mg, about 300 mg to about 3300 mg, about 300 mg to about 3200 mg, about 300 mg to about 3100 mg, about 300 mg to about 3000 mg, about 300 mg to about 2900 mg, about 300 mg to about 2800 mg, about 300 mg to about 2700 mg, about 300 mg to about 2600 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a subject may be administered 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, 2500 mg, 2525 mg, 2550 mg, 2575 mg, 2600 mg, 2625 mg, 2650 mg, 2675 mg, 2700 mg, 2725 mg, 2750 mg, 2775 mg, 2800 mg, 2825 mg, 2850 mg, 2875 mg, 2900 mg, 2925 mg, 2950 mg, 2975 mg, 3000 mg, 3025 mg, 3050 mg, 3075 mg, 3100 mg, 3125 mg, 3150 mg, 3175 mg, 3200 mg, 3225 mg, 3250 mg, 3275 mg, 3300 mg, 3325 mg, 3350 mg, 3375 mg, 3400 mg, 3425 mg, 3450 mg, 3475 mg, 3500 mg, 3525 mg, 3550 mg, 3575 mg, 3600 mg, 3625 mg, 3650 mg, 3675 mg, 3700 mg, 3725 mg, 3750 mg, 3775 mg, 3800 mg, 3825 mg, 3850 mg, 3875 mg, 3900 mg, 3925 mg, 3950 mg, 3975 mg, or 4000 mg.

The dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In one embodiment, a fixed dosage of one or more antibodies described herein may be administered to a subject. An exemplary, non-limited amount for a therapeutically or prophylactically effective amount of an antibody or antibody portion described herein includes up to about 200 mg/kg, up to about 190 mg/kg, up to about 180 mg/kg, up to about 170 mg/kg, up to about 160 mg/kg, up to about 150 mg/kg, up to about 140 mg/kg, up to about 130 mg/kg, up to about 120 mg/kg, up to about 110 mg/kg, up to about 100 mg/kg, up to about 90 mg/kg, up to about 80 mg/kg, up to about 70 mg/kg, up to about 60 mg/kg, up to about 50 mg/kg, up to about 40 mg/kg, up to about 30 mg/kg, up to about 20 mg/kg, up to about 10 mg/kg, up to about 5 mg/kg, or up to about 2.5 mg/kg.

Administration of antibodies to a patient can be intravenous, intraarterial, intraperitoneal, intramuscular, subcutaneous, intrapleural, intrathecal, intraocular, intravitreal, by perfusion through a regional catheter, or by direct intralesional injection. When administering therapeutic proteins by injection, the administration may be by continuous infusion or by single or multiple boluses. Intravenous injection provides a useful mode of administration due to the thoroughness of the circulation in rapidly distributing antibodies. The antibody may be administered orally, for example, with an inert diluent or an assimilable edible carrier. The antibody and other ingredients, if desired, may be enclosed in a hard or soft shell gelatin capsule, compressed into tablets, buccal tablets, troches, capsules, elixirs, suspensions, syrups, wafers, and the like.

In one embodiment, when one or more of the antibodies described herein is administered intravenously to a patient as fixed dose, a therapeutically or prophylactically effective amount of an antibody or antibody portion that can be administered is from about 50 mg to about 4000 mg, about 50 mg to about 3900 mg, about 50 mg to about 3800 mg, about 50 mg to about 3700 mg, about 50 mg to about 3600 mg, about 50 mg to about 3500 mg, about 50 mg to about 3400 mg, about 50 mg to about 3300 mg, about 50 mg to about 3200 mg, about 50 mg to about 3100 mg, about 50 mg to about 3000 mg, about 50 mg to about 2900 mg, about 50 mg to about 2800 mg, about 50 mg to about 2700 mg, about 50 mg to about 2600 mg, about 50 mg to about 2500 mg, about 50 mg to about 2400 mg, about 50 mg to about 2300 mg, about 50 mg to about 2200 mg, about 50 mg to about 2100 mg, about 50 mg to about 2000 mg, about 50 mg to about 1900 mg, about 50 mg to about 1800 mg, about 50 mg to about 1700 mg, about 50 mg to about 1600 mg, about 50 mg to about 1500 mg, about 50 mg to about 1400 mg, about 50 mg to about 1300 mg, about 50 mg to about 1200 mg, about 50 mg to about 1100 mg, about 50 mg to about 1000 mg, about 50 mg to about 900 mg, about 50 mg to about 800 mg, about 50 mg to about 700 mg, about 50 mg to about 600 mg, about 50 mg to about 500 mg, about 50 mg to about 400 mg, about 50 mg to about 300 mg, about 100 mg to about 4000 mg, about 100 mg to about 3900 mg, about 100 mg to about 3800 mg, about 100 mg to about 3700 mg, about 100 mg to about 3600 mg, about 100 mg to about 3500 mg, about 100 mg to about 3400 mg, about 100 mg to about 3300 mg, about 100 mg to about 3200 mg, about 100 mg to about 3100 mg, about 100 mg to about 3000 mg, about 100 mg to about 2900 mg, about 100 mg to about 2800 mg, about 100 mg to about 2700 mg, about 100 mg to about 2600 mg, about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 4000 mg, about 150 mg to about 3900 mg, about 150 mg to about 3800 mg, about 150 mg to about 3700 mg, about 150 mg to about 3600 mg, about 150 mg to about 3500 mg, about 150 mg to about 3400 mg, about 150 mg to about 3300 mg, about 150 mg to about 3200 mg, about 150 mg to about 3100 mg, about 150 mg to about 3000 mg, about 150 mg to about 2900 mg, about 150 mg to about 2800 mg, about 150 mg to about 2700 mg, about 150 mg to about 2600 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 4000 mg, about 200 mg to about 3900 mg, about 200 mg to about 3800 mg, about 200 mg to about 3700 mg, about 200 mg to about 3600 mg, about 200 mg to about 3500 mg, about 200 mg to about 3400 mg, about 200 mg to about 3300 mg, about 200 mg to about 3200 mg, about 200 mg to about 3100 mg, about 200 mg to about 3000 mg, about 200 mg to about 2900 mg, about 200 mg to about 2800 mg, about 200 mg to about 2700 mg, about 200 mg to about 2600 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 4000 mg, about 250 mg to about 3900 mg, about 250 mg to about 3800 mg, about 250 mg to about 3700 mg, about 250 mg to about 3600 mg, about 250 mg to about 3500 mg, about 250 mg to about 3400 mg, about 250 mg to about 3300 mg, about 250 mg to about 3200 mg, about 250 mg to about 3100 mg, about 250 mg to about 3000 mg, about 250 mg to about 2900 mg, about 250 mg to about 2800 mg, about 250 mg to about 2700 mg, about 250 mg to about 2600 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 4000 mg, about 300 mg to about 3900 mg, about 300 mg to about 3800 mg, about 300 mg to about 3700 mg, about 300 mg to about 3600 mg, about 300 mg to about 3500 mg, about 300 mg to about 3400 mg, about 300 mg to about 3300 mg, about 300 mg to about 3200 mg, about 300 mg to about 3100 mg, about 300 mg to about 3000 mg, about 300 mg to about 2900 mg, about 300 mg to about 2800 mg, about 300 mg to about 2700 mg, about 300 mg to about 2600 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a patient may be administered 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, 2500 mg, 2525 mg, 2550 mg, 2575 mg, 2600 mg, 2625 mg, 2650 mg, 2675 mg, 2700 mg, 2725 mg, 2750 mg, 2775 mg, 2800 mg, 2825 mg, 2850 mg, 2875 mg, 2900 mg, 2925 mg, 2950 mg, 2975 mg, 3000 mg, 3025 mg, 3050 mg, 3075 mg, 3100 mg, 3125 mg, 3150 mg, 3175 mg, 3200 mg, 3225 mg, 3250 mg, 3275 mg, 3300 mg, 3325 mg, 3350 mg, 3375 mg, 3400 mg, 3425 mg, 3450 mg, 3475 mg, 3500 mg, 3525 mg, 3550 mg, 3575 mg, 3600 mg, 3625 mg, 3650 mg, 3675 mg, 3700 mg, 3725 mg, 3750 mg, 3775 mg, 3800 mg, 3825 mg, 3850 mg, 3875 mg, 3900 mg, 3925 mg, 3950 mg, 3975 mg, or 4000 mg.

The dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In another embodiment, when one or more of the antibodies described herein is administered subcutaneously to a patient as fixed dose, a therapeutically or prophylactically effective amount of an antibody or antibody portion that can be administered is from about 50 mg to about 500 mg, 50 mg to about 400 mg, about 50 mg to about 300 mg, about 50 mg to about 200 mg, about 50 mg to about 100 mg, about 75 mg to about 500 mg, 75 mg to about 400 mg, about 75 mg to about 300 mg, about 75 mg to about 200 mg, about 75 mg to about 100 mg, about 100 mg to about 500 mg, about 100 mg to about 400 mg, about 100 mg to about 300 mg, about 100 mg to about 200 mg, about 125 mg to about 500 mg, 125 mg to about 400 mg, about 125 mg to about 300 mg, about 125 mg to about 200 mg, about 150 mg to about 500 mg, 150 mg to about 400 mg, about 150 mg to about 300 mg, about 150 mg to about 200 mg, about 175 mg to about 500 mg, 175 mg to about 400 mg, about 175 mg to about 300 mg, about 175 mg to about 200 mg, about 200 mg to about 500 mg, 200 mg to about 400 mg or about 200 mg to about 300 mg. Specifically, a subject may be administered 5 mg, 10 mg, 15 mg, 20 mg, 25 mg, 30 mg, 40 mg, 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg or 500 mg.

The dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In one embodiment, a treatment regimen of one or more antibodies described herein may be administered to a subject. An exemplary, non-limiting regimen is a multiple variable dose regimen. For example, a multiple variable dose regimen may comprise a loading dose followed by at least one treatment dose that is less than the loading dose.

Anti-RGMa antibodies may be subject to elimination via target mediated disposition. In certain embodiments, the loading dose is effective to overcome target mediated disposition.

In certain embodiments, the methods comprise early attainment steady state levels of the antibody by providing a loading dose of an anti-RGMa antibody followed by subsequent doses of smaller amounts of the antibody.

In certain embodiments, the methods comprise early attainment of an efficacious target trough serum concentration by providing a loading dose of an anti-RGMa antibody followed by subsequent doses of smaller amounts of the antibody.

In certain embodiments, the methods comprise early attainment of clinical efficacy by providing a loading dose of an anti-RGMa antibody followed by subsequent doses of smaller amounts of the antibody.

For example, the steady state levels, efficacious target trough serum concentration, and/or clinical efficacy is reached in 4 weeks or less, preferably 3 weeks or less, more preferably 2 weeks or less, and most preferably 1 week or less, including 6 days or less, 5 days or less, 4 days or less, 3 days or less, 2 days or less, and 1 day or less. The steady state level is thereafter maintained by the administration of maintenance doses of smaller amounts for the remainder of the treatment regimen or until suppression of disease symptoms is achieved.

In one embodiment, the treatment dose is an amount that is at least 10% less, at least 20% less, at least 30% less, at least 40% less, at least 50% less, at least 60% less, at least 70% less, at least 80% less, at least 90% less than the loading dose. Alternatively, the treatment dose can be about 10%, about 20%, about 30%, about 40%, about 50%, about 60%, about 70%, about 80%, or about 90% of the loading dose.

In one embodiment, a loading dose is from about 100 mg to about 4000 mg, about 100 mg to about 3900 mg, about 100 mg to about 3800 mg, about 100 mg to about 3700 mg, about 100 mg to about 3600 mg, about 100 mg to about 3500 mg, about 100 mg to about 3400 mg, about 100 mg to about 3300 mg, about 100 mg to about 3200 mg, about 100 mg to about 3100 mg, about 100 mg to about 3000 mg, about 100 mg to about 2900 mg, about 100 mg to about 2800 mg, about 100 mg to about 2700 mg, about 100 mg to about 2600 mg, about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 4000 mg, about 150 mg to about 3900 mg, about 150 mg to about 3800 mg, about 150 mg to about 3700 mg, about 150 mg to about 3600 mg, about 150 mg to about 3500 mg, about 150 mg to about 3400 mg, about 150 mg to about 3300 mg, about 150 mg to about 3200 mg, about 150 mg to about 3100 mg, about 150 mg to about 3000 mg, about 150 mg to about 2900 mg, about 150 mg to about 2800 mg, about 150 mg to about 2700 mg, about 150 mg to about 2600 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 4000 mg, about 200 mg to about 3900 mg, about 200 mg to about 3800 mg, about 200 mg to about 3700 mg, about 200 mg to about 3600 mg, about 200 mg to about 3400 mg, about 200 mg to about 3300 mg, about 200 mg to about 3200 mg, about 200 mg to about 3100 mg, about 200 mg to about 3000 mg, about 200 mg to about 2900 mg, about 200 mg to about 2800 mg, about 200 mg to about 2700 mg, about 200 mg to about 2600 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 4000 mg, about 250 mg to about 3900 mg, about 250 mg to about 3800 mg, about 250 mg to about 3700 mg, about 250 mg to about 3600 mg, about 250 mg to about 3500 mg, about 250 mg to about 3400 mg, about 250 mg to about 3300 mg, about 250 mg to about 3200 mg, about 250 mg to about 3100 mg, about 250 mg to about 3000 mg, about 250 mg to about 2900 mg, about 250 mg to about 2800 mg, about 250 mg to about 2700 mg, about 250 mg to about 2600 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a subject may be administered a loading dose of 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, 2500 mg, 2525 mg, 2550 mg, 2575 mg, 2600 mg, 2625 mg, 2650 mg, 2675 mg, 2700 mg, 2725 mg, 2750 mg, 2775 mg, 2800 mg, 2825 mg, 2850 mg, 2875 mg, 2900 mg, 2925 mg, 2950 mg, 2975 mg, 3000 mg, 3025 mg, 3050 mg, 3075 mg, 3300 mg, 3325 mg, 3350 mg, 3375 mg, 3200 mg, 3225 mg, 3250 mg, 3275 mg, 3300 mg, 3325 mg, 3350 mg, 3375 mg, 3400 mg, 3425 mg, 3450 mg, 3475 mg, 3500 mg, 3525 mg, 3550 mg, 3575 mg, 3600 mg, 3625 mg, 3650 mg, 3675 mg, 3700 mg, 3725 mg, 3750 mg, 3775 mg, 3800 mg, 3825 mg, 3850 mg, 3875 mg, 3900 mg, 3925 mg, 3950 mg, 3975 mg, or 4000 mg.

In certain embodiments, the loading dose comprises one or more doses, which may be administered over one or more days. In certain embodiments, the loading dose comprises two or more doses, each dose being administered on a separate day. Thus, a loading dose may be administered as one, two, or more doses, each dose being administered during a loading dose phase. The loading dose phase may comprise about 14, about 13, about 12, about 11, about 10, about 9, about 8, about 7, about 6, about 5, about 4, about 3, about 2, or about 1 day(s). For example, a loading dose may be administered as two doses on two consecutive days. In certain embodiments, the loading dose comprises a first dose of about 1800 mg and a second dose of about 1800 mg, optionally administered one or more days apart during the loading dose phase. In certain other embodiments, the loading dose comprises a single dose, such as a single dose of about 100 mg, about 300 mg, or about 1200 mg.

In certain embodiments, more than one loading dose is administered. For example, a first loading dose may be administered (over one or more days) at a first time and a subsequent loading dose may be administered (over one or more days) at a subsequent time. In certain embodiments, the interval between the first time and the subsequent time is at least one week, at least two weeks, at least three weeks, at least four weeks, at least one month, at least five weeks, at least six weeks, at least seven weeks, at least eight weeks, at least two months, at least nine weeks, at least ten weeks, at least eleven weeks, or at least twelve weeks. In particular embodiments, the interval between the first time and the subsequent time is about one week, about two weeks, about three weeks, about four weeks, about five weeks, about six weeks, about seven weeks, about eight weeks, about nine weeks, about ten weeks, about eleven weeks, or about twelve weeks.

In one embodiment, a treatment dose is from about 50 mg to about 2500 mg, about 50 mg to about 2400 mg, about 50 mg to about 2300 mg, about 50 mg to about 2200 mg, about 50 mg to about 2100 mg, about 50 mg to about 2000 mg, about 50 mg to about 1900 mg, about 50 mg to about 1800 mg, about 50 mg to about 1700 mg, about 50 mg to about 1600 mg, about 50 mg to about 1500 mg, about 50 mg to about 1400 mg, about 50 mg to about 1300 mg, about 50 mg to about 1200 mg, about 50 mg to about 1100 mg, about 50 mg to about 1000 mg, about 50 mg to about 900 mg, about 50 mg to about 800 mg, about 50 mg to about 700 mg, about 50 mg to about 600 mg, about 50 mg to about 500 mg, about 50 mg to about 400 mg, about 50 mg to about 300 mg, 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg, about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, about 150 mg to about 2500 mg, about 150 mg to about 2400 mg, about 150 mg to about 2300 mg, about 150 mg to about 2200 mg, about 150 mg to about 2100 mg, about 150 mg to about 2000 mg, about 150 mg to about 1900 mg, about 150 mg to about 1800 mg, about 150 mg to about 1700 mg, about 150 mg to about 1600 mg, about 150 mg to about 1500 mg, about 150 mg to about 1400 mg, about 150 mg to about 1300 mg, about 150 mg to about 1200 mg, about 150 mg to about 1100 mg, about 150 mg to about 1000 mg, about 150 mg to about 900 mg, about 150 mg to about 800 mg, about 150 mg to about 700 mg, about 150 mg to about 600 mg, about 150 mg to about 500 mg, about 150 mg to about 400 mg, about 150 mg to about 300 mg, about 200 mg to about 2500 mg, about 200 mg to about 2400 mg, about 200 mg to about 2300 mg, about 200 mg to about 2200 mg, about 200 mg to about 2100 mg, about 200 mg to about 2000 mg, about 200 mg to about 1900 mg, about 200 mg to about 1800 mg, about 200 mg to about 1700 mg, about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 200 mg to about 500 mg, about 200 mg to about 400 mg, about 200 mg to about 300 mg, about 250 mg to about 2500 mg, about 250 mg to about 2400 mg, about 250 mg to about 2300 mg, about 250 mg to about 2200 mg, about 250 mg to about 2100 mg, about 250 mg to about 2000 mg, about 250 mg to about 1900 mg, about 250 mg to about 1800 mg, about 250 mg to about 1700 mg, about 250 mg to about 1600 mg, about 250 mg to about 1500 mg, about 250 mg to about 1400 mg, about 250 mg to about 1300 mg, about 250 mg to about 1200 mg, about 250 mg to about 1100 mg, about 250 mg to about 1000 mg, about 250 mg to about 900 mg, about 250 mg to about 800 mg, about 250 mg to about 700 mg, about 250 mg to about 600 mg, about 250 mg to about 500 mg, about 250 mg to about 400 mg, about 250 mg to about 300 mg, about 300 mg to about 2500 mg, about 300 mg to about 2400 mg, about 300 mg to about 2300 mg, about 300 mg to about 2200 mg, about 300 mg to about 2100 mg, about 300 mg to about 2000 mg, about 300 mg to about 1900 mg, about 300 mg to about 1800 mg, about 300 mg to about 1700 mg, about 300 mg to about 1600 mg, about 300 mg to about 1500 mg, about 300 mg to about 1400 mg, about 300 mg to about 1300 mg, about 300 mg to about 1200 mg, about 300 mg to about 1100 mg, about 300 mg to about 1000 mg, about 300 mg to about 900 mg, about 300 mg to about 800 mg, about 300 mg to about 700 mg, about 300 mg to about 600 mg, about 300 mg to about 500 mg, about 300 mg to about 400 mg, about 350 mg to about 4000 mg, about 350 mg to about 3900 mg, about 350 mg to about 3800 mg, about 350 mg to about 3700 mg, about 350 mg to about 3600 mg, about 350 mg to about 3500 mg, about 350 mg to about 3400 mg, about 350 mg to about 3300 mg, about 350 mg to about 3200 mg, about 350 mg to about 3100 mg, about 350 mg to about 3000 mg, about 350 mg to about 2900 mg, about 350 mg to about 2800 mg, about 350 mg to about 2700 mg, about 350 mg to about 2600 mg, about 350 mg to about 2500 mg, about 350 mg to about 2400 mg, about 350 mg to about 2300 mg, about 350 mg to about 2200 mg, about 350 mg to about 2100 mg, about 350 mg to about 2000 mg, about 350 mg to about 1900 mg, about 350 mg to about 1800 mg, about 350 mg to about 1700 mg, about 350 mg to about 1600 mg, about 350 mg to about 1500 mg, about 350 mg to about 1400 mg, about 350 mg to about 1300 mg, about 350 mg to about 1200 mg, about 350 mg to about 1100 mg, about 350 mg to about 1000 mg, about 350 mg to about 900 mg, about 350 mg to about 800 mg, about 350 mg to about 700 mg, about 350 mg to about 600 mg, about 350 mg to about 500 mg, about 350 mg to about 400 mg, about 400 mg to about 4000 mg, about 400 mg to about 3900 mg, about 400 mg to about 3800 mg, about 400 mg to about 3700 mg, about 400 mg to about 3600 mg, about 400 mg to about 3500 mg, about 400 mg to about 3400 mg, about 400 mg to about 3300 mg, about 400 mg to about 3200 mg, about 400 mg to about 3100 mg, about 400 mg to about 3000 mg, about 400 mg to about 2900 mg, about 400 mg to about 2800 mg, about 400 mg to about 2700 mg, about 400 mg to about 2600 mg, about 400 mg to about 2500 mg, about 400 mg to about 2400 mg, about 400 mg to about 2300 mg, about 400 mg to about 2200 mg, about 400 mg to about 2100 mg, about 400 mg to about 2000 mg, about 400 mg to about 1900 mg, about 400 mg to about 1800 mg, about 400 mg to about 1700 mg, about 400 mg to about 1600 mg, about 400 mg to about 1500 mg, about 400 mg to about 1400 mg, about 400 mg to about 1300 mg, about 400 mg to about 1200 mg, about 400 mg to about 1100 mg, about 400 mg to about 1000 mg, about 400 mg to about 900 mg, about 400 mg to about 800 mg, about 400 mg to about 700 mg, about 400 mg to about 600 mg, about 400 mg to about 500 mg, about 450 mg to about 4000 mg, about 450 mg to about 3900 mg, about 450 mg to about 3800 mg, about 450 mg to about 3700 mg, about 450 mg to about 3600 mg, about 450 mg to about 3500 mg, about 450 mg to about 3400 mg, about 450 mg to about 3300 mg, about 450 mg to about 3200 mg, about 450 mg to about 3100 mg, about 450 mg to about 3000 mg, about 450 mg to about 2900 mg, about 450 mg to about 2800 mg, about 450 mg to about 2700 mg, about 450 mg to about 2600 mg, about 450 mg to about 2500 mg, about 450 mg to about 2400 mg, about 450 mg to about 2300 mg, about 450 mg to about 2200 mg, about 450 mg to about 2100 mg, about 450 mg to about 2000 mg, about 450 mg to about 1900 mg, about 450 mg to about 1800 mg, about 450 mg to about 1700 mg, about 450 mg to about 1600 mg, about 450 mg to about 1500 mg, about 450 mg to about 1400 mg, about 450 mg to about 1300 mg, about 450 mg to about 1200 mg, about 450 mg to about 1100 mg, about 450 mg to about 1000 mg, about 450 mg to about 900 mg, about 450 mg to about 800 mg, about 450 mg to about 700 mg, about 450 mg to about 600 mg, or about 450 mg to about 500 mg.

Specifically, a subject may be administered a treatment dose of 50 mg, 75 mg, 100 mg, 125 mg, 150 mg, 175 mg, 200 mg, 225 mg, 250 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg, 2375 mg, 2400 mg, 2425 mg, 2450 mg, 2475 mg, or 2500 mg.

In one embodiment, the loading dose is two times the treatment dose. For example, the loading dose can be 100 mg of the antibody or antigen-binding fragment thereof and subsequent treatment dose(s) can be 50 mg. As another example, the loading dose can be 300 mg of the antibody or antigen-binding fragment thereof and subsequent treatment dose(s) can be 150 mg. As yet another example, the loading dose can be 1200 mg of the antibody or antigen-binding fragment thereof and subsequent treatment dose(s) can be 600 mg. As still another example, the loading dose can be 3600 mg of the antibody or antigen-binding fragment thereof and subsequent treatment dose(s) can be 1800 mg.

The treatment dose of the antibody or antibody portion described herein may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

In certain exemplary embodiments, the method comprises administering from about 150 mg to about 1000 mg of an antibody once every 28 days.

In certain exemplary embodiments, the method comprises administering from about 300 mg to about 1200 mg of an antibody every month. For example, the method may comprise administering about 450 mg of an antibody every month.

In certain exemplary embodiments, the method comprises administering a loading dose of the antibody or antigen-binding fragment thereof and subsequently administering at least one treatment dose of the antibody or antigen-binding fragment thereof.

In some such embodiments, the loading dose is given in its entirety on one day. In other such embodiments, the loading dose is divided over multiple days (e.g., divided over two days). For example, the loading dose may be administered as two or more doses over two or more days.

In some such embodiments, the treatment dose is administered at least one week following administration of the loading dose. For example, a treatment dose may be administered one week following administration of the loading dose, two weeks following administration of the loading dose, three weeks following administration of the loading dose, four weeks following administration of the loading dose, five weeks following administration of the loading dose, six weeks following administration of the loading dose, seven weeks following administration of the loading dose, eight weeks following administration of the loading dose, nine weeks following administration of the loading dose, ten weeks following administration of the loading dose, eleven weeks following administration of the loading dose, or twelve weeks following administration of the loading dose.

In some such embodiments, the method comprises administering one or more treatment doses. For example, the method may comprise administering a loading dose, followed by one, two, three, four, five, six, seven, eight, nine, ten, eleven, twelve, thirteen, fourteen, fifteen, sixteen, seventeen, eighteen, nineteen, or twenty subsequent treatment doses. In a particular embodiment, the method may comprise administering a loading dose, followed by three treatment doses for a total of four doses. The interval between the loading dose and a first treatment dose may be at least one week, at least two weeks, at least three weeks, at least four weeks, at least one month, at least five weeks, at least six weeks, at least seven weeks, at least eight weeks, at least two months, at least nine weeks, at least ten weeks, at least eleven weeks, or at least twelve weeks. In particular, the interval between the loading dose and a first treatment dose may be about one week, about two weeks, about three weeks, about four weeks, about five weeks, about six weeks, about seven weeks, about eight weeks, about nine weeks, about ten weeks, about eleven weeks, or about twelve weeks. The one or more treatment doses may be administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks according to the individual need and the professional judgment of the person administering or supervising the administration of the compositions, and that dosage ranges set forth herein are exemplary only and are not intended to limit the scope or practice of the claimed composition.

The antibodies may be administered alone or they may be conjugated to liposomes, and can be formulated according to known methods to prepare pharmaceutically useful compositions, whereby the antibodies are combined in a mixture with a pharmaceutically acceptable carrier. A "pharmaceutically acceptable carrier" may be tolerated by a recipient patient. Sterile phosphatebuffered saline is one example of a pharmaceutically acceptable carrier. Other suitable carriers are well known to those in the art. See, for example, REMINGTON'S PHARMACEUTICAL SCIENCES, 19th Ed. (1995).

Additional treatment methods may be employed to control the duration of action of an antibody in a therapeutic application. Control release preparations can be prepared through the use of polymers to complex or adsorb the antibody. For example, biocompatible polymers include matrices of poly(ethylene-co-vinyl acetate) and matrices of a polyanhydride copolymer of a stearic acid dimer and sebacic acid. Sherwood et al., Bio/Technology 10:1446 (1992). The rate of release of an antibody from such a matrix depends upon the molecular weight of the protein, the amount of antibody within the matrix, and the size of dispersed particles. Saltzman et al., Biophys. J. 55:163 (1989); Sherwood et al., supra. Other solid dosage forms are described in REMINGTON'S PHARMACEUTICAL SCIENCES, 19th ed. (1995).

The following items are further described:

1. A method of treating a relapsing form of multiple sclerosis in a subject in need thereof, the method comprising administering a therapeutically effective amount of an antibody or antigen-binding fragment thereof that specifically binds Repulsive Guidance Molecule A (RGMa), wherein the antibody or antigen binding fragment comprises

(a) a variable heavy chain comprising a complementarity determining region (CDR)-1 comprising an amino acid sequence of SEQ ID NO:2, a CDR-2 comprising an amino acid sequence of SEQ ID NO:3, and a CDR-3 comprising an amino acid sequence of SEQ ID NO:4; and
(b) a variable light chain comprising a CDR-1 comprising an amino acid sequence of SEQ ID NO:6, a CDR-2 comprising an amino acid sequence of SEQ ID NO:7, and a CDR-3 comprising an amino acid sequence of SEQ ID NO:8.

2. The method of item **1**, wherein the antibody or antigen-binding fragment thereof is administered to a subject in an amount of from about 50 mg to about 4000 mg, or in an amount of from about 50 mg to about 2500 mg.

3. The method of item **2**, wherein the antibody or antigen-binding fragment thereof is administered in an amount of about 50 mg, 100 mg, 150 mg, 300 mg, 450 mg, 600 mg, 1000 mg, 1200 mg, 1600 mg, 1800 mg, 2400 mg, or 3600 mg.

4. The method of item **2**, wherein the antibody or antigen-binding fragment thereof is administered in an amount of about 50 mg, 75 mg, 100 mg, 120 mg, 125 mg, 150 mg, 175 mg, 200 mg, 250 mg, 300 mg, 325 mg, 350 mg, 375 mg, 400 mg, 425 mg, 450 mg, 475 mg or 500 mg.

5. The method of item **3**, wherein the antibody or antigen-binding fragment thereof is administered intravenously (IV).

6. The method of item **4**, wherein the antibody or antigen-binding fragment thereof is administered subcutaneously.

**7**. The method of any one of item **1-6**, wherein the variable heavy chain comprises an amino acid sequence of SEQ ID NO: 13 and the variable light chain comprises an amino acid sequence of SEQ ID NO: 14.

**8**. The method of any one of items **1-7**, the antibody is selected from the group consisting of a human antibody, an immunoglobulin molecule, a disulfide linked Fv, a monoclonal antibody, an affinity matured antibody, a scFv, a chimeric antibody, a CDR-grafted antibody, a diabody, a humanized antibody, a multispecific antibody, a Fab, a dual specific antibody, a DVD, a Fab', a bispecific antibody, a F(ab')$_2$, and a Fv.

**9**. The method of item **8**, wherein the antibody is a human antibody.

**10**. The method of item **8**, wherein the antibody is a monoclonal antibody.

**11**. The method of item **8**, wherein the antibody is an affinity matured antibody.

**12**. The method of item **8**, wherein the antibody is a chimeric antibody.

**13**. The method of item **8**, wherein the antibody is a humanized antibody.

**14**. The method of item **8**, wherein the antibody is a Fab, a Fab', a F(ab')$_2$ or Fv.

**15**. The method of item **8**, wherein the antibody is a dual specific antibody, a DVD or a bispecific antibody.

**16**. The method of item **7**, further comprising a constant sequence of SEQ ID NO: 12.

**17**. The method of any one of claims **1-16**, further comprising administering an additional therapeutic agent.

**18**. The method of item **17**, where the additional therapeutic agent is an immunosuppressant or an agent that treats one or more symptoms associated with multiple sclerosis.

**19**. The method of item **18**, wherein the additional therapeutic agent comprises a beta interferon, glatiramer (Copaxone), fingolimod (Gilenya), natalizumab (Tysabri), mitoxantrone (Novantrone), teriflunimide (Aubagio), BG-12 (Tecfidera), alemtuzumab (Lemtrada), daclizumab (Zinbryta), ocrelizumab (Ocrevus), amantadine (Symmetrel), amitriptyline (Elavil), nortriptyline, modafinil (Provigil), dalfampridine (Ampyra), a cognitive enhancing drug, an immunomodulatory drug, or a neuroprotective drug.

**20**. The method of item **19**, wherein the cognitive enhancing drug comprises an acetylcholine receptor agonist, an acetylcholinesterase inhibitor, a butyrylcholinesterase inhibitor, an N-methyl-D-aspartate (NMDA) receptor antagonist, an activity-dependent neuroprotective protein (ADNP) agonist, a serotonin 5-HT1A receptor agonist, a 5-HT4 receptor agonist, a 5-HT6 receptor antagonist, a serotonin 1A receptor antagonist, a histamine H3 receptor antagonist, a calpain inhibitor, a vascular endothelial growth factor (VEGF) protein or agonist, a trophic growth factor, an anti-apoptotic compound, an AMPA-type glutamate receptor activator, a L-type or N-type calcium channel blocker or modulator, a potassium channel blocker, a hypoxia inducible factor (HIF) activator, a HIF prolyl 4-hydroxylase inhibitor, an anti-inflammatory agent, an inhibitor of amyloid Aβ peptide or amyloid plaque, an inhibitor of tau hyperphosphorylation, a phosphodiesterase 5 inhibitor, a phosphodiesterase 4 inhibitor, a monoamine oxidase inhibitor, pharmaceutically acceptable salts thereof, or a combination thereof.

**21**. The method of item **20**, wherein the cognitive enhancing drug comprises donepezil (Aricept®), rivastigmine (Exelon®), galanthamine (Reminyl®), memantine (Namenda®), or a combination thereof.

**22**. The method of any one of items **1-21**, wherein the relapsing form of multiple sclerosis is relapsing remitting multiple sclerosis (RRMS) or relapsing-secondary progressive multiple sclerosis (SPMS).

**23**. The method of any one of items **1-22**, wherein the antibody or antigen-binding fragment thereof is administered according to a multiple variable dose regimen.

**24**. The method of item **23**, wherein the multiple variable dose regimen comprises a loading dose and a treatment dose that is lower than the loading dose.

**25**. The method of item **24**, wherein the loading dose is selected from the group consisting of 100 mg, 300 mg, 1200 mg, and 3600 mg.

**26**. The method of item **24**, wherein the treatment dose is selected from the group consisting of 50 mg, 150 mg, 600 mg, and 1800 mg

**27**. The method of item **24**, wherein a time interval between the loading dose and a first treatment dose is at least one week, at least two weeks, at least three weeks, at least four weeks, at least one month, at least five weeks, at least six weeks, at least seven weeks, at least eight weeks, at least two months, at least nine weeks, at least ten weeks, at least eleven weeks, or at least twelve weeks.

**28**. The method of item **1**, wherein the antibody or antigen-binding fragment thereof is administered once per week, once every other week, once every two weeks, once every three weeks, once every four weeks, once every month, once every five weeks, once every six weeks, once every seven weeks, once every eight weeks, once every two months, once every nine weeks, once every ten weeks, once every eleven weeks or once every twelve weeks.

**5. Examples**

The present invention has multiple aspects, illustrated by the following non-limiting examples.

**Example 1**

**Single Dose Study to Evaluate the Safety, Tolerability, Pharmacokinetics and Immunogenicity of AE12-1-Y-QL in Healthy Subjects**

**Study Design**: This is a single-dose, randomized, double-blind, placebo-controlled, single-center study. Up to 56 male and female subjects in general good health will be enrolled in this study. The objection of the study was to assess the safety, tolerability, pharmacokinetics and immunogenicity of single intravenous and subcutaneous doses of AE-12-1-Y-QL in healthy subjects, i.e., adult male and female subjects in general good health.

**Methodology**: There will be seven dose Groups with each Group consisting of 8 subjects. In each Group of 8 subjects, 6 subjects will be randomly assigned to receive a single dose of AE-12-1-Y-QL and 2 subjects to receive placebo. The doses in Groups 1 through 6 will be administered by intravenous infusion (IV). The dose in Group 7 will be administered by subcutaneous injection (SC). The doses to be administered are shown in Table 4.

**Table 4**

| Group | N = 6 | N = 2 |
| --- | --- | --- |
| 1 | 50 mg single IV dose on Day 1 | Placebo |
| 2* | 150 mg single IV dose on Day 1 | Placebo |
| 3* | 450 mg single IV dose on Day 1 | Placebo |
| 4* | 1000 mg single IV dose on Day 1 | Placebo |
| 5* | 1600 mg single IV dose on Day 1 | Placebo |
| 6* | 2400 mg single IV dose on Day 1 | Placebo |
| 7* | 150 mg single SC dose on Day 1 | Placebo |
| * Planned doses for Groups 2-7 may be adjusted after review of the available safety, tolerability and pharmacokinetic data from the prior dose group(s). | | |

In each Group, the dose will be administered in the morning of Day 1. The dosing in one Group will be separated from the dosing in the next Group by at least 5 weeks. Higher doses will be administered only after the available safety, tolerability and pharmacokinetic data for the lower dose(s) have been reviewed and evaluated. In Groups 1 through 7, on Day 1, one active and one placebo subject will be dosed and for subsequent days, six additional subjects will be dosed with an allocation of 5 active and 1 placebo. In each Group, a maximum of 2 subjects will be dosed per day. Dosing will be done on consecutive days, assuming no recruitment delays. The dosing in Group 7 may start as soon as 5 weeks after dosing of Group 2 is completed.

Serial blood samples for determination of AE-12-1-Y-QL concentrations will be collected by venipuncture as follows: prior to dosing (0 hour), and at 2, 4, 6, 10, 14, 24, 48, 72, 96, 120, 144, and 168 hours, and on Days 14, 28, 42, 56, 70, 84, 112, and 140 after dosing. Blood sample for determination of anti-drug antibodies will be collected prior to dosing (0 hour) on Day 1 and on Days 8 14, 28, 42, 56, and 84 after dosing.

Subjects will have two magnetic resonance imaging (MRI) scans: the baseline scan will be performed prior to dosing and prior to the baseline lumbar puncture.

Two lumbar punctures will be performed to collect CSF for determination of AE-12-1-Y-QL concentration and for biomarker analyses. The first lumbar puncture (baseline) will be performed prior to dosing and the second lumbar puncture will be performed on Day 7, approximately at the same time as the baseline lumbar puncture.

Diagnosis and Main Criteria for Inclusion/Exclusion:

**Main Inclusion:** A subject will be eligible for study participation if he/she meets the following criteria:

1. Male or female and age at Screening is between 18 and 55 years, inclusive.
2. If female, subject must:

- be of non-childbearing potential defined by:
- Subject is postmenopausal (no menses for at least 1 year and of appropriate age).
- Subject is surgically sterile, defined as bilateral oopheorectomy and/or hysterectomy, or has had a bilateral tubal occlusion (including ligation and blockage methods such as Essure®) since at least 3 months prior to Screening.

3. Females must have negative results for pregnancy tests at Screening on a urine specimen and prior to dosing on a serum sample obtained on Check-in Day.

4. If male, subject must be surgically sterile (vasectomy) or agree to practice at least one of the following methods of birth control from initial study drug administration until 140 days after the last dose of study drug:

- Total abstinence from sexual intercourse as the preferred lifestyle of the subject; periodic abstinence is not acceptable;
- Partner(s) using an IUD;
- Partner(s) using oral, injected or implanted methods of hormonal contraceptives;
- Subject and/or partner(s) using double-barrier method (male condom and

occlusive cap [diaphragm or cervical cap] or contraceptive sponge [all with spermicidal jellies or creams]);

5. Body Mass Index (BMI) is 18.0 to 29.9, inclusive. BMI is calculated as weight in kg divided by the square of height measured in meters.

6. A condition of general good health based upon the results of a medical history, physical examination, vital signs, laboratory profile, neurological examination and a 12-lead electrocardiogram (ECG).

**Main Exclusion:** A subject will not be eligible for study participation if he/she meets any of the following criteria:

1. Requirement for any over-the-counter and/or prescription medication, vitamins and/or herbal supplements on a regular basis.

2. Use of any medications (prescriptions or over-the-counter), vitamins and/or herbal supplements within the 14-day period prior to study drug administration or within 10 half-lives of the respective medication, whichever is longer, unless prior approval from the AbbVie study designated physician has been obtained.

3. Receipt of any depot drug by injection within 30 days prior to study drug administration.

4. Receipt of an investigational product within a time period equal to 10 half-lives, if known, or within 6 weeks prior to study drug administration, whichever is longer.

5. Positive urine screen for drugs of abuse, alcohol or cotinine.

6. Consumption of alcohol within 72 hours prior to study drug administration.

7. History of malignancy; however, subjects with a history of excised or treated basal cell carcinoma or fewer than 3 skin squamous cell carcinomas are eligible to participate in this study.

8. Known hypersensitivity to study drugs or their excipients.

9. History of drug or alcohol abuse within 2 years prior to study drug administration.

10. History of severe allergic or anaphylactic reactions.

11. History of seizure disorder or unexplained blackouts or history of a seizure within 6 months.

12. History of suicidal ideation or an episode of clinically severe depression within 1 month prior to study drug administration as evidenced by answering "yes" to questions 4 or 5 on the suicidal ideation portion of the Columbia-Suicide Severity Rating Scale (C-SSRS) completed at Screening, or any history of suicide attempts.

13. Positive test result for hepatitis A virus immunoglobulin M (HAV-IgM), hepatitis B surface antigen (HBsAg) or hepatitis C virus antibody (HCV Ab) or HIV antibodies (HIV Ab). Negative HIV status will be confirmed at Screening and the results will be maintained confidentially by the study site.

14. Varicella or herpes zoster virus infection or any severe viral infection within 6 weeks before screening.

15. Exposure to varicella zoster virus within 21 days before Screening.

16. History of human immunodeficiency virus (HIV) or other immunodeficient conditions.

17. Receipt of any type of live virus vaccine from 4 weeks before dosing, including but not limited to: measles/mumps/rubella vaccine, varicella zoster virus vaccine, oral polio vaccine, and nasal influenza vaccine.

18. Infection (viral, fungal, bacterial) requiring hospitalization or intravenous (IV) antibiotics within 8 weeks before dosing.

19. Elective surgery performed from 2 weeks prior to randomization or scheduled through the end of the study.

20. History of abnormal laboratory results that, in the opinion of the Investigator, are indicative of any significant cardiac, endocrine, hematological, hepatic, immunologic, metabolic, urologic, pulmonary, gastrointestinal, dermatologic, psychiatric, renal, neurological and/or other major disease:

21. Any of the following abnormal blood tests at screening:

- Hemoglobin $\leq$ 10.0 g/dL
- Platelets $< 150 \times 10^9$/L
- Lymphocytes $\leq 1.0 \times 10^9$/L
- Neutrophils $\leq 1.5 \times 10^9$/L

- Alanine aminotransferase/serum glutamate pyruvate transaminase (ALT/SGPT), or aspartate aminotransferase/serum glutamic oxaloacetic transaminase (AST/SGOT) > 1 × upper limit of normal (ULN)
- Serum creatinine ≥ 1 × ULN
- Serum iron, ferritin, transferrin saturation > 1 × ULN

22. Contraindication for lumbar puncture (e.g., lumbar scoliosis, coagulopathy, infected skin at needle puncture site) or use of blood-thinning compound, including non-steroidal anti-inflammatory agents (e.g., aspirin, ibuprofen, naproxen), clopidogrel, warfarin, heparin (or heparainoids), fondaparinux (or related compounds) or thrombin inhibitors (dabigatran) and factor Xa inhibitors. (Rivaroxaban and apixaban) within 14 days of lumbar puncture.

23. Subjects for whom MRI is contraindicated, (i.e., aneurysm clip, metal fragments, internal electrical devices such as a cochlear implant, spinal cord stimulator or pacemaker), are allergic to gadolinium, or have claustrophobia.

24. Baseline brain MRI scan shows the presence of intracranial mass or other evidence that might preclude the subject from undergoing a lumbar puncture.

25. Donation or loss of 450 mL or more blood volume (including plasmapheresis) or receipt of a transfusion of any blood product within 8 weeks prior to study drug administration.

26. Pregnant or breastfeeding female.

**Pharmacokinetic Variables:** The following pharmacokinetic parameters of AE-12-1-Y-QL will be determined using non-compartmental methods. The maximum observed serum concentration ($C_{max}$), the time to $C_{max}$ (peak time, $T_{max}$), terminal phase elimination rate constant ($\beta$), terminal phase elimination half-life ($T_{1/2}$), the area under the plasma concentration-time curve (AUC) from time 0 to the time of the last measurable concentration (AUCt), AUC from time 0 to infinite time (AUC∞), and clearance (CL for IV doses and CL/F for SC dose) will be determined. Additionally, following SC administration the absolute bioavailability ($F_{abs}$) will be estimated. Anti-drug antibody titers will be determined for assessment of immunogenicity. Additional parameters may be calculated if useful in the interpretation of the data.

**Pharmacogenetic Variables.** The DNA sample labeled "PG-DNA blood" may be analyzed for genetic factors contributing to the subj ect's response to AE-12-1-Y-QL, or other study treatment, in terms of pharmacokinetics, pharmacodynamics, tolerability, and safety. Such genetic factors may include genes for drug metabolizing enzymes, drug transport proteins, genes within the target pathway, or other genes believed to be related to drug response. Some genes currently insufficiently characterized or unknown may be understood to be important at the time of analysis. The samples may be analyzed as part of a multi-study assessment of genetic factors involved in the response to AE-12-1-Y-QL or drugs of this class. The samples may also be used for the development of diagnostic tests related to AE-12-1-Y-QL (or drugs of this class). The results of pharmacogenetic analyses may not be reported with the study summary.

The DNA sample labeled "PGDM-DNA blood" will be analyzed in order to characterize the genes for drug metabolizing enzymes or drug transport proteins, genes within the target pathway, or other genes believed to be related to drug response to any medication. The analysis of samples for pharmacogenetic variables may be performed by a non-Good Laboratory Practice (GLP) laboratory.

**Safety:** The safety assessments will include: adverse event monitoring, vital signs, physical examination, neurological examination, electrocardiograms, laboratory tests, Columbia-Suicide Severity Rating Scale and brain MRI. MRI will be performed at baseline and at 28 days after dosing. The following MRI sequences will be performed: T1 weighted; T2 weighted FLAIR; T1 with gadolinium; T2 weighted; Diffusion weighted; and Gradient echo (GRE).

Adverse events will be coded by Medical Dictionary for Regulatory Activities (MedDRA). The number and percentage of subjects reporting treatment-emergent adverse events will be tabulated by MedDRA preferred term and system organ class (SOC) with a breakdown by route and dose level. Tabulations will also be provided in which the number of subjects reporting an adverse event (MedDRA term) is additionally broken down by rating (mild, moderate or severe) and by whether possibly related to study drug. Any deaths, other serious adverse events and other significant adverse events will be separately identified. Laboratory test values and measurements on vital signs and quantitative ECG variables that are potentially clinically significant, according to predefined criteria, will be identified.

## Example 2

### Single Dose Study to Evaluate the Safety, Tolerability, Pharmacokinetics and Immunogenicity of AE12-1-Y-QL in Healthy Subjects

**Study Design**: This was a single-dose, randomized, double-blind, placebo-controlled, single-center study. Forty-seven (47) male and female subjects in general good health were enrolled in this study. The object of the study was to assess the safety, tolerability, pharmacokinetics and immunogenicity of single intravenous and subcutaneous doses of AE-12-1-Y-QL in healthy subjects, i.e., adult male and female subjects in general good health.

**Methodology**: There were six dose Groups with each Group consisting of 7 or 8 subjects. Each of Groups 1 through 5 contained 8 subjects; 6 subjects were randomly assigned to receive a single dose of AE-12-1-Y-QL and 2 subjects to receive placebo. The doses in Groups 1 through 5 were administered by intravenous infusion (IV). Group 7 contained 7 subjects; 5 subjects were randomly assigned to receive a single dose of AE-12-1-Y-QL and 2 subjects to receive placebo. The dose in Group 7 was administered by subcutaneous injection (SC). The doses to be administered are shown in Table 5.

**Table 5**

| Group | Treatment | |
|---|---|---|
| 1 | AE-12-1-Y-QL 50 mg (IV) | Placebo |
| | N=6 | N=2 |
| 2 | AE-12-1-Y-QL **150 mg** (IV) N=6 | Placebo N=2 |
| 3 | AE-12-1-Y-QL **450 mg** (IV) N=6 | Placebo N=2 |
| 4 | AE-12-1-Y-QL **1000 mg** (IV) N=6 | Placebo N=2 |
| 5 | AE-12-1-Y-QL **1600 mg** (IV) N=6 | Placebo N=2 |
| 7 | AE-12-1-Y-QL **150 mg** (SC) N=5 | Placebo N=2 |

In each Group, the dose was administered in the morning of Day 1. The dosing in one Group was separated from the dosing in the next Group by at least 5 weeks. Higher doses were administered only after the available safety, tolerability and pharmacokinetic data for the lower dose(s) were reviewed and evaluated. On Day 1, one active and one placebo subject was dosed and for subsequent days, six additional subjects were dosed with an allocation of 5 active and 1 placebo. In each Group, a maximum of 2 subjects were dosed per day. Dosing was done on consecutive days. The dosing in Group 7 started as soon as 5 weeks after dosing of Group 2 was completed.

For Groups 1-3 and 7, serial blood samples for determination of AE-12-1-Y-QL concentrations were collected by venipuncture as follows: prior to dosing (0 hour), and at 2, 4, 6, 10, 14, 24, 48, 72, 96, 120, 144, and 168 hours, and on Days 14, 28, 42, 56, 70, 84, 112, and 140 after dosing. For Group 4, serial blood samples for determination of AE-12-1-Y-QL concentrations were collected by venipuncture as follows: prior to dosing (0 hour), and at 2, 4, 6, 10, 14, 24, 48, 72, 96, 120, 144, and 168 hours, and on Days 14, 28, 42, 56, 70, 84, 112, 140, and > 196 after dosing. For Group 5, serial blood samples for determination of AE-12-1-Y-QL concentrations were collected by venipuncture as follows: prior to dosing (0 hour), and at 2, 4, 6, 10, 14, 24, 48, 72, 96, 120, 144, and 168 hours, and on Days 14, 28, 42, 56, 70, 84, 112, 140, 168, and 196 after dosing. Blood sample for determination of anti-drug antibodies was collected prior to dosing (0 hour) on Day 1 and on Days 8 14, 28, 42, 56, and 84 after dosing.

Subjects had two magnetic resonance imaging (MRI) scans: the baseline scan was performed prior to dosing and prior to the baseline lumbar puncture.

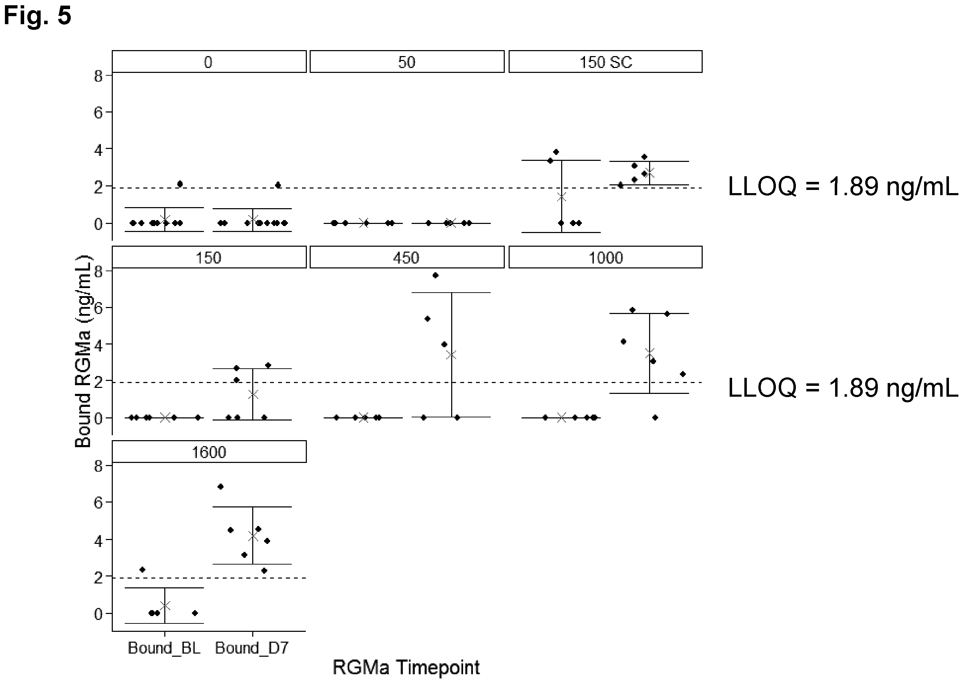

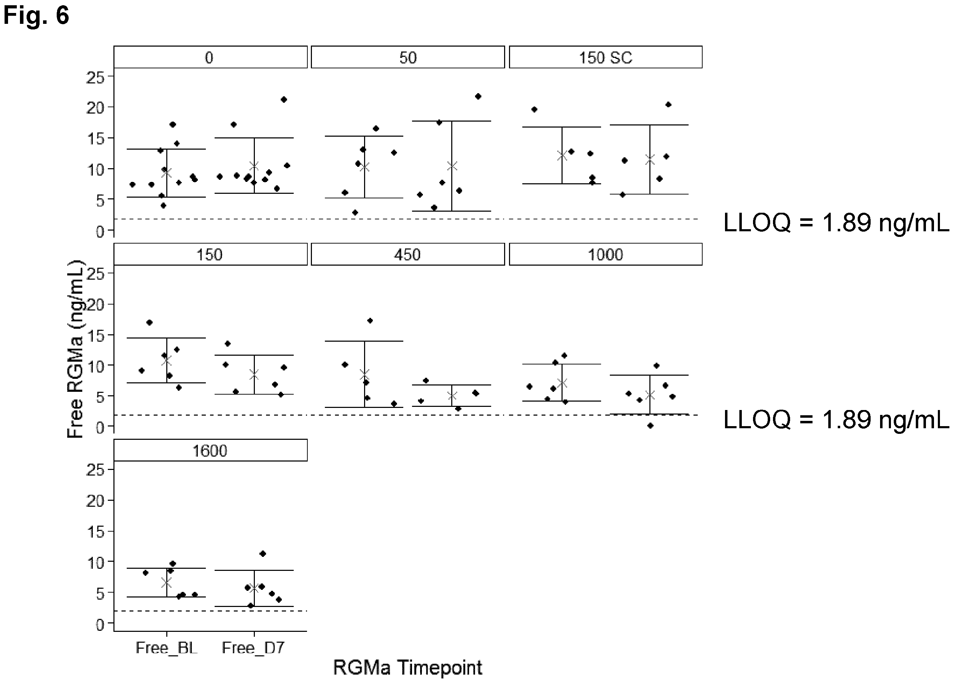

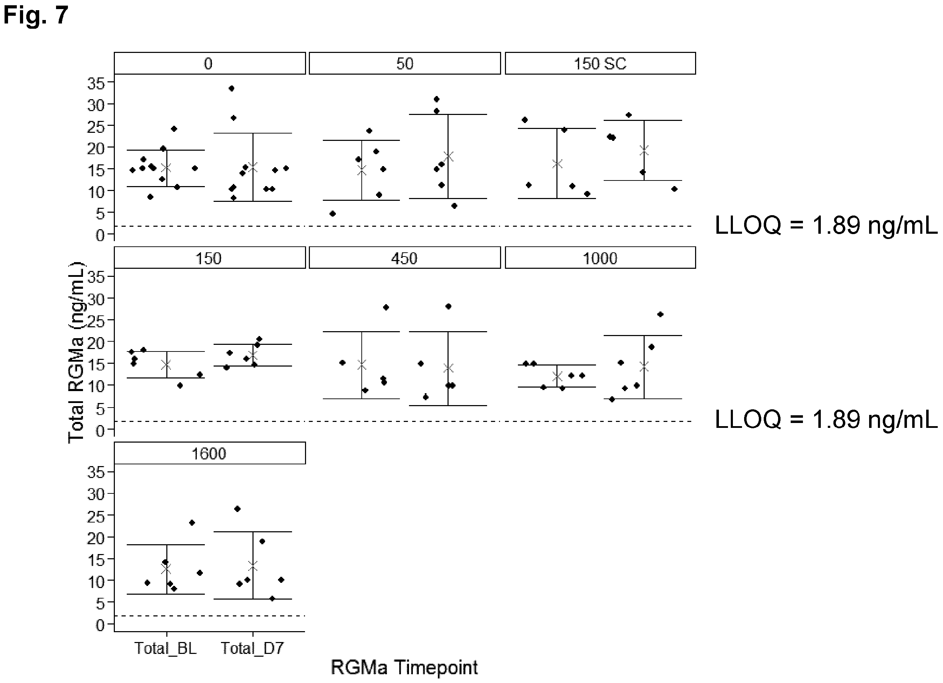

Two lumbar punctures were performed to collect CSF for determination of AE-12-1-Y-QL concentration; total (see Fig. 7), free (see Fig. 6) and bound (see Fig. 5) sRGMa levels; and for biomarker analyses. The first lumbar puncture (baseline) was performed prior to dosing (baseline lumbar puncture) and the second lumbar puncture will be performed on Day 7, approximately at the same time as the baseline lumbar puncture.

Diagnosis and Main Criteria for Inclusion/Exclusion:

**Main Inclusion:** A subject was eligible for study participation if he/she met the following criteria:

1. Male or female and age at Screening is between 18 and 55 years, inclusive.
2. If female, subject must:

- be of non-childbearing potential defined by:
- Subject is postmenopausal (no menses for at least 1 year and of appropriate age).
- Subject is surgically sterile, defined as bilateral oopheorectomy and/or hysterectomy, or has had a bilateral tubal occlusion (including ligation and blockage methods such as Essure®) since at least 3 months prior to Screening.

3. Females must have negative results for pregnancy tests at Screening on a urine specimen and prior to dosing on a serum sample obtained on Check-in Day.
4. If male, subject must be surgically sterile (vasectomy) or agree to practice at least one of the following methods of birth control from initial study drug administration until 140 days after the last dose of study drug:

- Total abstinence from sexual intercourse as the preferred lifestyle of the subject; periodic abstinence is not acceptable;
- Partner(s) using an IUD;
- Partner(s) using oral, injected or implanted methods of hormonal contraceptives;
- Subject and/or partner(s) using double-barrier method (male condom and

occlusive cap [diaphragm or cervical cap] or contraceptive sponge [all with spermicidal jellies or creams]);

5. Body Mass Index (BMI) is 18.0 to 29.9, inclusive. BMI is calculated as weight in kg divided by the square of height measured in meters.

6. A condition of general good health based upon the results of a medical history, physical examination, vital signs, laboratory profile, neurological examination and a 12-lead electrocardiogram (ECG).

**Main Exclusion:** A subject was not eligible for study participation if he/she met any of the following criteria:

1. Requirement for any over-the-counter and/or prescription medication, vitamins and/or herbal supplements on a regular basis.

2. Use of any medications (prescriptions or over-the-counter), vitamins and/or herbal supplements within the 14-day period prior to study drug administration or within 10 half-lives of the respective medication, whichever is longer, unless prior approval from the AbbVie study designated physician has been obtained.

3. Receipt of any depot drug by injection within 30 days prior to study drug administration.

4. Receipt of an investigational product within a time period equal to 10 half-lives, if known, or within 6 weeks prior to study drug administration, whichever is longer.

5. Positive urine screen for drugs of abuse, alcohol or cotinine.

6. Consumption of alcohol within 72 hours prior to study drug administration.

7. History of malignancy; however, subjects with a history of excised or treated basal cell carcinoma or fewer than 3 skin squamous cell carcinomas are eligible to participate in this study.

8. Known hypersensitivity to study drugs or their excipients.

9. History of drug or alcohol abuse within 2 years prior to study drug administration.

10. History of severe allergic or anaphylactic reactions.

11. History of seizure disorder or unexplained blackouts or history of a seizure within 6 months.

12. History of suicidal ideation or an episode of clinically severe depression within 1 month prior to study drug administration as evidenced by answering "yes" to questions 4 or 5 on the suicidal ideation portion of the Columbia-Suicide Severity Rating Scale (C-SSRS) completed at Screening, or any history of suicide attempts.

13. Positive test result for hepatitis A virus immunoglobulin M (HAV-IgM), hepatitis B surface antigen (HBsAg) or hepatitis C virus antibody (HCV Ab) or HIV antibodies (HIV Ab). Negative HIV status will be confirmed at Screening and the results will be maintained confidentially by the study site.

14. Varicella or herpes zoster virus infection or any severe viral infection within 6 weeks before screening.

15. Exposure to varicella zoster virus within 21 days before Screening.

16. History of human immunodeficiency virus (HIV) or other immunodeficient conditions.

17. Receipt of any type of live virus vaccine from 4 weeks before dosing, including but not limited to: measles/mumps/rubella vaccine, varicella zoster virus vaccine, oral polio vaccine, and nasal influenza vaccine.

18. Infection (viral, fungal, bacterial) requiring hospitalization or intravenous (IV) antibiotics within 8 weeks before dosing.

19. Elective surgery performed from 2 weeks prior to randomization or scheduled through the end of the study.

20. History of abnormal laboratory results that, in the opinion of the Investigator, are indicative of any significant cardiac, endocrine, hematological, hepatic, immunologic, metabolic, urologic, pulmonary, gastrointestinal, dermatologic, psychiatric, renal, neurological and/or other major disease:

21. Any of the following abnormal blood tests at screening:

- Hemoglobin $\leq$ 10.0 g/dL
- Platelets < 150 $\times$ 10$^9$/L
- Lymphocytes $\leq$ 1.0 $\times$ 10$^9$/L
- Neutrophils $\leq$ 1.5 $\times$ 10$^9$/L
- Alanine aminotransferase/serum glutamate pyruvate transaminase (ALT/SGPT), or aspartate aminotransferase/serum glutamic oxaloacetic transaminase (AST/SGOT) > 1 $\times$ upper limit of normal (ULN)
- Serum creatinine $\geq$ 1 $\times$ ULN

- Serum iron, ferritin, transferrin saturation > 1 × ULN

22. Contraindication for lumbar puncture (e.g., lumbar scoliosis, coagulopathy, infected skin at needle puncture site) or use of blood-thinning compound, including non-steroidal anti-inflammatory agents (e.g., aspirin, ibuprofen, naproxen), clopidogrel, warfarin, heparin (or heparainoids), fondaparinux (or related compounds) or thrombin inhibitors (dabigatran) and factor Xa inhibitors. (Rivaroxaban and apixaban) within 14 days of lumbar puncture.

23. Subjects for whom MRI is contraindicated, (i.e., aneurysm clip, metal fragments, internal electrical devices such as a cochlear implant, spinal cord stimulator or pacemaker), are allergic to gadolinium, or have claustrophobia.

24. Baseline brain MRI scan shows the presence of intracranial mass or other evidence that might preclude the subject from undergoing a lumbar puncture.

25. Donation or loss of 450 mL or more blood volume (including plasmapheresis) or receipt of a transfusion of any blood product within 8 weeks prior to study drug administration.

26. Pregnant or breastfeeding female.

**Pharmacokinetic Variables:** The following pharmacokinetic parameters of AE-12-1-Y-QL were determined using non-compartmental methods. The maximum observed serum concentration ($C_{max}$), the time to $C_{max}$ (peak time, $T_{max}$), terminal phase elimination rate constant ($\beta$), terminal phase elimination half-life ($T_{1/2}$), the area under the plasma concentration-time curve (AUC) from time 0 to the time of the last measurable concentration (AUCt), AUC from time 0 to infinite time (AUC∞), coefficient of variation in % (%CV) and clearance (CL for IV doses and CL/F for SC dose) were determined. Additionally, following SC administration the absolute bioavailability ($F_{abs}$) was estimated. Anti-drug antibody titers were determined for assessment of immunogenicity. Additional parameters may be calculated if useful in the interpretation of the data.

**Preliminary Results.** The preliminary mean (%CV) pharmacokinetic parameters of AE-12-1-Y-QL are shown in Table 6.

**Table 6**

| Parameter | Units | 50 mg[a](N= 6) | 150 mg[b] (N=5) | 150 mg[a] (N=6) | 450 mg[a] (N=6) | 1000 mg[a] (N=6) | **1600** mg[a] (N=6) |
|---|---|---|---|---|---|---|---|
| $T_{max}$ | h | 5.0 (49) | 134 (35) | 6.0 (52) | 4.0 (0) | 6.7 (65) | 4.7 (22) |
| $C_{max}$ | μg/mL | 15.1 (21) | 15.0 (25) | 53.7 (25) | 121 (15) | 304 (15) | 488 (11) |
| $AUC_t$ | μg•h/mL | 4600 (22) | 14400 (29) | 22200 (22) | 79900 (16) | 269000 (19) | 48500 0 (18)[d] |
| $AUC_\infty$ | μg•h/mL | 4640 (22) | 14600 (29) | 22400 (22) | 81400 (16) | 271000 (20) | 52200 0 (20)[d] |
| $t_{1/2}$[c] | days | 22.6 (85) | 22.5 (17) | 18.7 (16) | 20.8 (22) | 35.2 (16) | 50.1 (17)[d] |
| $C_{max}$/Dose | μg/mL/mg | 0.30 (21) | 0.10 (25) | 0.36 (25) | 0.27 (15) | 0.30 (15) | 0.31 (11) |
| $AUC_\infty$/ Dose | μg•h/mL/ mg | 92.8 (22) | 97.5 (29) | 149 (22) | 181 (16) | 271 (20) | 326 (20)[d] |
| a. IV administration; b. SC administration; c. Harmonic mean (pseudo CV); d. Estimates based on N=5. | | | | | | | |

$C_{max}$ values increased proportionally with dose while AUC values increase greater than dose proportionally up to 1600 mg, indicating nonlinear PK. The harmonic mean half-life ranged from 19 to 50 days across the 50 to 1600 mg dose range, with higher values for the 1000 mg and 1600 mg doses. Variability of AE-12-1-Y-QL exposure is low based on $C_{max}$ and AUC (15-22% CV).

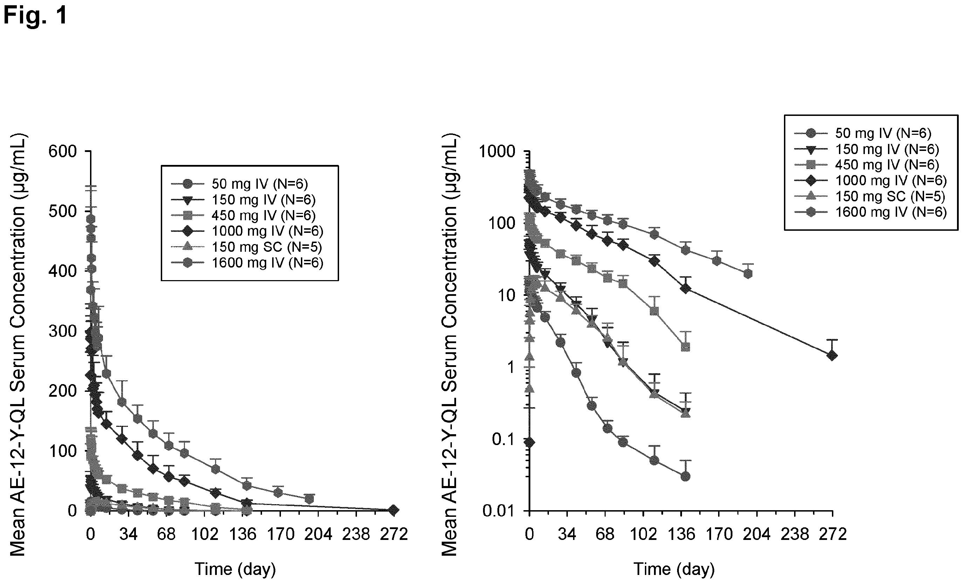

The preliminary mean (+SD) concentration-time profile of AE-12-1-Y-QL is shown in Fig. 1.

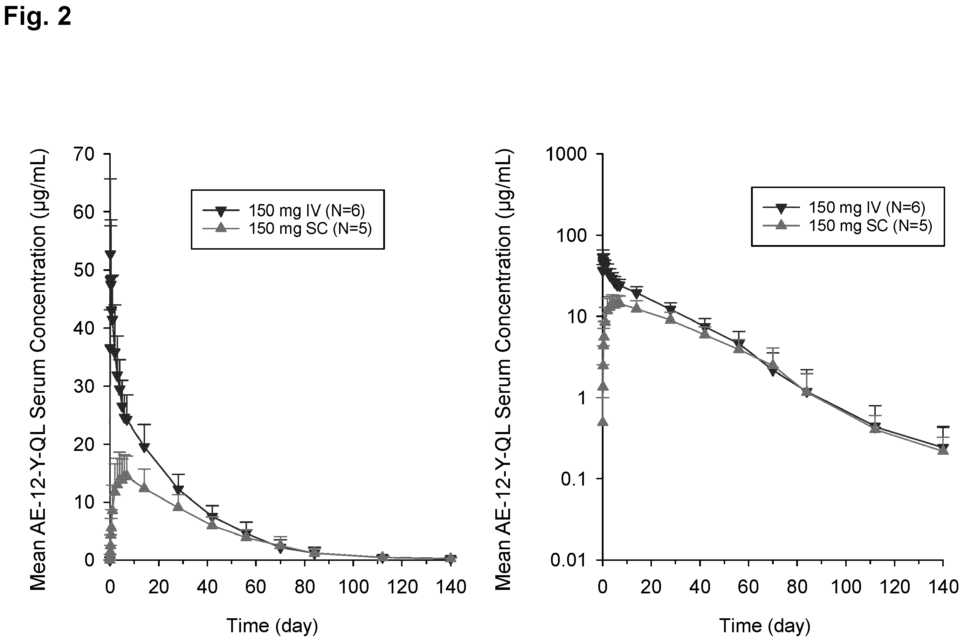

The preliminary mean (+SD) concentration-time profile of 150 mg AE-12-1-Y-QL is shown in Fig. 2. Relative bioavailability following a single SC dose of 150 mg AE-12-1-Y-QL is approximately 65% based on the exposure of both the 150 mg IV and SC dose groups.

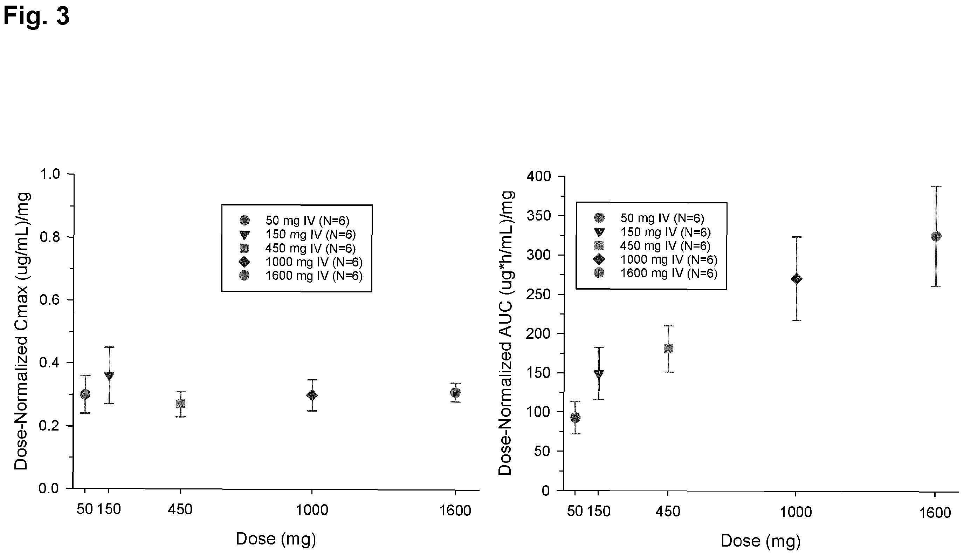

The preliminary mean (±SD) dose-normalized $C_{max}$ and $AUC_\infty$ vs. AE-12-1-Y-QL dose is shown in Fig. 3.

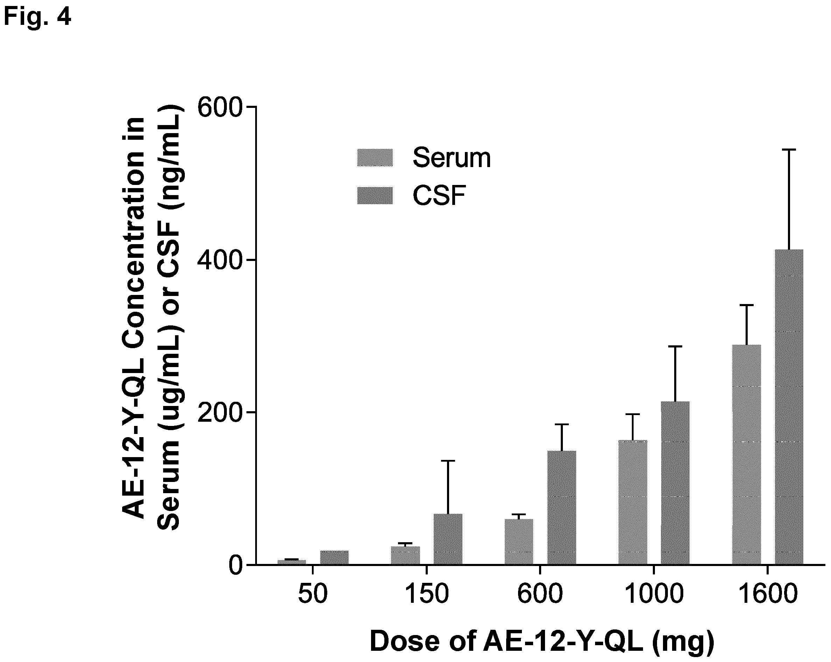

CSF and serum samples were assayed for AE-12-1-Y-QL levels at day 7. The limit of detection for the assay is 16.3 ng/mL. AE-12-1-Y-QL exposure following a single dose of AE-12-Y-QL is shown in Table 7. AE-12-1-Y-QL concentration on day 7 in serum and CSF is presented in Fig. 4. The overall CSF exposure of AE-12-1-Y-QL on post-dose day 7 was approximately 0.2% of the serum exposure.

**Table 7**

| Group | N | Dose (mg) | Mean CSF Conc. at Day 7 $\pm$ SD (ng/mL) | Mean CSF Percent of Serum Conc. at Day 7 $\pm$ SD |
|---|---|---|---|---|
| 1 | 1 | 50 | 19 | 0.33 |
| 2 | 4 | 150 | $67\pm70$ | $0.33\pm0.36$ |
| 3 | 5 | 450 | $150\pm34$ | $0.26\pm0.06$ |
| 4 | 6 | 1000 | $214\pm72$ | $0.13\pm0.04$ |
| 5 | 6 | 1600 | $413\pm131$ | $0.15\pm0.06$ |

The variability in CSF to serum concentrations is low (CV <35%) for groups 3-5; group 2 has one outlier which makes variability very large. AE-12-1-Y-QL concentrations in CSF continue to increase with dose (increase is less than dose proportional)--although the CSF to serum ratio decreases with increasing dose.

CSF samples were assayed for bound, free, and total RGMa at baseline and day 7. Bound RGMa from CSF samples is presented in Fig. 5. Free RGMa from CSF samples is presented in Fig. 6. Total RGMa from CSF samples is presented in Fig. 7.

AE-12-1-Y-QL produces a statistically significant dose dependent decrease on free RGMa concentration in CSF from healthy volunteers at 7 days post dose at doses ranging from 50-1600 mg. AE-12-1-Y-QL produces a statistically significant dose dependent increase on antibodybound RGMa concentration in CSF from healthy volunteers at 7 days post dose at doses ranging from 50-1600 mg. There was no indication of an effect on the overall concentration of total RGMa. These results indicate that AE-12-1-Y-QL reduces the concentration of free RGMa in CSF.

**Safety:** The safety assessments included: adverse event (AE) monitoring, serial assessment of vital signs, physical examination, neurological examination, psychological evaluation using the Columbia-Suicide Severity Rating Scale, electrocardiograms, laboratory tests, including comprehensive blood chemistries and complete blood counts and differential, CSF parameters, and brain MRI. MRI will be performed at baseline and at 28 days after dosing.

Four of 12 of subjects treated with placebo (33%) and in 25 of 35 of subjects treated with AE-12-1-Y-QL (71%) had at least 1 AE with no trend observed in the frequency of AEs with each ascending dose group. The most frequently reported AEs for AE-12-1-Y-QL -treated subjects were headache, procudural pain, procedural headache, and nausea. A summary of adverse events is shown in Table 8.

**Table 8**

|  | Placebo | AE12-1-Y-QL | | | | | | |
|---|---|---|---|---|---|---|---|---|
|  |  | Intravenous | | | | | SC | Total |
|  |  | 50 mg | 150 mg | 450 mg | 1000 mg | 1600 mg | 150 mg |  |
| n (%) | n=12 | n=6 | n=6 | n=6 | n=6 | n=6 | n=5 | n=35 |
| Any Adverse Event (AE) | 4 (33) | 2 (33) | 5 (83) | 3 (50) | 4 (67) | 6 (100) | 5 (100) | 25 (71) |
| Any Serious AE | 0 | 0 | 1(17) | 0 | 0 | 1 (17) | 0 | 2 (5.7) |
| Deaths | 0 | 0 | 1(17) | 0 | 0 | 1 (17) | 0 | 2 (5.7) |
| **Any AE in $\geq$2 Subjects in Any Treatment Group** | | | | | | | | |
| Headache | 1 (8.3) | 1 (17) | 2 (33) | 3 (50) | 1 (17) | 2 (33) | 3 (60) | 12 (34) |
| Procedural Pain | 1 (8.3) | 0 | 0 | 0 | 2 (33) | 4 (67) | 0 | 6 (17) |
| Procedural Headache | 1 (8.3) | 0 | 0 | 0 | 1 (17) | 2 (33) | 1 (20) | 4 (11) |
| Nausea | 1 (8.3) | 1 (17) | 1 (17) | 0 | 1 (17) | 0 | 0 | 3 (10) |
| Asthenia | 0 | 0 | 1 (17) | 0 | 1 (17) | 0 | 0 | 2 (5.7) |
| Back Pain | 0 | 0 | 1 (17) | 0 | 0 | 1 (17) | 0 | 2 (5.7) |
| Pruritus | 0 | 0 | 0 | 1 (17) | 0 | 1 (17) | 0 | 2 (5.7) |
| AE= adverse event; SC= subcutaneous | | | | | | | | |

Most of the treatment-related AEs assessed by the investigator were minor in severity (mild to moderate) and spontaneously resolved without treatment. There were no major doserelated biochemical or hematological alterations. There was no discontinuation due to treatment related AEs.

Two subjects had SAEs resulting in death and both were determined to be unrelated to AE12-1-Y-QL. In both cases, autopsy and toxicology reports concluded that the deaths were due to other causes not related to AE12-1-Y-QL. Each SAE was considered to not have impacted the overall risk/benefit profile of AE12-1-Y-QL.

**Conclusions.** AE12-1-Y-QL was well tolerated in healthy subjects up to 1600 mg. The most frequently reported AEs were associated with lumbar punctures. PK results indicated serum exposures that increased greater than dose-proportionally and CSF to serum ratio similar to other monoclonal antibodies. Given the current estimated AE12-1-Y-QL half-life, once monthly dosing is expected to achieve efficacious exposures. The safety profile and serum/CSF PK data support the use of AE12-1-Y-QL as a therapeutic agent in relapsing forms of multiple sclerosis.

**Example 3**

**Dosing Study of AE12-1-Y-QL in Subjects with Relapsing Forms of Multiple Sclerosis**

The primary objective of this study is to assess the safety, tolerability, pharmacokinetics and immunogenicity of AE12-1-Y-QL in subjects with Relapsing Forms of Multiple Sclerosis (RFMS) who are on maintenance glatiramer acetate (GA) treatment. The secondary objective is to assess the effect of AE12-1-Y-QL on the clinical and neuroimaging parameters of Multiple Sclerosis (MS) disease activity.

**Study Population:** Adult male and female subjects with RFMS who are on maintenance GA treatment.

**Methodology:** This is a double-blind, placebo-controlled, randomized, escalating multiple-dose study. Approximately 48-56 subjects will participate in this study. There will be four treatment groups. Groups 1 and 2 will consist of 8 subjects each who are currently taking GA. In each group of 8 subjects, 6 subjects will be randomly assigned to receive 4 doses of AE12-1-Y-QL co-administered with their current dose(s) of GA and 2 subjects to receive the matching placebo co-administered with their current dose(s) of GA. Group 3a will be comprised of a minimum of 8 subjects up to a maximum of 16 subjects, 6 to a maximum of 12 subjects will be randomly assigned to receive 4 doses of AE12-1-Y-QL co-administered with their current dose(s) of GA and 2 to a maximum of 4 subjects to receive the matching placebo co-administered with their current dose(s) of GA. Group 3b will consist of approximately 12 subjects randomly assigned to receive 4 doses of AE12-1-Y-QL co-administered with their current dose(s) of GA and 12 subjects to receive the matching placebo co-administered with their current dose(s) of GA.

Subjects in Groups 1 through 3a will have conventional MRIs performed at baseline and Days 57, 112 and 175 of the study to monitor for disease activity and safety. The conventional MRIs performed on subjects in Groups 1 through 3a will include T1 weighted (with and without Gadolinium contrast), T2 weighted, T2 weighted fluid attenuated inversion recovery (FLAIR), proton density (PD) weighted, T2*/susceptibility weighted and diffusion weighted images. Total number enrolled in Group 3b may be increased in order to provide 12 subjects in each treatment group completing through Day 112.

Subjects in Group 3b will have conventional MRIs performed at baseline and Days 57, 112 and 175 of the study to monitor for disease activity and safety as described for Groups 1-3a. Additionally, subjects in Group 3b will also have non-conventional MRI sequences performed at baseline and Day 112 of the study to evaluate the effect of AE-12-1-Y-QL on brain white matter pathophysiology. A sufficient number of subjects will be enrolled such that up to 12 subjects in each treatment arm of Group 3b complete the Day 112 visit with usable MRI data. The nonconventional MRIs will include magnetization transfer and diffusion tensor images.

**Nonconventional MRI Analyses**. Nonconventional brain MRI will be integrated into this MAD study to explore the potential effects of AE-12-1-Y-QL remyelination and axonal regeneration in patients with RFMS.

Patients treated in Group 3b receiving AE-12-1-Y-QL 1200 mg (n=12) or placebo (n=12) on maintenance GA will undergo evaluation by quantitative MRI outcome measures sensitive to remyelination and axonal regeneration (in addition to conventional MRI). Patients will derive from Stanford University and the University of California (UCSF), San Francisco investigative sites. Quantitative MRI at baseline and Day 112 post dosing will be performed on 3T MRI at the single investigative site at UCSF. The primary outcome measures will be Magnetization Transfer Ratio (MTR) and Radial Diffusivity (RD) in lesions defined on baseline T2/FLAIR MRI.

Increase in MTR and a reduction in RD within pre-existing lesions in the AE-12-1-Y-QL treated subjects relative to the placebo will be interpreted as evidence of AE-12-1-Y-QL associated improvement in axonal and myelin pathophysiology. 12 RFMS patients per group will allow detection of 5% increase in T2 lesion MTR in a baseline-adjusted analysis of placebo-controlled parallel group trial with 80% power at $\alpha=0.05$ (one-tail).

Primary MRI endpoints:

- Magnetization Transfer Ratio (MTR) at Baseline and Day 112 in lesions defined on Baseline T2/FLAIR MRI
- Radial Diffusivity (RD) at Baseline and Day 112 in lesions defined on Baseline T2/FLAIR MRI (Axial Diffusivity (AD)) may also be conducted.

For each outcome measure, the difference in mean from placebo, adjusted for baseline for each AE-12-1-Y-QL regimen, will be presented based on the ANCOVA framework. Primary statistical inference will be performed on estimate of effect at significance level of 0.05.

Assumptions for power analysis was based on MTR in T2 lesions from RRMS patients [Altmann DR et al., 2013] for a one-sided comparison of baseline- to-follow-up MTR changes between trial arms (ANCOVA). The baseline mean in T2 lesion is 30.3.The population standard deviation is the same for both the baseline measurement and the post-dose measurement. The standard deviation in T2 lesion is 1.91. The correlation coefficient between the baseline and post-dose measurements is 0.70 (data from Altmann DR et al., 2013).

All doses of AE12-1-Y-QL will be administered by intravenous (IV) infusion in the morning. Subjects will continue with their current dosing regimen of GA, i.e., Copaxone® 20 mg administered subcutaneously once a day or Copaxone® 40 mg administered subcutaneously three times weekly or generic GA 20 mg administered subcutaneously once a day. Subjects will need to have been receiving Copaxone® for at least 2 weeks prior to being randomized and must remain on the same regimen throughout the study. For each group, subjects will receive a total of 4 doses, with each dose 4 weeks apart. Higher doses will be administered only after the available safety, tolerability and pharmacokinetic data for the lower dose(s) have been reviewed and evaluated by the sponsor in consultation with the investigator.

The doses to be administered are shown in Table 9.

**Table 9**

| Group | N | Treatment[a] |
|---|---|---|
| 1 | 6 | 150 mg AE12-1-Y-QL |
| | 2 | Placebo |
| 2* | 6 | 600 mg AE12-1-Y-QL |
| | 2 | Placebo |
| 3a[b]* | 6-12 | 1200 mg AE12-1-Y-QL |
| | 2-4 | Placebo |
| 3b[c]* | 12 | 1200 mg AE12-1-Y-QL |
| | 12 | Placebo |

[a]. One dose IV every 4 weeks for a total of four doses; [b]. Group 3a will comprise of a minimum of 8, maximum of 16 total subjects; [c]. A sufficient number of subjects will be enrolled such that up to 12 subjects in each treatment arm of Group 3b complete the Day 112 visit with usable MRI data;

\* The doses may be adjusted upon review of the available safety, tolerability and pharmacokinetic data from the previous dose group(s).

Serial serum samples for determination of AE-12-1-Y-QL will be collected until 175 days after initiation of dosing. Serial serum samples for determination of anti-drug antibodies will be collected until 112 days after initiation of dosing.

Two lumbar punctures will be performed to collect CSF for the following: routine laboratory tests consisting of cell count and differential, glucose, total protein, albumin, immunoglobin; determination of AE-12-1-Y-QL concentration; total, free and bound sRGMa levels; AE-12-1-Y-QL availability for interaction with membrane bound BMP receptor using a reporter gene assay; and for biomarker analyses. The first lumbar puncture will be performed prior to the first dose (baseline), but after the baseline brain MRI; and the second lumbar puncture will be performed 27 days after the fourth dose.

After successful completion of the Screening visit, eligible subjects will undergo baseline brain magnetic resonance imaging (MRI). Baseline MRI may take place at any time following the Screening visit and at least 7 days prior to start of confinement (Day -10). If a recent MRI (within the 6-week period prior to Screening) is not available from subject's medical history at screening, then the baseline MRI will be used to determine subject eligibility. Subjects whose brain MRI show evidence of overt vascular lesions, masses, mass effect or other abnormalities other than those compatible with MS will be excluded. All subjects will undergo MRI at baseline, Days 57, 112 and 175.

Safety and tolerability will be assessed throughout the study. This will include adverse event collection, laboratory tests, neurological examination, measurements of vital signs and electrocardiograms (ECGs) and MRI scans. If needed, unscheduled relapse assessment visits will occur within 72 hours of the onset of any new neurological symptoms that may indicate the onset of a clinical relapse. These visits will consist of neurological examination, Expanded Disability Status Scale (EDSS), vital signs, blood chemistry and hematology and urinalysis. Subjects who experience a suspected MS relapse may be treated with IV methylprednisolone 1000 mg/day for 3 to 5 days.

Multiple sclerosis disease activity will be monitored during scheduled serial clinic visits and at unscheduled visits as needed. Clinical events that will be captured and recorded include relapses and disability progression measured on the expanded disability status scale (EDSS) and the Multiple Sclerosis Functional Composite (MSFC) and on the individual domains of the MSFC, the Timed 25 Foot Walk (T25FW), the 9 Hole Peg Test (9HPT) and the Paced Auditory Serial Arithmetic Test (PASAT). In addition, the patient recorded outcome measures will be obtained using the instruments Multiple Sclerosis Impact Scale (MSIS-29) and Multiple Sclerosis Quality of Life-54 (MSQOL-54).

Formal comparative statistical analyses for annualized relapse rate (ARR) and proportion of patients relapse-free will not be undertaken and results will be summarized as descriptive statistics by treatment group and visit. Disability progression can only be confirmed from the EDSS scores obtained according to the protocol-defined schedule of assessments at regular visits. The treating nurse must inform the treating neurologist if a subject experiences at least a 1.0-point increase on the EDSS from a baseline EDSS ≥1.0 that is sustained for 12 weeks that is sustained for 12 weeks. The subject must be informed they have experienced a worsening of physical disability.

Diagnosis and Main Criteria for Inclusion/Exclusion:

**Main Inclusion Criteria:** To be eligible for this study, candidates must meet the following eligibility criteria prior to randomization or at the time point specified in the individual criteria listed below:

1. Male or female and age is between 18 and 60 years, inclusive.
2. Subject is currently receiving Copaxone® 20 mg administered subcutaneously once a day or Copaxone® 40 mg administered subcutaneously three times weekly or generic glatiramer acetate 20 mg administered subcutaneously once a day for the treatment of MS for at least 2 weeks prior to randomization.
3. If female, subject must be:

- of non-childbearing potential [surgically sterile (oophorectomy, complete hysterectomy, bilateral tubal ligation), postmenopausal for at least 2 years (hormone replacement therapy is acceptable)] or
- if of childbearing potential, must practice total abstinence from sexual intercourse as the preferred life style of the subject; periodic abstinence is not acceptable; have a male monogamous sexual partner who is vasectomized at least 6 months prior to study or agree to utilize effective contraception (copper or hormonal intrauterine device (IUD), oral hormonal contraception, or double barrier protection methods) during the entire treatment and follow up period.

4. Females must have negative results for pregnancy tests prior to study drug administration
5. If male, subject must

- have documentation of having undergone male contraceptive surgery e.g., vasectomy, or
- agree to be sexually inactive or agree to us a barrier method of birth control until 90 days after the last dose of study drug, or total abstinence from sexual intercourse as the preferred life style of the subject; periodic abstinence is not acceptable.

6. Diagnosis of relapsing-remitting MS (RRMS) or secondary progressive MS, (SPMS) known commonly as relapsing forms of MS (RFMS).
7. Subjects with RRMS must have a confirmed diagnosis according to revised McDonald criteria and those subjects with RFMS must have evidence of ongoing disease activity as evidenced by:

- at least 1 relapse within the 12 months prior to randomization, with a cranial MRI demonstrating lesion(s) consistent with MS (it is not necessary to obtain a current scan if a scan performed within the 6 months prior to Screening is available from the subject's history; if a scan is not available from the subject's history, then the baseline scan may be used). Relapses are defined as new or recurrent neurological symptoms not associated with fever or infection, lasting at least 24 hours, and accompanied by new objective neurological findings upon examination by the examining neurologist. The subject must have objective signs on the examining neurologist's examination confirming the event. Time since relapse should be measured from the time of relapse onset, OR
- show evidence of Gd-enhancing lesions of the brain on an MRI performed within the 6 months prior to randomization (if scan is not available from the subject's history, then baseline scan may be used).

8. Neurologically stable at the Screening Visit, in the investigator's judgment and not actively experiencing or recovering from a recent relapse in the 30 days preceding the Screening Visit.

9. Must have a baseline EDSS between 1.0 and 6.0, inclusive.

10. Body Mass Index (BMI) is 18.0 to 32.0, inclusive. BMI is calculated as weight in kg divided by the square of height measured in meters.

11. Has a brain MRI scan, interpreted by a radiologist, that did not show evidence of overt vascular lesions, masses, mass effect or other abnormalities other than those compatible with MS, which would preclude the subject from undergoing a lumbar puncture/spinal tap for CSF collection, baseline MRI scan interpreted by a radiologist at least 7 days prior to confinement, may be used.

12. A condition of general good health, except for MS, based upon the results of a medical history, physical examination, vital signs, laboratory profile, neurological examination and a 12-lead electrocardiogram (ECG).

**Exclusion Criteria:** A subject will not be eligible for study participation if he/she meets any of the following criteria:

Medical History:

1. Diagnosis of primary progressive MS.

2. History or abnormal laboratory results that, in the opinion of the investigator, are indicative of any significant cardiac, endocrinologic, hematologic, hepatic, immunologic, metabolic, urologic, pulmonary, gastrointestinal, dermatologic, psychiatric, renal, neurologic (other than MS), and/or other major disease that would preclude administration of AE-12-1-Y-QL or GA.

3. An MS relapse that has occurred within the 30 days prior to randomization AND/OR the subject has not stabilized from a previous relapse prior to randomization

4. Subjects for whom MRI is contraindicated, (i.e., aneurysm clip, metal fragments, internal electrical devices such as a cochlear implant, spinal cord stimulator or pacemaker), contraindicated for or allergic to gadolinium (including renal impairment, abnormal eGFR, previous diagnosis of nephrogenic systemic fibrosis and allergy), have claustrophobia that cannot be medically managed or are unable to lie still for 1 hour or more for the imaging procedures.

5. Receipt of any depot drug by injection within 30 days prior to study drug administration.

6. Receipt of an investigational product within a time period equal to 10 half-lives, if known, or within 6 weeks months prior to study drug administration.

7. Positive screen for drugs of abuse or alcohol as detected at Screening or Day -3.

8. History of malignancy; however, subjects with a history of excised or treated basal cell carcinoma or fewer than 3 squamous cell carcinomas are eligible to participate in this study.

9. Known hypersensitivity to study drugs or their excipients.

10. History of drug or alcohol abuse within 2 years prior to study drug administration.

11. History of severe allergic or anaphylactic reactions.

12. History of seizure disorder or unexplained blackouts OR history of a seizure within 6 months.

13. History of suicidal ideation or an episode of clinically severe depression within 1 month prior to study drug administration as evidenced by answering "yes" to questions 4 or 5 on the suicidal ideation portion of the Columbia-Suicide Severity Rating Scale (C-SSRS) completed at Screening, or any history of suicide attempts.

14. Known history of, or positive screening test result for hepatitis C or hepatitis B virus.

15. Varicella or herpes zoster virus infection or any severe viral infection within 6 weeks before screening.

16. Exposure to varicella zoster virus within 21 days before screening.

17. History of human immunodeficiency virus (HIV) or other immunodeficient conditions.

18. Any type of live virus vaccine less than 4 weeks before randomization, including but not limited to: measles/mumps/rubella vaccine, varicella zoster virus vaccine, oral polio vaccine, and nasal influenza vaccine.

19. Infection (viral, fungal, bacterial) requiring hospitalization or intravenous (IV) antibiotics within 8 weeks before randomization.

20. Elective surgery performed from 2 weeks prior to randomization or scheduled through the end of the study.

21. Findings on brain MRI scan indicating any clinically significant brain abnormality other than MS.

22. Any of the following abnormal blood tests at screening:

- Hemoglobin $\leq 10.0$ g/dL
- Platelets $\leq 100 \times 10^9$/L
- Lymphocytes $\leq 1.0 \times 10^9$/L
- Neutrophils $\leq 1.5 \times 10^9$/L
- Alanine aminotransferase/serum glutamate pyruvate transaminase (ALT/SGPT), aspartate aminotransferase/serum glutamic oxaloacetic transaminase (AST/SGOT), or gamma glutamyl-transferase ≥ 2 times the upper limit of normal (ULN)

- Serum iron, ferritin, transferrin saturation > ULN.
- Serum creatinine ≥ ULN.

23. Contraindication for lumbar puncture (e.g., lumbar scoliosis, coagulopathy, or hypocoagulation medications, infected skin at needle puncture site)

24. Donation or loss of 550 mL or more blood volume (including plasmapheresis) or receipt of a transfusion of any blood product within 8 weeks prior to study drug administration.

Treatment History:

25. Prior treatment with the any of the following:

- Total lymphoid irradiation
- Cladribine or mitoxantrone
- T cell or T cell receptor vaccination

26. Prior treatment with cyclophosphamide or alemtuzumab, within 1 year prior to randomization.

27. Prior treatment with any of the following medications or procedures within the 6 months prior to randomization:

- Natalizumab
- Rituximab
- Daclizumab
- Cyclosporine
- Azathioprine
- Methotrexate
- Mycophenolate mofetil
- Intravenous immunoglobulin (IVIg)
- Plasmapheresis or cytapheresis

28. Treatment with any of the following medications within the 30 days prior to randomization:

- IV corticosteroid treatment
- Oral corticosteroid treatment
- Beta-interferon
- Fingolimod
- Dimethyl fumarate
- Teriflunomide

29. Initiation of treatment or dose adjustment of commercially available Fampridine-SR within the last 90 days. It is acceptable if a patient already is receiving a stable dose of Fampridine-SR prior to randomization and plans to remain on this dose and regimen throughout study.

**Pharmacokinetics:** The following values for the pharmacokinetic parameters will be estimated using non-compartmental methods: maximum observed serum concentration ($C_{max}$), the time to $C_{max}$ (peak time, $T_{max}$) and the area under the concentration time curve (AUC) from time 0 to the time of the last measurable concentration ($AUC_t$) will be estimated after the first and fourth doses. The observed serum concentration prior to dose ($C_{trough}$), terminal phase elimination rate constant ($\beta$), terminal phase elimination half-life ($t_{1/2}$) and apparent clearance (CL/F) will be estimated after the fourth dose. Anti-drug antibodies will be determined for assessment of immunogenicity. Additional parameters may be calculated if useful in the interpretation of the data.

**CSF Biomarkers:** A panel of biomarkers representing markers for pro- and anti-inflammation, neuroregeneration/neuroprotection, neurodegeneration, and/or remyelination will be assessed.

**Pharmacodynamics:** Exploratory Brain MRI Outcomes (Group 3b)

The study will include MRI outcome measures sensitive to changes in axonal and myelin pathophysiology in the brain and disease activity as exploratory endpoints. The following outcome measures will be evaluated:

Primary MRI endpoints:

- Magnetization Transfer Ratio (MTR) at Baseline and Day 112 in lesions defined on Baseline T2/FLAIR MRI

- Radial Diffusivity (RD) at Baseline and Day 112 in lesions defined on Baseline T2/FLAIR MRI

- Total number of new Gadolinium-enhancing T1 lesions across Day 57 and Day 112

- Total number of new or newly-enlarging T2 hyperintense lesions at Day 112

- Total lesion volume of new and newly enlarging T2 hyperintense lesions at Day 112

Secondary MRI Endpoints:

Fractional Anisotropy (FA) and Axial Diffusivity (AD) at Baseline and Day 112 in lesions defined on Baseline T2/FLAIR MRI
MTR in Normal-Appearing Gray Matter (NAGM) defined on baseline MRI at Baseline and Day 112
MTR and FA in Normal-Appearing White Matter (NAWM) defined on baseline MRI at Baseline and Day 112
**Safety:** The safety variable will include the following: adverse event monitoring, vital signs, physical examination, neurological examination, electrocardiograms, laboratory tests assessments, and C-SSRS and MRI scans. If needed, unscheduled relapse assessment visits will occur within 72 hours of the onset of any new neurological symptoms that may indicate the onset of a clinical relapse. These visits will consist of neurological examination, EDSS, vital signs, blood chemistry and hematology, urinalysis and MRI if necessary. Subjects who experience a suspected MS relapse or who are found to have at least 2 new T1 gadolinium enhancing lesions or 1 new T1 gadolinium enhancing lesion in a critical area on the Day 57 safety MRI may be treated with IV methylprednisolone 1000 mg/day for 3 to 5 days,
**[0236]** Adverse events will be coded by Medical Dictionary for Regulatory Activities (MedDRA). For each of the individual groups, the number and percentage of subjects reporting treatment-emergent adverse events will be tabulated by MedDRA Preferred Term and System Organ Class. Tabulations will also be provided in which the number of subjects reporting an adverse event (MedDRA term) is additionally broken down by rating (mild, moderate or severe) and by whether possibly related to study drug. Any deaths, other serious adverse events and other significant adverse events will be separately identified. Laboratory test values and measurements on vital signs that are potentially clinically significant, according to predefined criteria, will be identified.

**Example 4**

**A [11C]-PBR28 Positron Emission Tomography Study to Evaluate the Effect of AE-12-1-Y-QL on Central Nervous System Inflammation in Subjects with Relapsing Forms of Multiple Sclerosis**

**Study Design:** This is an open-label positron emission tomography to examine the effect of AE-12-1-Y-QL on translocator protein expression (TSPO) in the central nervous system of subjects with relapsing forms of multiple sclerosis. Approximately 24 subjects with relapsing forms of multiple sclerosis with stable disease will be enrolled in this study. The objective of this study is to investigate the effect of single dose of AE-12-1-Y-QL on [11C]-PBR28 radioligand binding to translocator protein (TSPO) in the brain of subjects with relapsing forms of multiple sclerosis (RFMS) and to assess the safety and tolerability of a single dose of AE-12-1-Y-QL in this subject population.
**Background and Rationale for PET Imaging:** The 18 kilodalton translocator protein (TSPO) is a mitochondrial protein, initially described for its ability to bind a variety of benzodiazepine drugs and thus also previously known as peripheral benzodiazepine receptor (PBR). TSPO expression is present throughout the body and the CNS and is relatively high in microglia, macrophages and peripherally recruited (monocyte derived macrophages) myeloid cells. Focal areas of CNS inflammation in MS brains reveal increased density of activated microglial and macrophage immune cells accompanied by increased expression of TSPO relative to normal healthy brain tissue. The magnitude of TSPO density in these focal lesions of MS brains has been demonstrated to be strongly correlated with the density of microglial and macrophage immune cells and with MS disease severity. For this reason, the TSPO targeting positron emission tomography (PET) radioligands such as [11C]-PK11195, [11C]-PBR28, [18F]-PBR111 have been applied to study disease processes that involve microglial activation or the recruitment of macrophages, such as MS and to investigate the effect of novel therapeutic agents on the innate immune system in the brain of patients with CNS diseases.
[11C]-PBR28 is a selective and high binding affinity PET radioligand for TSPO. [11C]-PBR28 dosimetry and whole body biodistribution have been completed in human subjects and demonstrated an effective radiation exposure dose of 6.6 $\mu$Sv/MBq acceptable for human use. [11C]-PBR28 has the characteristics of an optimal PET radioligand and has been successfully used to quantify changes in TSPO levels in healthy volunteers and patients with multiple sclerosis, Alzheimer's disease and Parkinson's disease, thus supporting the use of this radioligand in clinical studies. Given the relatively low dose required to detect binding in the brain, this allows for multiple brain PET scans of good image quality to be performed in an individual subject. Test-retest studies in healthy volunteers have demonstrated an intra-class correlation for equilibrium volume distribution ($V_T$) of greater than 0.9 in most brain regions.

This open-label PET study employing a selective high affinity TSPO radioligand [$^{11}$C]-PBR28 is designed to determine the effect of AE-12-1-Y-QL on TSPO expression level in the central nervous system (CNS) of subjects with relapsing forms of multiple sclerosis. Findings from this study may demonstrate whether AE-12-1-Y-QL has immune modulatory effect in the CNS and will provide information on the safety and tolerability of AE-12-1-Y-QL as monotherapy in MS patients.

**Methodology:** This is an open-label positron emission tomography (PET) study using the selective high affinity translocator protein (TSPO) radioligand [$^{11}$C]-PBR28 to examine the effect of AE-12-1-Y-QL on TSPO expression in the central nervous system of subjects with relapsing forms of multiple sclerosis (RFMS). Approximately 24 subjects with RFMS with stable disease will be enrolled in the study according to the selection criteria to ensure at least 18 subjects will complete all study visits and have evaluable imaging data for all time points (4 in Part I and 14 in Part II). Subjects participating in Part I of the study will not be eligible to participate in Part II of the study. The study will be divided into two parts as shown below.

**Part I (Test-Retest Assessment):** Part I examined the within subject test retest variability of [$^{11}$C]-PBR28 PET outcome measures over an interval of 15 +/- 5 days. Subjects were not administered AE-12-1-Y-QL. Four subjects with RFMS completed all study visits and had evaluable imaging data for all time points for Part I. Evaluable subjects are defined as subjects who complete the baseline magnetic resonance imaging (MRI) and the two scheduled PET imaging sessions with acceptable quality of the MRI, PET images and arterial data as determined by the Sponsor for each time point. Subjects completed 2 dynamic PET scans of the brain with arterial sampling and 1 MRI scan of the brain over the course of 4 visits as follows:

- Visit 1: Screening
- Visit 2: Baseline MRI scan
- Visit 3: Baseline PET scan
- Visit 4: Follow-up PET scan

**Part II (AE-12-1-Y-QL Treatment Group):** Part II will begin after completion of Part I and will examine the effect of single intravenous administration of AE-12-1-Y-QL on TSPO expression in the central nervous system of subjects with RFMS. Based on the available information, AE-12-1-Y-QL dose of 1600 mg was chosen for this study. The dose for the current study may be lowered or increased to up to 2400 mg based on safety and pharmacokinetic information obtained from Example 1. The dose may also be adjusted based on the available safety and pharmacodynamics results from all previous subjects in the current study. At least 14 subjects with RFMS will complete all study visits and have evaluable imaging data for all time points for Part II. Subjects will complete 2 dynamic PET scans of the brain with arterial sampling and 2 MRI scans of the brain over the course of 6 visits as follows:

- Visit 1: Screening
- Visit 2: Baseline MRI
- Visit 3: Baseline PET scan
- Visit 4: AE-12-1-Y-QL administration
- Visit 5: Follow-up PET scan
- Visit 6: Follow-up clinical and safety assessments including MRI

**Screening:** In both Part I and Part II, subject eligibility will be evaluated at Visit 1 which will occur within 30 days prior to Visit 3. Visit 2 may take place at any time following the Screening (Visit 1) and at least 7 days prior to PET scan (Visit 3).

Following procedures will be performed to determine subject's eligibility for the study: medical history, physical examination (including height, weight and BMI), neurological exam, Columbia-Suicide Severity Rating Scale (C-SSRS), clinical laboratory assessment (standard hematology, clinical chemistry and urinalysis), screens for hepatitis, HIV, urine test for drugs of abuse and alcohol screen, 12-lead ECG, vital signs (blood pressure, heart rate), review of concomitant medications, test for pregnancy, Allen's test and blood collection to determine TSPO genetic polymorphism.

**Magnetic Resonance Imaging (MRI).** After successful completion of the Screening (Visit 1), all eligible subjects will undergo baseline brain MRI (Visit 2). Visit 2 may take place at any time following the Screening (Visit 1) and at least 7 days prior to PET scan (Visit 3). All scheduled MRI will be performed at Imanova Imaging Center on a 3 Tesla system. Subjects whose brain MRI show evidence of overt vascular lesions, masses, mass effect or other abnormalities other than those compatible with MS will be excluded. The baseline MRI will additionally be used to delineate demyelinating lesions and anatomical regions of interest (ROI) for individual PET images. For MRI imaging, intravenous catheter (for Gadolinium contrast agent infusion) will be inserted according to standard clinical practice to all subjects. The following imaging sequence types will be performed on all eligible subjects at Visit 2 (Part I); Visit 2 and Visit 6 (Part II):

- T1 weighted
- Diffusion weighted imaging
- T2 weighted FLAIR
- PD weighted
- T2 weighted
- T1 weighted with gadolinium

Additional imaging sequences may be included. The total imaging session is expected to be approximately 60 minutes in duration and will not exceed 90 minutes. Detailed scanning parameters, sequences, imaging planes and the order in which the acquisitions are required to be performed will be provided in the study Imaging Manual.

**Arterial Catheter Placement and Monitoring:** At the Imanova Imaging Center, prior to the scheduled PET scans, a radial-arterial catheter for blood sampling will be inserted in all subjects. The procedure should be performed by an anesthesiologist or appropriately trained physician and monitored throughout the study by a trained nurse. Risks of radial artery cannulation will be minimized by having the procedure performed by an experienced physician. Pain will be minimized by using local anesthesia. Bleeding will be prevented by local pressure or pressure dressing applied for a minimum of 10 minutes after catheter removal. Subjects will have their hand and finger blood supply examined after arterial cannulation and again following catheter removal. Also, subjects will be asked to abstain from using aspirin, non-steroidal anti-inflammatory drug (NSAIDS) or anticoagulants. Subjects will be provided a 24-hour emergency physician telephone number to call if they encounter pain, discoloration, numbness, tingling, coolness, or any other unusual symptoms in the wrist or hand, fever, chills or drainage from the vascular puncture sites following the procedure. Subjects will be verbally instructed regarding problems to watch for and procedures to follow should such problems occur. Infection is to be avoided by adequate cleansing of the skin prior to intravascular line insertion.

The site of cannulation may be alternated between the two wrists between subsequent PET scan visits based upon the judgment of the anesthesiologist or the physician performing the procedure. Re-cannulation of the same site in a patient can be performed if the procedures are separated by at least a week.

**Positron Emission Tomography (PET)** In Part I, subjects were not administered AE-12-1-Y-QL and underwent PET imaging session at two time points (Visits 3 and 4) for the test and retest scans. Visit 4 were 15 +/- 5 days after Visit 3.

In Part II, subjects will be administered AE-12-1-Y-QL and will undergo PET imaging session at two time points: one at baseline (Visit 3) and another scan (Visit 5) 15 +/- 5 days following AE-12-1-Y-QL dose (Visit 4). The interval between Visit 4 and 5 for Part II may be adjusted by the Sponsor based on the pharmacokinetic information obtained from the single ascending dose of Example 1 and the available pharmacodynamics results from previously scanned subjects in the current study.

For PET imaging, intravenous catheter (for radioligand infusion) and radial-arterial catheter (for radioligand measurements) will be inserted according to standard clinical practice in all subjects.

Low-dose Computed Tomography (CT) scans will be obtained to correct for the attenuation of the positron emission signal. After completion of the CT scan subjects will receive an IV bolus injection (infused over a period approximately to 20 seconds) of not more than 400 MBq of [$^{11}$C]-PBR28. The exact mass of [$^{11}$C]-PBR28 to be administered will be determined immediately prior to dosing, but will not exceed 10 $\mu$g. The dynamic brain PET data acquisition will begin simultaneously with the administration of the radioligand and continue for approximately 90 minutes.

Serial blood samples for pharmacokinetic assays of [$^{11}$C]-PBR28 will be collected from the arterial catheter for arterial input function estimation, radiometabolite analysis and plasma free fraction estimation.

**[$^{11}$C]-PBR28 Dosing and Administration:** Subjects in Parts I and II will each receive two doses of the PET radioligand [$^{11}$C]-PBR28, one immediately prior to each PET scan. Due to the relatively short half-life of carbon-11 (approximately 20 minutes), [$^{11}$C]-PBR28 will be prepared onsite at Imanova Imaging Center immediately prior to administration.

Each subject will be exposed to a maximum radiation dose of 400 MBq of [$^{11}$C]-PBR28 in each of their PET scans. In addition, one low-dose CT scan of the head may be acquired on each PET visit to estimate attenuation correction. The effective dose from this study for each subject will be up to 5.28 mSv from the two [$^{11}$C]-PBR28 dosings combined and 0.72 mSv from the low-dose CT scans combined, yielding 6 mSv in total. In the rare event of an equipment failure or unusable data, the subject may undergo a maximum of 3 PET/CT imaging sessions and the maximum effective dose from this study will be up to 7.92 mSv from the three [$^{11}$C]-PBR28 dosing combined and 1.08 mSv from the three low-dose CT scans combined, yielding 9 mSv in total. This study will therefore fall within category IIb of the International Commission on Radiological Protection (ICRP): less than 10 mSv in addition to natural background radiation in the previous 3 years including the dose from this study.

<u>Image Analysis and PET Pharmacokinetic Modeling:</u> PET images will be reconstructed with scatter and attenuation correction, and automatically corrected for motion via frame to frame registration. The PET images will be co-registered to the individual's structural MRI image. Anatomical regions of interest (ROIs) will be delineated based on the MRI data, and applied to individual dynamic PET data to generate regional time activity curves. Estimates of volumes of distribution ($V_T$) for each ROI will be obtained using appropriate pharmacokinetic models with the parent arterial plasma input function. For the primary analysis, an optimal pharmacokinetic modeling approach (two tissue compartmental model or graphical model) will be determined from Part I and will be applied to Part II. The two tissue compartmental model will be preferred for the primary analysis. However, in the case of poor goodness of fit or if the model is not identifiable then the graphical model approach will be pursued. Data from all subjects from all time points will be subject to a single analysis approach. As a pre-specified sensitivity analysis an appropriate alternate pharmacokinetic modeling approach (specifically, two tissue compartmental model versus graphical model) will be applied to Part I and Part II data. The volume of distribution ratio (DVR) for each ROI will also be estimated based on the selection of a pseudo reference region in order to account for the variability in the blood.

PET images of both Parts I and II will be examined in an attempt to identify a reference region that is appropriate across all the subjects. The reference region will be chosen based on the following criteria:

- consistent across all subjects
- defined within the gray matter or normal appearing white matter
- stable $V_T$ values across the two PET scans such that

> o the difference in means between the two PET scans is not statistically significant and
> o the average absolute variability between the two PET scans is < 20%
> across all the subjects, where absolute variability is defined by

$$\text{absolute variability} = 100 \times \frac{|V_{T.1} - V_{T.2}|}{(V_{T.1} + V_{T.2})/2}$$

All subjects in Part II will visit the study site 70 +/- 5 days after the dose of AE-12-1-Y-QL for clinical and safety assessments. Following procedures will be performed: physical examination, neurological examination, C-SSRS (Part II only), clinical laboratory assessment (standard hematology, clinical chemistry and urinalysis), 12-lead ECG, vital signs (blood pressure, heart rate), MRI, and pregnancy test.

**Safety:** Safety will be assessed throughout the study. If needed, unscheduled relapse assessment visits will occur, when possible, within 72 hours of the onset of any new neurological symptoms that may indicate the onset of a clinical relapse. This will include adverse event collection, laboratory tests, physical examination, neurological examination, measurements of vital signs and ECGs.

Diagnosis and Main Criteria for Inclusion/Exclusion:

**Main Inclusion:** A subject will be eligible for study participation if he/she meets the following criteria:

1. Male or female, between 18 and 60 years of age, inclusive, at Screening.
2. If female:

- Non-childbearing potential (surgically sterile [oophorectomy, complete hysterectomy, bilateral tubal ligation], postmenopausal for at least 2 years [hormone replacement therapy is acceptable]), or
- Childbearing potential who provide a negative pregnancy test at:

> o screening, prior to each [11C]-PBR28 administration and within 24 hours of administration of study drug; and
> o Must practice total abstinence from sexual intercourse as the preferred life style of the subject; (periodic abstinence is not acceptable; or have a male monogamous sexual partner who is vasectomized at least 6 months prior to study; or agree to utilize effective contraception (copper or hormonal intrauterine device [IUD]) during the entire treatment and follow-up period.

3. If male, must have documentation of having undergone male contraceptive surgery e.g., vasectomy, or agree to be sexually inactive or agree to use a barrier method of birth control until 90 days after the dose of study drug.

4. Diagnosis of relapsing-remitting MS (RRMS) or relapsing-secondary progressive MS (SPMS), known as relapsing forms of MS (RFMS). Patients with RRMS must have a confirmed diagnosis according to revised McDonald criteria and the RFMS patients must have evidence of ongoing disease activity as evidenced by:

- Have experienced at least 1 relapse within the 12 months prior to randomization, with a cranial MRI demonstrating lesion(s) consistent with MS (it is not necessary to obtain a current scan if prior MRI scan performed within the 6 months prior to Screening is available from the subject's history). For inclusion purposes, a relapse is defined as neurologic signs and/or symptoms documented by a neurologist in the medical record and of at least 24 hours duration to be determined by the Investigator or the Treating Neurologist. Time since relapse should be measured from the time of relapse onset, OR
- Show evidence of Gadolinium (Gd)-enhancing lesions of the brain on an MRI performed within the 6 months prior to Screening.

5. Neurologically stable at the Screening Visit, in the investigator's judgment and not actively experiencing or recovering from a recent relapse in the 30 days preceding the Screening Visit.

6. A Kurtzke Expanded Disability Status Scale (EDSS) score of 1.0 to 6.0, inclusive at the Screening Visit.

7. High or mixed affinity binder of the TSPO, as determined by rs6971 polymorphism genotyping at Screening.

8. Body Mass Index (BMI) is 18.0 to 35.0, inclusive, at Screening.

9. With the exception of MS, the subject is in general good health, based upon the results of a medical history, physical examination, vital signs, laboratory profile, and a 12-lead electrocardiogram.

**Main Exclusion:** A subject will not be eligible for study participation if he/she meets any of the following criteria:

1. Diagnosis of primary progressive or non-relapsing secondary progressive MS.

2. Receipt of any depot drug by injection (except contraceptives) within 30 days prior to study drug administration.

3. Receipt of an investigational product within a time period equal to 10 half-lives, if known, or within 6 weeks prior to study drug administration.

4. Positive screen for drugs of abuse or alcohol.

5. Smoking more than 10 cigarettes per day or use of a nicotine patch.

6. History of malignancy; however, subjects with a history of excised or treated basal cell carcinoma or fewer than 3 squamous cell carcinomas are eligible to participate in this study.

7. Known hypersensitivity to study drugs or their excipients.

8. History of drug or alcohol abuse within 2 years prior to study drug administration.

9. History of severe allergic or anaphylactic reactions.

10. History of seizure disorder or unexplained blackouts or history of a seizure within 6 months prior to Screening.

11. History of suicidal ideation or an episode of clinically severe depression within 1 month prior to study drug administration as evidenced by answering "yes" to questions 4 or 5 on the suicidal ideation portion of the Columbia-Suicide Severity Rating Scale (C-SSRS) completed at Screening, or any history of suicide attempts.

12. Known history of, or positive screening test result for hepatitis B virus or hepatitis C virus.

13. Varicella or herpes zoster virus infection or any severe viral infection within 6 weeks prior to Screening.

14. Exposure to varicella zoster virus within 21 days prior to Screening.

15. History of human immunodeficiency virus (HIV) or other immunodeficient conditions.

16. Any type of live virus vaccine from 4 weeks before Visit 2, including but not limited to: measles/mumps/rubella vaccine, varicella zoster virus vaccine, oral polio vaccine, and nasal influenza vaccine.

17. Infection (viral, fungal, bacterial) requiring hospitalization or intravenous (IV) antibiotics within 8 weeks before Visit 2.

18. Elective surgery performed from 14 days prior to Visit 2 or scheduled through the end of the study.

19. History of abnormal laboratory results that, in the opinion of the Investigator, are indicative of any significant cardiac, endocrine, hematological, hepatic, immunologic, metabolic, urologic, pulmonary, gastrointestinal, dermatologic, psychiatric, renal, neurological (other than MS), and/or other major disease that would preclude administration of AE-12-1-Y-QL.

20. Any of the following abnormal blood tests at Screening:

- Hemoglobin $\leq$ 10.0 g/dL
- Platelets $\leq$ 100 $\times$ $10^9$/L
- Lymphocytes $\leq$ 1.0 $\times$ $10^9$/L

- Neutrophils $\leq 1.5 \times 10^9$/L
- Alanine aminotransferase/serum glutamate pyruvate transaminase (ALT/SGPT), aspartate aminotransferase/serum glutamic oxaloacetic transaminase (AST/SGOT), or gamma glutamyl-transferase $\geq 2$ times the upper limit of normal (ULN)
- Serum creatinine $\geq$ ULN
- APTT $\geq$ ULN
- INR $\geq$ ULN
- eGFR $\leq 30$ mL/min/1.73 m2

21. Donation or loss of 550 mL or more blood volume (including plasmapheresis) or receipt of a transfusion of any blood product within 8 weeks prior to study drug administration.

22. An MS relapse that has occurred within the 30 days prior to Screening and/or the subject has not stabilized from a previous relapse prior to Screening.

23. Subjects who are unable or unwilling to undergo MRI or PET procedures.

24. Subject has a contraindication to arterial line insertion determined by abnormal Allen's test or abnormal coagulation profile at screening

25. Subjects for whom MRI is contraindicated, (i.e., aneurysm clip, metal fragments, internal electrical devices such as a cochlear implant, spinal cord stimulator or pacemaker), contraindicated for are allergic to gadolinium (including renal impairment, abnormal eGFR, previous diagnosis of nephrogenic systemic fibrosis and allergy), have claustrophobia that cannot be medically managed or are unable to lie still for 1 hour or more for the imaging procedures.

26. Subjects with history of prior radiation exposure for research purposes within the past year such that participation in this study would place them over International Commission on Radiological Protection (ICRP)/Radioactive Drug Research Committee (RDRC) limits for annual radiation exposure. This guideline is an effective dose of 10 mSv received per year.

27. Findings on brain MRI scan indicating any clinically significant brain abnormality other than MS.

28. Subject has a past history of cerebrovascular disease or vasculitis.

29. Homozygous for the low-affinity binding form of TSPO by TSPO genotype analysis (Ala147Thr polymorphism in rs6971 SNP in exon 4 of the TSPO gene) at Screening.

30. Use of high dose diazepam 5 half-life prior to PET imaging session.

31. Prior treatment with the any of the following:

- Total lymphoid irradiation
- Cladribine or mitoxantrone
- T cell or T cell receptor vaccination

32. Prior treatment with cyclophosphamide or Alemtuzumab within 1 year prior to Visit 2.

33. Prior treatment with any of the following medications or procedures within 6 months prior to Visit 2:

- Natalizumab
- Rituximab
- Daclizumab
- Cyclosporine
- Azathioprine
- Methotrexate
- Mycophenolate mofetil
- Intravenous immunoglobulin (IVIg)
- Plasmapheresis or cytapheresis

34. Treatment with any of the following medications within the 30 days prior to Visit 2:.

- IV corticosteroid treatment

- Oral corticosteroid treatment

- Glatiramer acetate

- Fingolimod

- Dimethyl fumarate

- Teriflunomide

- Beta-interferon

35. Initiation of treatment or dose adjustment of commercially available Fampridine-SR within the last 90 days prior to Screening (subjects who have been on a stable dose of commercially available Fampridine-SR for longer than 90 days are not excluded. Use of compounded or other formulations of 4-aminiopyridine is excluded).

36. Female subjects considering becoming pregnant while in the study.

37. Female subjects who are currently pregnant or breastfeeding.

**Safety and Tolerability:** Adverse event monitoring, vital signs (blood pressure, heart rate, ECGs), clinical laboratory assessments, neurological assessments, C-SSRS (Part II Only) and brain MRI.

**Pharmacokinetic** (Part II Only): Serum concentrations of AE-12-1-Y-QL and anti-drug antibodies will be determined from the samples collected.

**Blood Samples for [$^{11}$C]-PBR28 PET Pharmacokinetic Modeling.** Prior to each scheduled PET imaging session, a radial-arterial catheter, for arterial blood sampling will be inserted in all subjects. A continuous sampling system will be used to measure whole blood activity each second for the first 15 minutes of each scan. Approximately 75 mL of whole blood will be sampled for each scan during continuous sampling. In addition a total of approximately 60 mL of discrete blood samples will be withdrawn to facilitate measurement of whole blood and plasma activity at the following time points in Parts I and II after start of scan: 5, 10, 15, 20, 25, 30, 40, 50, 60, 70, 80, and 90 minutes. Samples taken at 5, 10, 15, 20, 30, 50, 70 and 90 minute time points will be analyzed using HPLC to determine the fraction of parent radioactivity in arterial plasma. In addition, two samples (up to 7 mL for each sample) will be drawn prior to [$^{11}$C]-PBR28 injection for estimation of the free fraction (not bound to protein) of parent [$^{11}$C]-PBR28 in plasma.

**Pharmacodynamic:** Equilibrium volume of distribution ($V_T$) will be determined in tissue regions identified below. $V_T$ is defined as the ratio of radioligand concentration in a tissue region of interest to that observed in the plasma. A corresponding volume of distribution ratio (DVR) for each of the tissue regions may also be determined. DVR is defined as the ratio of $V_T$ in a tissue region of interest to that in an appropriately chosen reference tissue. PET images of both Parts I and II will be examined in an attempt to identify a reference region that is appropriate across all the subjects. If an appropriate reference region is
identified, values of DVR will be reported.

Primary Variables:

- $V_T$ in lesion and peri-lesion regions of interest defined on baseline T2/FLAIR MRI

Secondary Variables:

- $V_T$ in cortical and subcortical gray matter regions of interest
- $V_T$ in normal-appearing white matter regions of interest defined on baseline MRI
- $V_T$ in Gd enhancing lesion and peri-lesion regions of interest defined on baseline MRI
- DVR for the $V_T$ variables will be reported if an appropriate pseudo reference region is identified

**Safety:** The safety variable will include the following: Adverse event monitoring, vital signs, physical examination, neurological examination, electrocardiograms, laboratory tests assessments, and Columbia-Suicide Severity Rating Scale (Part II only) and MRI scans. If at any time during the study an MS relapse occurs, an unscheduled Relapse Assessment Visit will occur within 72 hours of the onset of any new neurological symptoms that may indicate the onset of a clinical relapse. These visits will consist of neurological examination, EDSS, vital signs, blood chemistry and hematology, urinalysis, and MRI, if indicated. Subjects who experience a suspected MS relapse may be treated with IV methylprednisolone 1000 mg/day for 3 to 5 days. Subjects who experience a confirmed MS relapse or who have new T1 gadolinium enhancing lesions as described above will be required to re-consent for continued study participation. After the Visit 5 in Part II, subjects who experienced a suspected MS relapse during the study or who are found to have at least 2 new T1 gadolinium enhancing lesions or 1 new T1 gadolinium enhancing lesion in a critical area can be started on open-label alternative, approved MS therapy for the duration of the study.

**Blood Sample for TSPO Genetic Polymorphism:** A single 5 mL whole blood sample will be collected at Screening for genotype analysis for the polymorphism in rs6971 SNP in exon 4 of the TSPO gene.

**Preliminary Results from Part I**. The intra-subject coefficient of variation of lesion and perilesion volume of distribution ($V_T$) was estimated to be 15%.

**Example 5**

**Dosing Study of AE12-1-Y-QL in Subjects with Relapsing Forms of Multiple Sclerosis**

The primary objective of this study is to assess the safety, tolerability, pharmacokinetics and immunogenicity of AE12-1-Y-QL in subjects with Relapsing Forms of Multiple Sclerosis (RFMS) who are on maintenance glatiramer acetate (GA) treatment. The secondary objective is to assess the effect of AE12-1-Y-QL on the clinical and neuroimaging parameters of Multiple Sclerosis (MS) disease activity.

**Study Population:** Adult male and female subj ects with RFMS who are on maintenance GA treatment.

**Methodology:** This is a two-part, double-blind, placebo-controlled, randomized, escalating multiple-dose study. Up to 56 subjects will participate in this study. Part 1 will consist of up to four treatment groups (Groups 1-4) and Part 2 will consist of one treatment group (Group 5). Groups 1-4 will consist of 8 subjects each who are currently taking GA. In each group of 8 subjects, 6 subjects will be randomly assigned to receive 4 doses of AE12-1-Y-QL co-administered with their current dose(s) of GA and 2 subjects to receive the matching placebo co-administered with their current dose(s) of GA. Group 5 will consist of approximately 12 subjects randomly assigned to receive 4 doses of AE12-1-Y-QL co-administered with their current dose(s) of GA and 12 subjects to receive the matching placebo co-administered with their current dose(s) of GA.

Subjects in Groups 1 through 5 will have conventional MRIs performed at baseline and Days 57, 113 and 176 of the study to monitor for disease activity and safety. The conventional MRIs performed on subjects in Groups 1 through 4 will include T1 weighted (with and without Gadolinium contrast), T2 weighted, T2 weighted fluid attenuated inversion recovery (FLAIR), proton density (PD) weighted, T2*/susceptibility weighted and diffusion weighted images. Total number enrolled in Group 5 may be increased in order to provide 12 subjects in each treatment group completing through Day 113.

Subjects in Group 5 will have conventional MRIs performed at baseline and Days 57, 113 and 176 of the study to monitor for disease activity and safety as described for Groups 1-4. Additionally, subjects in Group 5 will also have non-conventional MRI sequences performed at baseline and Day 113 of the study to evaluate the effect of AE-12-1-Y-QL on brain white matter pathophysiology. A sufficient number of subjects will be enrolled such that up to 12 subjects in each treatment arm of Group 5 complete the Day 113 visit with usable MRI data. The non-conventional MRIs will include magnetization transfer and diffusion tensor images.

**Nonconventional MRI Analyses.** Nonconventional brain MRI will be integrated into this MAD study to explore the potential effects of AE-12-1-Y-QL remyelination and axonal regeneration in patients with RFMS.

Patients treated in Group 5 receiving a 3600 mg loading dose of AE-12-1-Y-QL (split over two days) and three subsequent 1200 mg treatment doses (n=12) or placebo (n=12) on maintenance GA will undergo evaluation by quantitative MRI outcome measures sensitive to remyelination and axonal regeneration (in addition to conventional MRI). The non-conventional MRIs for all subjects in Part 2 will be performed at a single sponsor determined imaging center and 3T MRI scanner selected for the purposes of this study.

The primary outcome measures will be Magnetization Transfer Ratio (MTR), Fractional Anisotrophy (FA), and Radial Diffusivity (RD) in lesions defined on baseline T2/FLAIR MRI. Strong correlations between axonal density, myelin content and MTR have been observed in post mortem MS tissues and animal models, supporting the use of this measure for assessing axonal and myelin pathophysiology in MS patients.

Increase in MTR, increase in FA, and/or a reduction in RD within pre-existing lesions in the AE-12-1-Y-QL treated subjects relative to the placebo will be interpreted as evidence of AE-12-1-Y-QL associated improvement in axonal and myelin pathophysiology. 12 RFMS patients per group will allow detection of 5% increase in T2 lesion MTR in a baseline-adjusted analysis of placebo-controlled parallel group trial with 82% power at $\alpha=0.05$ (one-tail).

Primary MRI endpoints for Group 5 include:

• Magnetization Transfer Ratio (MTR) at Baseline and Day 113 in lesions defined on Baseline T2/FLAIR MRI;
• Fractional Anisotrophy (FA) at Baseline and Day 113 in lesions defined on Baseline T2/FLAIR MRI; and
• Radial Diffusivity (RD) at Baseline and Day 113 in lesions defined on Baseline T2/FLAIR MRI.

For each outcome measure, the difference in mean from placebo, adjusted for baseline for each AE-12-1-Y-QL regimen, will be presented based on the ANCOVA framework. Primary statistical inference will be performed on estimate of effect at significance level of 0.05.

Assumptions for power analysis was based on MTR in T2 lesions from RRMS patients [Altmann DR et al., 2013] for a one-sided comparison of baseline- to-follow-up MTR changes between trial arms (ANCOVA). It was assumed that the population standard deviation at both baseline and Day 113 is 1.91 and the correlation between the measurements at Day 113 and baseline is 0.70.

In Groups 1-5, a loading dose of two times the designated treatment dose will be administered for the first dose. Subsequent doses will be administered four weeks apart. For Groups 1-3, the loading dose will be administered on Day 1 (e.g., 100 mg for the 50 mg treatment dose; 300 mg for the 150 mg treatment dose; 1200 mg for the 600 mg treatment dose). For Group 4 and Group 5, which receive a treatment dose of 1800 mg, the loading dose will be administered in equal divided doses on Days 1 and 2.

All doses of AE12-1-Y-QL will be administered by intravenous (IV) infusion in the morning. Subjects will continue with their current dosing regimen of GA (i.e., Copaxone® 20 mg administered subcutaneously once a day or Copaxone® 40 mg administered subcutaneously three times weekly or generic GA 20 mg administered subcutaneously once a day). Subjects will need to have been receiving Copaxone® for at least 2 weeks prior to being randomized and must remain on the same regimen throughout the study. A total of four doses will be administered, including the loading dose (a loading dose split across two days counts as one dose). Higher doses will be administered only after the available safety, tolerability and pharmacokinetic data for the lower dose(s) have been reviewed and evaluated by the sponsor in consultation with the investigator.

The doses to be administered are shown in Table 10.

**Table 10**

| Part | Group | N | Loading Dose[a] | Treatment Dose[b] |
|------|-------|---|-----------------|-------------------|
| 1 | 1 | 6 | 100 mg AE12-1-Y-QL | 50 mg AE12-1-Y-QL |
|   |   | 2 | Placebo | Placebo |
|   | 2* | 6 | 300 mg AE12-1-Y-QL | 150 mg AE12-1-Y-QL |
|   |   | 2 | Placebo | Placebo |
|   | 3* | 6 | 1200 mg AE12-1-Y-QL | 600 mg AE12-1-Y-QL |
|   |   | 2 | Placebo | Placebo |
|   | 4* | 6 | 3600 mg AE12-1-Y-QL | 1800 mg AE12-1-Y-QL |
|   |   | 2 | Placebo | Placebo |
| 2 | 5c* | 12 | 3600 mg AE12-1-Y-QL | 1800 mg AE12-1-Y-QL |
|   |   | 12 | Placebo | Placebo |

[a] Loading dose for Groups 1-3 will be administered on Day 1. For Groups 4 and 5, the loading dose will be administered as two divided doses on Days 1 and 2; [b] One dose IV every 4 weeks for a total of three additional doses; [c.] A sufficient number of subjects will be enrolled in Part 2 such that approximately 12 subjects in each treatment arm of Part 2 complete the Day 113 visit with evaluable non-conventional MRI data; * The doses may be adjusted upon review of the available safety, tolerability and pharmacokinetic data from the previous dose group(s).

Serial serum samples for determination of concentrations of AE-12-1-Y-QL and biomarkers will be collected in all groups until 113 after initiation of dosing. Serial serum samples for determination of anti-drug antibodies will be collected in all groups until 176 days after initiation of dosing.

Subjects in Part 1 will have two lumbar punctures performed to collect CSF for the following: routine laboratory tests consisting of cell count and differential, glucose, total protein, albumin, immunoglobulin; determination of AE-12-1-Y-QL concentration; total, free and bound sRGMa levels; AE-12-1-Y-QL availability for interaction with membrane bound BMP receptor using a reporter gene assay; and for biomarker analyses. The first lumbar puncture will be performed prior to the first dose (baseline), but after the baseline brain MRI; and the second lumbar puncture will be performed 28 days after the fourth dose.

After successful completion of the Screening visit, eligible subjects will undergo baseline brain magnetic resonance imaging (MRI). For subjects in Part 1, the Screening MRI may take place at any time following the Screening Visit and prior to the start of confinement (Day - 10). For subjects in Part 2, the Screening MRI may take place at any time following the Screening visit and prior to Day -1. Subjects whose brain MRI show evidence of overt vascular lesions, masses, mass effect or other abnormalities other than those compatible with MS will be excluded. All subjects will undergo MRI at baseline, Days 57, 113 and 176.

Safety and tolerability will be assessed throughout the study. This will include adverse event collection, laboratory tests, neurological examination, measurements of vital signs and electrocardiograms (ECGs) and MRI scans. If needed, unscheduled relapse assessment visits will occur within 72 hours of the onset of any new neurological symptoms that may indicate the onset of a clinical relapse. These visits will consist of neurological examination, with accompanying assessments for the Functional Status Scale (FSS) and Expanded Disability Status Scale (EDSS), vital signs, blood chemistry and hematology and urinalysis. Subjects who experience a suspected MS relapse may be treated with IV methylprednisolone 1000 mg/day for 1 to 5 consecutive days.

Multiple sclerosis disease activity will be monitored during scheduled serial clinic visits and at unscheduled visits as needed. Clinical events that will be captured and recorded include relapses and disability progression measured on the expanded disability status scale (EDSS) and the Multiple Sclerosis Functional Composite (MSFC) and on the individual domains of the MSFC, the Timed 25 Foot Walk (T25FW), the 9 Hole Peg Test (9HPT) and the Paced Auditory Serial Arithmetic Test (PASAT). In addition, the patient recorded outcome measures will be obtained using the instruments Multiple Sclerosis Impact Scale (MSIS-29) and Multiple Sclerosis Quality of Life-54 (MSQOL-54).

Disability progression can only be confirmed from the EDSS scores obtained according to the protocol-defined schedule of assessments at regular visits. The treating nurse must inform the treating neurologist if a subject experiences at least a 1.0-point increase on the EDSS from a baseline EDSS $\geq$1.0 that is sustained for 12 weeks. The subject must be informed they have experienced a worsening of physical disability.

Diagnosis and Main Criteria for Inclusion/Exclusion:

**Main Inclusion Criteria:** To be eligible for this study, candidates must meet the following eligibility criteria prior to randomization or at the time point specified in the individual criteria listed below:

1. Male or female and age is between 18 and 60 years, inclusive.

2. Subject is currently receiving Copaxone® 20 mg administered subcutaneously once a day or Copaxone® 40 mg administered subcutaneously three times weekly or generic glatiramer acetate 20 mg administered subcutaneously once a day for the treatment of MS and has received Copaxone® or GA maintenance treatment for at least 3 months. For subjects transitioning from one formulation of Copaxone® or GA to another, subjects must have received the newer formulation for at least 2 weeks prior to randomization.

3. If female, subject must be:

- of non-childbearing potential [surgically sterile (oophorectomy, complete hysterectomy, bilateral tubal ligation), postmenopausal for at least 2 years (hormone replacement therapy is acceptable)] or
- if of childbearing potential, must practice total abstinence from sexual intercourse as the preferred life style of the subject; periodic abstinence is not acceptable; have a male monogamous sexual partner who is vasectomized at least 6 months prior to study or agree to utilize effective contraception (copper or hormonal intrauterine device (IUD), oral hormonal contraception, or double barrier protection methods) during the entire treatment and follow up period.

4. Females of childbearing potential must have negative results for pregnancy tests prior to study drug administration and throughout entire study participation.

5. If male, subject must

- have documentation of having undergone male contraceptive surgery e.g., vasectomy, or
- agree to be sexually inactive or agree to us a barrier method of birth control until 90 days after the last dose of study drug, or total abstinence from sexual intercourse as the preferred life style of the subject; periodic abstinence is not acceptable.

6. Diagnosis of relapsing-remitting MS (RRMS) or secondary progressive MS, (SPMS) known commonly as relapsing forms of MS (RFMS).

7. Subjects must have a confirmed diagnosis of RFMS according to the revised McDonald criteria and have a cranial MRI demonstrating lesion(s) consistent with MS. In addition to having RFMS, subjects in Part 1 must have evidence of ongoing disease activity as evidenced by:

- Having experienced at least 1 relapse within the 12 months prior to randomization. For inclusion purposes, a relapse is defined as new or recurrent neurological symptoms documented in the medical record, not associated with fever or infection, lasting at least 24 hours, to be determined by the investigator. Time since relapse should be measured from the time of relapse onset, OR,
- Showing evidence of Gadolinium (Gd)-enhancing (Gd+) lesions of the brain on an MRI performed within the 6 months prior to randomization (if a prior MRI scan documenting presence of T1 Gd+ lesion activity is not available over the prior 6 months to screening from the subject's history, then the baseline brain MRI scan may be used).

8. Neurologically stable at the Screening Visit, in the investigator's judgment and not actively experiencing or recovering from a recent relapse in the 30 days preceding the Screening Visit.

9. Must have a baseline EDSS between 1.0 and 6.0, inclusive.

10. Body Mass Index (BMI) is 18.0 to 32.0, inclusive. BMI is calculated as weight in kg divided by the square of height measured in meters.

11. Has a brain MRI scan at screening, interpreted by a radiologist, that did not show evidence of overt vascular lesions, masses, mass effect or other abnormalities other than those compatible with MS, which would preclude the subject from undergoing a lumbar puncture/spinal tap for CSF collection.

12. A condition of general good health, except for MS, based upon the results of a medical history, physical examination, vital signs, laboratory profile, neurological examination and a 12-lead electrocardiogram (ECG)

Must voluntarily sign and date each informed consent, approved by an Independent Ethics Committee (IEC)/Institutional Review Board (IRB), prior to the initiation of any screening or study-specific procedures.

**Exclusion Criteria:** A subject will not be eligible for study participation if he/she meets any of the following criteria:

Medical History:

1. Diagnosis of primary progressive MS.

2. History or abnormal laboratory results that, in the opinion of the investigator, are indicative of any significant cardiac, endocrinologic, hematologic, hepatic, immunologic, metabolic, urologic, pulmonary, gastrointestinal, dermatologic, psychiatric, renal, neurologic (other than MS), and/or other major disease that would preclude administration of AE-12-1-Y-QL or GA.

3. An MS relapse that has occurred within the 30 days prior to randomization AND/OR the subject has not stabilized from a previous relapse prior to randomization

4. Subjects for whom MRI is contraindicated, (i.e., aneurysm clip, metal fragments, internal electrical devices such as a cochlear implant, spinal cord stimulator or pacemaker), contraindicated for or allergic to gadolinium (including renal impairment, abnormal estimated glomerular filtration rate (eGFR), previous diagnosis of nephrogenic systemic fibrosis and allergy), have claustrophobia that cannot be medically managed or are unable to lie still for 1 hour or more for the imaging procedures.

5. Receipt of any depot drug by injection (exclusive of contraceptives) within 30 days prior to study drug administration.

6. Receipt of an investigational product within a time period equal to 10 half-lives, if known, or within 6 weeks months prior to study drug administration.

7. Positive screen for drugs of abuse or alcohol as detected at Screening or Day -2.

8. History of malignancy; however, subjects with a history of excised or treated basal cell carcinoma or fewer than 3 squamous cell carcinomas are eligible to participate in this study.

9. Known hypersensitivity to study drugs or their excipients.

10. Subject has a history of recreational drug use, drug abuse, misuse, or engagement in non-medical use of either prescribed or over-the-counter medication within 2 years prior to study drug administration, or plans to use recreational drugs over the course of participating in the study through the last follow-up visit.

11. History of severe allergic or anaphylactic reactions.

12. History of seizure disorder or unexplained blackouts OR history of a seizure within 6 months.

13. History of suicidal ideation or an episode of clinically severe depression within 1 month prior to study drug administration as evidenced by answering "yes" to questions 4 or 5 on the suicidal ideation portion of the Columbia-Suicide Severity Rating Scale (C-SSRS) completed at Screening, or any history of suicide attempts.

14. Known history of, or positive screening test result for hepatitis C or hepatitis B virus.

15. Varicella or herpes zoster virus infection or any severe viral infection within 6 weeks before screening.

16. Exposure to individuals with active varicella zoster virus infections within 21 days before screening.

17. History of human immunodeficiency virus (HIV) or other immunodeficient conditions.

18. Any type of live virus vaccine less than 4 weeks before randomization, including but not limited to: measles/mumps/rubella vaccine, varicella zoster virus vaccine, oral polio vaccine, and nasal influenza vaccine.

19. Infection (viral, fungal, bacterial) requiring hospitalization or intravenous (IV) antibiotics within 8 weeks before randomization.

20. Elective surgery performed from 2 weeks prior to randomization or scheduled through the end of the study.

21. Findings on brain MRI scan indicating any clinically significant brain abnormality other than MS.

22. Any of the following abnormal blood tests at screening:

- Hemoglobin $\leq$ 10.0 g/dL
- Platelets $\leq$ 100 $\times$ $10^9$/L
- Lymphocytes $\leq$ 1.0 $\times$ $10^9$/L
- Neutrophils $\leq$ 1.5 $\times$ $10^9$/L
- Alanine aminotransferase/serum glutamate pyruvate transaminase (ALT/SGPT), aspartate aminotransferase/serum glutamic oxaloacetic transaminase (AST/SGOT), or gamma glutamyl-transferase $\geq$ 2 times the upper limit of normal (ULN)
- Serum iron, ferritin, transferrin saturation > ULN.
- Serum creatinine $\geq$ ULN.

23. For subjects being screened for potential randomization into Part 1 only, contraindication for lumbar puncture (e.g., lumbar scoliosis, coagulopathy, or hypocoagulation medications, infected skin at needle puncture site)

24. Donation or loss of 550 mL or more blood volume (including plasmapheresis) or receipt of a transfusion of any blood product within 8 weeks prior to study drug administration.

25. Prior treatment with the any of the following:

- Total lymphoid irradiation
- Cladribine or mitoxantrone
- T cell or T cell receptor vaccination

26. Prior treatment with cyclophosphamide or alemtuzumab, within 1 year prior to randomization.

27. Prior treatment with any of the following medications or procedures within the 6 months prior to randomization:

- Natalizumab
- Rituximab
- Daclizumab
- Cyclosporine
- Azathioprine
- Methotrexate
- Mycophenolate mofetil
- Intravenous immunoglobulin (IVIg)
- Plasmapheresis or cytapheresis

28. Treatment with any of the following medications within the 30 days prior to randomization:

- IV corticosteroid treatment
- Oral corticosteroid treatment
- Beta-interferon
- Fingolimod
- Dimethyl fumarate
- Teriflunomide

29. Initiation of treatment or dose adjustment of commercially available Fampridine sustained release (SR) within the last 90 days. It is acceptable if a patient already is receiving a stable dose of Fampridine-SR prior to randomization and plans to remain on this dose and regimen throughout study.

30. Female who is or is considering becoming pregnant while participating in the study, or is breastfeeding.

31. Current enrollment in another clinical study or previous enrollment in this study.

32. Consideration by the investigator, for any reason, that the subject is an unsuitable candidate to receive AE-12-1-Y-QL.

**Pharmacokinetics:** The following values for the pharmacokinetic parameters will be estimated using non-compartmental methods: maximum observed serum concentration ($C_{max}$), the time to $C_{max}$ (peak time, $T_{max}$) and the area under the concentration time curve (AUC) will be estimated after the first and fourth doses. The observed serum concentration prior to dose ($C_{trough}$) will be measured on Days 29, 57, 85 and 113. Terminal phase elimination rate

constant ($\beta$), terminal phase elimination half-life ($t_{1/2}$) and apparent clearance (CL/F) will be estimated after the fourth dose. Anti-drug antibody (ADA) titers will be determined for assessment of immunogenicity. Additional parameters may be calculated if useful in the interpretation of the data.

**CSF Biomarkers:** Analysis for CSF biomarkers will be done in Part 1 only. A panel of biomarkers representing markers for pro- and anti-inflammation, neuroregeneration/neuroprotection, neurodegeneration, and/or remyelination will be assessed.

**Pharmacodynamics:** Exploratory Brain MRI Outcomes (Part 2)

The study will include MRI outcome measures sensitive to changes in axonal and myelin pathophysiology in the brain and disease activity as exploratory endpoints.

Conventional MRI assessments will be performed on all subjects at Baseline and at Days 57, 113 and 176. Non-conventional MRI assessments will be performed in Part 2 at an imaging center with an MRI scanner selected for the purposes of this study at Baseline and at Day 113.

The following outcome measures will be evaluated:

Primary MRI endpoints:

- Magnetization Transfer Ratio (MTR) at Baseline and Day 113 in lesions defined on Baseline T2/FLAIR MRI (Part 2 only)

- Fractional Anisotropy (FA) at Baseline and Day 113 in lesions defined on Baseline T2/FLAIR MRI (Part 2 only)

- Radial Diffusivity (RD) at Baseline and Day 113 in lesions defined on Baseline T2/FLAIR MRI (Part 2 only)

- Number of new Gd$^+$ T1 lesions at day 113 (all subjects)

Secondary MRI Endpoints:

- MTR and FA in Normal-Appearing Gray Matter (NAGM) defined on baseline MRI at Baseline and Day 113 (Part 2 only)

- MTR and FA in Normal-Appearing White Matter (NAWM) defined on baseline MRI at Baseline and Day 113 (Part 2 only)

- Spinal cord gray matter area and total cord area at Baseline and Day 113 (Part 2 only)

- Number of new Gd$^+$ T1 lesions across Day 57 and Day 113 (all subjects)

- Number of new, newly-enlarging T2 hyperintense lesions at Day 113 (all subjects)

- Lesion volume of new, newly enlarging T2 hyperintense lesions at Day 113 (all subjects)

**Safety:** The safety variables will include the following: adverse event monitoring, vital signs, physical examination, neurological examination, electrocardiograms, laboratory tests assessments, and C-SSRS and MRI scans. If needed, unscheduled relapse assessment visits will occur, when possible, within 7 days of the onset of any new neurological symptoms that may indicate the onset of a clinical relapse. These visits will consist of neurological examination, with accompanying assessments for the Functional Status Scale (FSS) and Expanded Disability Status Scale (EDSS), vital signs, blood chemistry and hematology and urinalysis. Subjects who experience a suspected MS relapse may be treated with IV methylprednisolone 1000 mg/day for 1 to 5 consecutive days.

Adverse events will be coded by Medical Dictionary for Regulatory Activities (MedDRA). Adverse event data will be summarized separately for Part 1 and Part 2 of the study. The number and percentage of subjects reporting treatment-emergent adverse events will be tabulated by MedDRA Preferred Term and System Organ Class with a breakdown by dose level. Tabulations will also be provided in which the number of subjects reporting an adverse event (MedDRA term) is additionally broken down by rating (mild, moderate or severe) and by whether possibly related to study drug. Any deaths, other serious adverse events and other significant adverse events will be separately identified. Laboratory test values and measurements on vital signs that are potentially clinically significant, according to predefined criteria, will be identified.

**Example 6**

**Dosing Study of AE12-1-Y-QL in Subjects with Relapsing Forms of Multiple Sclerosis**

The primary objective of this study is to assess the safety, tolerability, pharmacokinetics and immunogenicity of AE12-1-Y-QL in subjects with Relapsing Forms of Multiple Sclerosis (RFMS) who are on maintenance glatiramer acetate (GA) treatment. The secondary objective is to assess the effect of AE12-1-Y-QL on the clinical and neuroimaging parameters of Multiple Sclerosis (MS) disease activity and on changes in levels of cerebrospinal fluid (CSF) and blood biomarkers.

**Study Population:** Adult male and female subj ects with RFMS who are on maintenance GA treatment.

**Methodology:** This is a multi-center Phase 1, double-blind, placebo-controlled, randomized, escalating multiple-dose study. Up to approximately 21-28 subjects will participate in this study.

The study will consist of three groups (Groups 1 - 3). Each group will be comprised of 7 subjects. In addition to a maintenance GA regimen, in each group of 7 subjects, 5 subjects will be randomly assigned to treatment with AE12-1-Y-QL and 2 subjects randomly assigned to treatment with matching placebo. An optional Group 4 (AE12-1-Y-QL 50 mg monthly or placebo) may be added after a review of the data from Groups 1 - 3.

For Groups 1 and 2, a loading dose of two times the designated treatment dose will be administered for the first dose; subsequent treatment doses will be administered 4 weeks apart. For Groups 1 and 2, the loading dose will be administered on Day 1 (e.g., 300 mg for the 150 mg treatment dose in Group 1). For Group 3, the loading dose will be administered in two equal amounts (totaling 3600 mg) on Days 1 and 2. The study drug regimens will consist of a total of four doses, 4 weeks apart with the loading dose counted as the first dose. All doses of AE12-1-Y-QL or matching placebo will be administered by IV infusion at a constant rate over a 2-hour interval.

A review of the safety, pharmacokinetic, and biomarker data is planned after all subjects in Groups 1 - 3 have been dosed. An optional Group 4 consisting of AE12-1-Y-QL 50 mg or placebo (loading dose of 100 mg) may be enrolled and dosed.

Subjects will have conventional MRIs performed at baseline and Days 57, 113 and 176 of the study to evaluate disease activity and safety. The MRIs will include all or a subset of T1 weighted (with and without Gadolinium contrast), T2 weighted, T2 weighted fluid attenuated inversion recovery (FLAIR), proton density (PD) weighted, T2*/susceptibility weighted and diffusion weighted images.

A loading dose of two times the designated treatment dose will be administered for the first dose. Subsequent doses will be administered four weeks apart. For Groups 1 and 2, the loading dose will be administered on Day 1 (e.g., 300 mg for the 150 mg treatment dose; 1200 mg for the 600 mg treatment dose). For Group 3, which receives a treatment dose of 1800 mg, the loading dose of 3600 mg will be administered in equal divided doses on Days 1 and 2.

Dosing will begin with Group 1, followed by Groups 2 and 3. Subjects in Groups 1 and 2 will enroll uninterruptedly without a pause between dose groups. Dosing in Group 3 will begin upon a review of available data after all subjects from Group 2 have received at least two doses. An optional dose group of 50 mg monthly with a 100 mg loading dose (Group 4) may be enrolled after a review of available data from Groups 1 - 3.

All doses of AE12-1-Y-QL will be administered by intravenous (IV) infusion in the morning. Subjects will continue with their current dosing regimen of GA (i.e., Copaxone® 20 mg administered subcutaneously once a day or Copaxone® 40 mg administered subcutaneously three times weekly or generic GA 20 mg administered subcutaneously once a day). For subjects transitioning from one formulation of Copaxone® or GA to another, subjects must have received the newer formulation for at least 2 weeks prior to randomization and must remain on the same dose regimen throughout the duration of the study.

The doses to be administered are shown in Table 11.

**Table 11**

| Group | N | Loading Dose[a] | Treatment Dose[b] |
|---|---|---|---|
| 1 | 5 | 300 mg AE12-1-Y-QL | 150 mg AE12-1-Y-QL |
| | 2 | Placebo | Placebo |
| 2* | 5 | 1200 mg AE12-1-Y-QL | 600 mg AE12-1-Y-QL |
| | 2 | Placebo | Placebo |
| 3* | 5 | 3600 mg AE12-1-Y-QL | 1800 mg AE12-1-Y-QL |
| | 2 | Placebo | Placebo |
| 4[c] | 5 | 100 mg AE12-1-Y-QL | 50 mg AE12-1-Y-QL |

(continued)

| Group | N | Loading Dose[a] | Treatment Dose[b] |
|---|---|---|---|
| (optional) | 2 | Placebo | Placebo |

a. Loading dose for Groups 1-2 will be administered on Day 1. For Group 3, the loading dose will be administered as two divided doses on Days 1 and 2; b. One dose IV every 4 weeks for a total of four doses; c. Optional dose group (determined after review of data from Groups 1 - 3); * The doses may be adjusted upon review of the available safety, tolerability and pharmacokinetic data from the previous dose group(s).

Serial blood samples for determination of concentrations of AE12-1-Y-QL, anti-drug antibodies and biomarkers will be collected in all groups until 176 days after initiation of dosing.

Subjects will have three lumbar punctures performed to collect CSF for the following: routine laboratory tests consisting of cell count and differential, glucose, IgG Index, oligoclonal bands and myelin basic protein; determination of AE-12-1-Y-QL concentration; total, free and bound soluble RGMa (sRGMa) levels; AE-12-1-Y-QL availability for interaction with membrane bound BMP receptor using a reporter gene assay; and for biomarker analyses.

The first lumbar puncture will be performed prior to the first dose (baseline), but after the baseline brain MRI; and the second lumbar puncture will be performed approximately 28 days after the first dose and the last lumbar puncture will be performed approximately 28 days after the fourth dose.

After successful completion of the Screening visit, eligible subjects will undergo baseline brain magnetic resonance imaging (MRI). The Screening MRI may take place at any time following the Screening Visit and prior to the start of confinement (Day -10). Subjects whose brain MRI show evidence of overt vascular lesions, masses, mass effect or other abnormalities other than those compatible with MS will be excluded. All subjects will undergo MRI at screening (baseline MRI), Days 57, 113 and 176.

Safety and tolerability will be assessed throughout the study. This will include adverse event collection, laboratory tests, neurological examination, measurements of vital signs and electrocardiograms (ECGs) and MRI scans. If needed, unscheduled relapse assessment visits will occur within 7 days of the onset of any new neurological symptoms that may indicate the onset of a clinical relapse. These visits will consist of neurological examination, with accompanying assessments for the Functional Status Scale (FSS) and Expanded Disability Status Scale (EDSS), vital signs, blood chemistry and hematology and urinalysis. Subjects who experience a suspected MS relapse may be treated with IV methylprednisolone 1000 mg/day for 1 to 5 consecutive days.

Multiple sclerosis disease activity will be monitored during scheduled serial clinic visits and at unscheduled visits as needed. Clinical events that will be captured and recorded include relapses and disability progression measured on the expanded disability status scale (EDSS). In addition, the patient recorded outcome measures will be obtained using the instruments Multiple Sclerosis Impact Scale (MSIS-29) and Multiple Sclerosis Quality of Life-54 (MSQOL-54).

Disability progression can only be confirmed from the EDSS scores obtained according to the protocol-defined schedule of assessments at regular visits. The study coordinator must inform the treating neurologist if a subject experiences at least a 1.0-point increase on the EDSS from a baseline EDSS $\geq$ 1.0 that is sustained for 12 weeks. The subject must be informed they have experienced a worsening of physical disability.

Diagnosis and Main Criteria for Inclusion/Exclusion:

**Main Inclusion Criteria:** To be eligible for this study, candidates must meet the following eligibility criteria prior to randomization or at the time point specified in the individual criteria listed below:

1. Male or female and age is between 18 and 60 years, inclusive.
2. Subject is currently receiving Copaxone® 20 mg administered subcutaneously once a day or Copaxone® 40 mg administered subcutaneously three times weekly or generic glatiramer acetate 20 mg administered subcutaneously once a day for the treatment of MS and has received Copaxone® or GA maintenance treatment for at least 3 months. For subjects transitioning from one formulation of Copaxone® or GA to another, subjects must have received the newer formulation for at least 2 weeks prior to randomization.
3. If female, subject must be:

- of non-childbearing potential [surgically sterile (oophorectomy, complete hysterectomy, bilateral tubal ligation), postmenopausal for at least 2 years (hormone replacement therapy is acceptable)] or
- if of childbearing potential, must practice total abstinence from sexual intercourse as the preferred life style of the subject; periodic abstinence is not acceptable; have a male monogamous sexual partner who is vasectomized at least 6 months prior to study or agree to utilize effective contraception (copper or hormonal intrauterine device (IUD), oral hormonal contraception, or double barrier protection methods) during the entire treatment and follow up period.

4. Females of childbearing potential must have negative results for pregnancy tests prior to study drug administration and throughout entire study participation.
5. If male, subject must

- have documentation of having undergone male contraceptive surgery e.g., vasectomy, or
- agree to be sexually inactive or agree to us a barrier method of birth control until 90 days after the last dose of study drug, or total abstinence from sexual intercourse as the preferred life style of the subject; periodic abstinence is not acceptable.

6. Diagnosis of relapsing-remitting MS (RRMS) or secondary progressive MS, (SPMS) known commonly as relapsing forms of MS (RFMS).
7. Subjects must have a confirmed diagnosis of RFMS according to the revised McDonald criteria and have a cranial MRI demonstrating lesion(s) consistent with MS.
8. Neurologically stable at the Screening Visit, in the investigator's judgment and not actively experiencing or recovering from a recent relapse in the 30 days preceding the Screening Visit.
9. Must have a baseline EDSS between 1.0 and 6.0, inclusive.
10. Body Mass Index (BMI) is 18.0 to 32.0, inclusive. BMI is calculated as weight in kg divided by the square of height measured in meters.
11. Has a brain MRI scan at screening, interpreted by a radiologist, that did not show evidence of overt vascular lesions, masses, mass effect or other abnormalities other than those compatible with MS, which would preclude the subject from undergoing a lumbar puncture/spinal tap for CSF collection.
12. A condition of general good health, except for MS, based upon the results of a medical history, physical examination, vital signs, laboratory profile, neurological examination and a 12-lead electrocardiogram (ECG)
13. Must voluntarily sign and date each informed consent, approved by an Independent Ethics Committee (IEC)/Institutional Review Board (IRB), prior to the initiation of any screening or study-specific procedures.

**Exclusion Criteria:** A subject will not be eligible for study participation if he/she meets any of the following criteria:

Medical History:

1. Diagnosis of primary progressive MS.
2. History or abnormal laboratory results that, in the opinion of the investigator, are indicative of any significant cardiac, endocrinologic, hematologic, hepatic, immunologic, metabolic, urologic, pulmonary, gastrointestinal, dermatologic, psychiatric, renal, neurologic (other than MS), and/or other major disease that would preclude administration of AE-12-1-Y-QL or GA.
3. An MS relapse that has occurred within the 30 days prior to randomization AND/OR the subject has not stabilized from a previous relapse prior to randomization
4. Subjects for whom MRI is contraindicated, (i.e., aneurysm clip, metal fragments, internal electrical devices such as a cochlear implant, spinal cord stimulator or pacemaker), contraindicated for or allergic to gadolinium (including renal impairment, abnormal estimated glomerular filtration rate (eGFR), previous diagnosis of nephrogenic systemic fibrosis and allergy), have claustrophobia that cannot be medically managed or are unable to lie still for 1 hour or more for the imaging procedures.
5. Receipt of any depot drug by injection (exclusive of contraceptives) within 30 days prior to study drug administration.
6. Receipt of an investigational product within a time period equal to 10 half-lives, if known, or within 6 weeks months prior to study drug administration.
7. Positive screen for drugs of abuse or alcohol as detected at Screening or Day -2.
8. History of malignancy; however, subjects with a history of excised or treated basal cell carcinoma or fewer than 3 squamous cell carcinomas are eligible to participate in this study.
9. Known hypersensitivity to study drugs or their excipients.
10. Subject has a history of recreational drug use, drug abuse, misuse, or engagement in non-medical use of either prescribed or over-the-counter medication within 2 years prior to study drug administration, or plans to use recreational drugs over the course of participating in the study through the last follow-up visit.

11. History of severe allergic or anaphylactic reactions.

12. History of seizure disorder or unexplained blackouts OR history of a seizure within 6 months.

13. History of suicidal ideation or an episode of clinically severe depression within 1 month prior to study drug administration as evidenced by answering "yes" to questions 4 or 5 on the suicidal ideation portion of the Columbia-Suicide Severity Rating Scale (C-SSRS) completed at Screening, or any history of suicide attempts.

14. Known history of, or positive screening test result for hepatitis C or hepatitis B virus.

15. Varicella or herpes zoster virus infection or any severe viral infection within 6 weeks before screening.

16. Exposure to individuals with active varicella zoster virus infectionswithin 21 days before screening.

17. History of human immunodeficiency virus (HIV) or other immunodeficient conditions.

18. Any type of live virus vaccine less than 4 weeks before randomization, including but not limited to: measles/mumps/rubella vaccine, varicella zoster virus vaccine, oral polio vaccine, and nasal influenza vaccine.

19. Infection (viral, fungal, bacterial) requiring hospitalization or intravenous (IV) antibiotics within 8 weeks before randomization.

20. Elective surgery performed from 2 weeks prior to randomization or scheduled through the end of the study.

21. Findings on brain MRI scan indicating any clinically significant brain abnormality other than MS.

22. Any of the following abnormal blood tests at screening:

- Hemoglobin $\leq$ 10.0 g/dL
- Platelets $\leq$ 100 $\times$ $10^9$/L
- Lymphocytes $\leq$ 1.0 $\times$ $10^9$/L
- Neutrophils $\leq$ 1.5 $\times$ $10^9$/L
- Alanine aminotransferase/serum glutamate pyruvate transaminase (ALT/SGPT), aspartate aminotransferase/serum glutamic oxaloacetic transaminase (AST/SGOT), or gamma glutamyl-transferase $\geq$ 2 times the upper limit of normal (ULN)
- Serum iron, ferritin, transferrin saturation > ULN.
- Serum creatinine $\geq$ ULN.

23. Contraindication for lumbar puncture (e.g., lumbar scoliosis, coagulopathy, or hypocoagulation medications, infected skin at needle puncture site).

24. Donation or loss of 550 mL or more blood volume (including plasmapheresis) or receipt of a transfusion of any blood product within 8 weeks prior to study drug administration.

25. Prior treatment with the any of the following:

- Total lymphoid irradiation
- Cladribine or mitoxantrone
- T cell or T cell receptor vaccination

26. Prior treatment with cyclophosphamide or alemtuzumab, within 1 year prior to randomization.

27. Prior treatment with any of the following medications or procedures within the 6 months prior to randomization:

- Natalizumab
- Rituximab
- Daclizumab
- Cyclosporine
- Azathioprine
- Methotrexate
- Mycophenolate mofetil
- Intravenous immunoglobulin (IVIg)
- Plasmapheresis or cytapheresis

28. Treatment with any of the following medications within the 30 days prior to randomization:

- IV corticosteroid treatment
- Oral corticosteroid treatment
- Beta-interferon
- Fingolimod
- Dimethyl fumarate

- Teriflunomide

29. Initiation of treatment or dose adjustment of commercially available Fampridine sustained release (SR) within the last 90 days. It is acceptable if a patient already is receiving a stable dose of Fampridine-SR prior to randomization and plans to remain on this dose and regimen throughout study.

30. Female who is or is considering becoming pregnant while participating in the study, or is breastfeeding.

31. Current enrollment in another clinical study or previous enrollment in this study.

32. Consideration by the investigator, for any reason, that the subject is an unsuitable candidate to receive AE-12-1-Y-QL.

**Pharmacokinetics:** The following values for the pharmacokinetic parameters will be estimated using non-compartmental methods: maximum observed serum concentration ($C_{max}$), the time to $C_{max}$ (peak time, $T_{max}$) and the area under the concentration time curve (AUC) will be estimated for the first and final dose intervals. The observed serum concentration at the end of a dose interval ($C_{trough}$) will be measured on Days 29, 57, 85 and 113. Terminal phase elimination rate constant ($\beta$), terminal phase elimination half-life ($t_{1/2}$) and apparent clearance (CL/F) will be estimated after the final dose. Anti-drug antibody (ADA) titers will be determined for assessment of immunogenicity. Additional parameters may be calculated if useful in the interpretation of the data.

Pharmacodynamics:

Target Engagement:

The CSF concentration of Soluble Repulsive Guidance Molecule A (sRGMA) bound to AE12-1-Y-QL will be determined as a measure of target engagement.

Brain MRI Outcomes

- Number of new Gd+ T1 lesions at Day 113
- Number of new Gd+ T1 lesions across Day 57 and Day 113
- Number of new, newly-enlarging T2 hyperintense lesions at Day 113
- Lesion volume of new, newly enlarging T2 hyperintense lesions at Day 113

CSF and Blood Exploratory Biomarkers:

- Pharmacodynamic activity of CSF AE12-1-Y-QL may be assessed using ex vivo reporter gene assay that measures the degree of inhibition of membrane-bound RGMa. In addition, AE12-1-Y-QL binding to membrane bound RGMa and CD profiling may be assessed in blood and CSF monocytic populations. Profiled CD ligands include, but are not limited to, CD11b, CD163, CD206, CD68, CD80, CD86, CD40 and CD279. Finally, change from baseline and summary statistics may be performed for exploratory CSF and blood biomarkers reflective of immunomodulation, inflammation and remyelination. Current biomarkers may include, but are not limited to IL-6, IL-10, IL-17, neurofilament light and heavy chain (NfL, NfH), glial fibrillary acidic protein (GFAP), osteopontin, MCP-1, sCD27, MMP9, B lymphocyte chemoattractant (CXCL13), interferon gamma, TNFalpha and IL12(p70).

**Safety:** The safety variables will include the following: adverse event monitoring, vital signs, physical examination, neurological examination, electrocardiograms, laboratory tests assessments, and C-SSRS and MRI scans. If needed, unscheduled relapse assessment visits will occur, when possible, within 7 days of the onset of any new neurological symptoms that may indicate the onset of a clinical relapse. These visits will consist of neurological examination, with accompanying assessments for the Functional Status Scale (FSS) and Expanded Disability Status Scale (EDSS), vital signs, blood chemistry and hematology and urinalysis. Subjects who experience a suspected MS relapse may be treated with IV methylprednisolone 1000 mg/day for 1 to 5 consecutive days.

Adverse events will be coded by Medical Dictionary for Regulatory Activities (MedDRA). The number and percentage of subjects reporting treatment-emergent adverse events will be tabulated by MedDRA Preferred Term and System Organ Class with a breakdown by dose level. Tabulations will also be provided in which the number of subjects reporting an adverse event (MedDRA term) is additionally broken down by rating (mild, moderate or severe) and by whether possibly related to study drug. Any deaths, other serious adverse events and other significant adverse events will be separately identified. Laboratory test values and measurements on vital signs that are potentially clinically significant, according to predefined criteria, will be identified.

**Example 7**

Clause 1. A method of treating a relapsing form of multiple sclerosis in a subject in need thereof, the method comprising administering a therapeutically effective amount of an antibody or antigen-binding fragment thereof that specifically binds Repulsive Guidance Molecule A (RGMa), wherein the antibody or antigen binding fragment comprises

(a) a variable heavy chain comprising a complementarity determining region (CDR)-1 comprising an amino acid sequence of SEQ ID NO:2, a CDR-2 comprising an amino acid sequence of SEQ ID NO:3, and a CDR-3 comprising an amino acid sequence of SEQ ID NO:4; and

(b) a variable light chain comprising a CDR-1 comprising an amino acid sequence of SEQ ID NO:6, a CDR-2 comprising an amino acid sequence of SEQ ID NO:7, and a CDR-3 comprising an amino acid sequence of SEQ ID NO:8.

Clause 2. The method of clause 1, wherein the antibody or antigen-binding fragment thereof is administered to a subject in an amount of from about 50 mg to about 4000 mg, or in an amount of from about 50 mg to about 2500 mg.
Clause 3. The method of clauses 1 or 2, wherein the antibody or antigen-binding fragment thereof is administered in an amount of about 50 mg, 100 mg, 150 mg, 300 mg, 450 mg, 600 mg, 1000 mg, 1200 mg, 1600 mg, 1800 mg, 2400 mg, or 3600 mg.
Clause 4. The method of clauses 1 or 2, wherein the antibody or antigen-binding fragment thereof is administered in an amount of about 50 mg, 75 mg, 100 mg, 120 mg, 125 mg, 150 mg, 175 mg, 200 mg, 250 mg, 300 mg, 325 mg, 350 mg, 375 mg, 400 mg, 425 mg, 450 mg, 475 mg or 500 mg.
Clause 5. The method of clause 3, wherein the antibody or antigen-binding fragment thereof is administered intravenously (IV).
Clause 6. The method of clause 4, wherein the antibody or antigen-binding fragment thereof is administered subcutaneously.
Clause 7. The method of any one of clauses 1-6, wherein the variable heavy chain comprises an amino acid sequence of SEQ ID NO: 13 and the variable light chain comprises an amino acid sequence of SEQ ID NO: 14.
Clause 8. The method of any one of clauses 1-7, the antibody is selected from the group consisting of a human antibody, an immunoglobulin molecule, a disulfide linked Fv, a monoclonal antibody, an affinity matured antibody, a scFv, a chimeric antibody, a CDR-grafted antibody, a diabody, a humanized antibody, a multispecific antibody, a Fab, a dual specific antibody, a DVD, a Fab', a bispecific antibody, a F(ab')$_2$, and a Fv.
Clause 9. The method of clause 8, wherein the antibody is a human antibody.
Clause 10. The method of clause 8, wherein the antibody is a monoclonal antibody.
Clause 11. The method of clause 8, wherein the antibody is an affinity matured antibody.
Clause 12. The method of clause 8, wherein the antibody is a chimeric antibody.
Clause 13. The method of clause 8, wherein the antibody is a humanized antibody.
Clause 14. The method of clause 8, wherein the antibody is a Fab, a Fab', a F(ab')$_2$ or Fv.
Clause 15. The method of clause 8, wherein the antibody is a dual specific antibody, a DVD or a bispecific antibody.
Clause 16. The method of clause 7, further comprising a constant sequence of SEQ ID NO: 12.
Clause 17. The method of any one of clauses 1-16, further comprising administering an additional therapeutic agent.
Clause 18. The method of clause 17, where the additional therapeutic agent is an immunosuppressant or an agent that treats one or more symptoms associated with multiple sclerosis.
Clause 19. The method of clause 18, wherein the additional therapeutic agent comprises a beta interferon, Glatiramer (Copaxone), Fingolimod (Gilenya), Natalizumab (Tysabri), Mitoxantrone (Novantrone), or a cognitive enhancing drug.
Clause 20. The method of clause 19, wherein the cognitive enhancing drug comprises an acetylcholine receptor agonist, an acetylcholinesterase inhibitor, a butyrylcholinesterase inhibitor, an N-methyl-D-aspartate (NMDA) receptor antagonist, an activity-dependent neuroprotective protein (ADNP) agonist, a serotonin 5-HT1A receptor agonist, a 5-HT4 receptor agonist, a 5-HT6 receptor antagonist, a serotonin 1A receptor antagonist, a histamine H3 receptor antagonist, a calpain inhibitor, a vascular endothelial growth factor (VEGF) protein or agonist, a trophic growth factor, an anti-apoptotic compound, an AMPA-type glutamate receptor activator, a L-type or N-type calcium channel blocker or modulator, a potassium channel blocker, a hypoxia inducible factor (HIF) activator, a HIF prolyl 4-hydroxylase inhibitor, an anti-inflammatory agent, an inhibitor of amyloid Aβ peptide or amyloid plaque, an inhibitor of tau hyperphosphorylation, a phosphodiesterase 5 inhibitor, a phosphodiesterase 4 inhibitor, a monoamine oxidase inhibitor, pharmaceutically acceptable salts thereof, or a combination thereof.

Clause 21. The method of clause 20, wherein the cognitive enhancing drug comprises donepezil (Aricept®), rivastigmine (Exelon®), galanthamine (Reminyl®), memantine (Namenda®), or a combination thereof.

Clause 22. The method of any one of clauses 1-21, wherein the relapsing form of Multiple Sclerosis is relapsing remitting Multiple Sclerosis (RRMS) or relapsing-secondary progressive Multiple Sclerosis (SPMS).

Clause 23. The method of any one of clauses 1-22, wherein the antibody or antigen-binding fragment thereof is administered according to a multiple variable dose regimen.

Clause 24. The method of clause 23, wherein the multiple variable dose regimen comprises a loading dose and a treatment dose that is lower than the loading dose.

Clause 25. The method of clause 24, wherein the loading dose is selected from the group consisting of 100 mg, 300 mg, 1200 mg, and 3600 mg.

Clause 26. The method of clause 24, wherein the treatment dose is selected from the group consisting of 50 mg, 150 mg, 600 mg, and 1800 mg.

It is understood that the foregoing detailed description and accompanying examples are merely illustrative and are not to be taken as limitations upon the scope of the invention, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the spirit and scope thereof.

**Claims**

1. A method of assessing Multiple Sclerosis (MS) disease activity in a subject having relapsing form of multiple sclerosis comprising:
   performing a magnetic resonance imaging (MRI) assessment of the brain of the subject having relapsing form of multiple sclerosis previously administered with an anti-Repulsive Guidance Molecule a (RGMa) antibody.

2. The method of claim 1, wherein the antibody comprises:

   (a) a variable heavy chain region comprising a complementarity determining region (CDR)-1 comprising an amino acid sequence of SEQ ID NO:2, a CDR-2 comprising an amino acid sequence of SEQ ID NO:3, and a CDR-3 comprising an amino acid sequence of SEQ ID NO:4; and
   (b) a variable light chain region comprising a CDR-1 comprising an amino acid sequence of SEQ ID NO:6, a CDR-2 comprising an amino acid sequence of SEQ ID NO:7, and a CDR-3 comprising an amino acid sequence of SEQ ID NO:8.

3. The method of claim 1 or 2, wherein the MRI assessment comprises an assessment every two months.

4. The method of any one of claims 1 to 3, wherein the MRI assessment comprises determining a neuroimaging parameter which is Magnetization Transfer Ratio.

5. The method of any one of claims 1 to 3, wherein the MRI assessment comprises determining a neuroimaging parameter which is Radial Diffusivity.

6. The method of claim 4 or claim 5, wherein an increase in Magnetization Transfer Ratio or a reduction in Radial Diffusibility within pre-existing lesions in the anti-RGMa antibody treated subject relative to the placebo indicates anti-RGMa antibody associated improvement in remyelination and axonal regeneration in the subject having relapsing form of multiple sclerosis.

7. The method of any one of claims 4 to 6, wherein the magnetic resonance imaging (MRI) assessment comprises: (i) performing magnetization transfer and/or diffusion tensor images of the brain of the subject at baseline and day 112 post-administration of the anti-RGMa antibody and (ii) determination of Magnetization Transfer Ratio and/or Radial Diffusibility in lesions defined on baseline T2-weighed-Fluid Attenuated Inversion Recovery (T2/FLAIR) MRI.

8. The method of any one of claims 1 to 7, wherein the relapsing form of multiple sclerosis is selected from the group consisting of: relapsing-remitting multiple sclerosis (RRMS) and relapsing-secondary progressive multiple sclerosis (RSPMS).

## Fig. 1

**Fig. 2**

Mean AE-12-Y-QL Serum Concentration (µg/mL)

Time (day)

150 mg IV (N=6)
150 mg SC (N=5)

**Fig. 3**

Fig. 4

EP 4 324 476 A2

**Fig. 5**

LLOQ = 1.89 ng/mL

LLOQ = 1.89 ng/mL

**Fig. 6**

LLOQ = 1.89 ng/mL

LLOQ = 1.89 ng/mL

EP 4 324 476 A2

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6914128 B1
- WO 2001058956 A
- WO 0202773 A
- WO 2007024715 A
- US 7612181 B
- US 4554101 A
- WO 2013112922 A
- US 6019968 A
- US 5985320 A
- US 5985309 A
- US 5934272 A
- US 5874064 A
- US 5855913 A
- US 5290540 A
- US 4880078 A
- WO 9219244 A
- WO 9732572 A
- WO 9744013 A
- WO 9831346 A
- WO 9966903 A
- US 5679377 A
- US 5916597 A
- US 5912015 A
- US 5989463 A
- US 5128326 A
- WO 9915154 A
- WO 9920253 A
- US 4526 A
- US 938 A
- WO 9105548 A
- WO 9620698 A
- WO 04078140 A
- US 2006104968 A
- US 428082
- WO 9925044 A

**Non-patent literature cited in the description**

- **MARKS et al.** *BioTechnology,* 1992, vol. 10, 779-783
- **BARBAS et al.** *Proc. Nat. Acad. Sci. USA,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-3319
- **HAWKINS et al.** *J. Mol. Biol.,* 1992, vol. 226, 889-896
- **WU, C. et al.** *Nature Biotechnology,* 2007, vol. 25 (11), 1290-1297
- **MILSTEIN et al.** *Nature,* 1983, vol. 305 (5934), 537-540
- **STAERZ et al.** *Nature,* 1985, vol. 314 (6012), 628-631
- **HOLLIGER et al.** *Proc. Natl. Acad. Sci. USA,* 1993, vol. 90 (14), 6444-6448
- **KABAT et al.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 877-883
- **PADLAN.** *FASEB J.,* 1995, vol. 9, 133-139
- **MACCALLUM.** *J. Mol. Biol.,* 1996, vol. 262 (5), 732-745
- **WU et al.** *Nature Biotech.,* 2007, vol. 25, 1290-1297
- **WINNAKER.** From Genes to Clones. Verlagsgesellschaft, 1987
- **BIRD et al.** *Science,* 1988, vol. 242, 423-426
- **HUSTON et al.** *PNAS USA,* 1988, vol. 85, 5879-5883
- **MCCAFFERTY et al.** *Nature,* 1990, vol. 348, 552-554
- **LUBLIN, F. D. ; REINGOLD, S. O.** *Neurology,* 1996, (46), 907-911
- **WU, C. et al.** *Nature Biotechnology,* 2007, vol. 25, 1290-1297
- **KYTE et al.** *J. Mol. Biol.,* 1982, vol. 157, 105-132
- **FUNARO et al.** *BMC Biotechnology,* 2008, (8), 85
- **WILBUR, W. J. ; LIPMAN, D. J.** *Proc. Natl. Acad. Sci. USA,* 1983, vol. 80, 726-730
- **WU ; WU.** *J. Biol. Chem.,* 1987, vol. 262, 4429-4432
- **SEFTON.** *CRC Crit. Ref. Biomed. Eng.,* 1987, vol. 14, 20
- **BUCHWALD et al.** *Surgery,* 1980, vol. 88, 507

- **SAUDEK et al.** *N. Engl. J. Med.,* 1989, vol. 321, 574
- Medical Applications of Controlled Release. CRC Pres, 1974
- Controlled Drug Bioavailability, Drug Product Design and Performance. Wiley, 1984
- **RANGER ; PEPPAS.** *J., Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 7 (1), 105
- **GOODSON.** *Medical Applications of Controlled Release,* 1984, vol. 2, 115-138
- **LANGER.** *Science,* 1990, vol. 249, 1527-1533
- **NING et al.** Intratumoral Radioimmunotheraphy of a Human Colon Cancer Xenograft Using a Sustained-Release Gel. *Radiotherapy &Oncology,* 1996, vol. 39, 179-189
- **SONG et al.** Antibody Mediated Lung Targeting of Long- Circulating Emulsions. *PDA Journal of Pharmaceutical Science & Technology,* 1995, vol. 50, 372-397
- **CLEEK et al.** Biodegradable Polymeric Carriers for a bFGF Antibody for Cardiovascular Application. *Pro. Int'l. Symp. Control. Rel. Bioact. Mater.,* 1997, vol. 24, 853-854
- **LAM et al.** Microencapsulation of Recombinant Humanized Monoclonal Antibody for Local Delivery. *Proc. Int'l. Symp. Control Rel. Bioact. Mater.,* 1997, vol. 24, 759-760
- Sustained and Controlled Release Drug Delivery Systems. Marcel Dekker, Inc, 1978
- REMINGTON'S PHARMACEUTICAL SCIENCES. 1995
- **SHERWOOD et al.** *Bio/Technology,* 1992, vol. 10, 1446
- **SALTZMAN et al.** *Biophys. J.,* 1989, vol. 55, 163